(19) Europäisches Patentamt
European Patent Office
Office européen des brevets

(11) EP 2 781 216 A1

(12) EUROPEAN PATENT APPLICATION
published in accordance with Art. 153(4) EPC

(43) Date of publication:
24.09.2014 Bulletin 2014/39

(21) Application number: 12827928.8

(22) Date of filing: 31.08.2012

(51) Int Cl.:
A61K 31/519 (2006.01) A61K 31/5377 (2006.01)
A61P 17/00 (2006.01) A61P 17/02 (2006.01)
A61P 17/04 (2006.01) A61P 17/06 (2006.01)
A61P 17/10 (2006.01) A61P 29/00 (2006.01)
A61P 37/08 (2006.01) A61P 43/00 (2006.01)
C07D 487/04 (2006.01) C07D 519/00 (2006.01)
C12N 15/09 (2006.01)

(86) International application number:
PCT/JP2012/072104

(87) International publication number:
WO 2013/031931 (07.03.2013 Gazette 2013/10)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 02.09.2011 JP 2011192130

(71) Applicant: Kyowa Hakko Kirin Co., Ltd.
Tokyo 100-8185 (JP)

(72) Inventors:
- YAMAMOTO, Keisuke
Tokyo 100-8185 (JP)
- ARATAKE, Seiji
Tokyo 100-8185 (JP)
- HEMMI, Kazuki
Tokyo 100-8185 (JP)
- MIZUTANI, Mirai
Tokyo 100-8185 (JP)
- SENO, Yuko
Tokyo 100-8185 (JP)

(74) Representative: Vossius & Partner
Siebertstrasse 4
81675 München (DE)

# (54) CHEMOKINE RECEPTOR ACTIVITY REGULATOR

(57) The present invention provides a chemokine receptor activity modulator containing a pyrazolopyrimidine derivative represented by the formula (I)

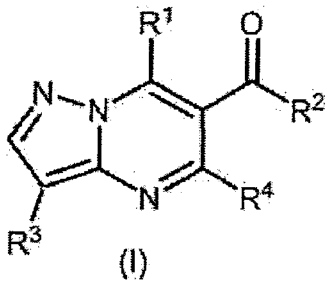

(I)

{wherein, $R^1$ represents $-NR^{1a}R^{1b}$ (wherein, $R^{1a}$ and $R^{1b}$ are the same or different and each represents a hydrogen atom, aralkyl and the like) and the like,
$R^2$ represents

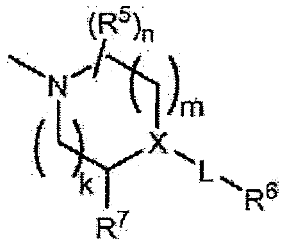

[wherein, k and m represent each an integer of 0 to 2, n represents an integer of 0 to 2,
L represents a single bond and the like,
$R^5$ represents halogen and the like,
$R^6$ represents aryl and the like,
X represents $-CR^8$ (wherein, $R^8$ represents a hydrogen atom and the like) and the like,
$R^7$ represents a hydrogen atom and the like] and the like,
$R^3$ represents $-SO_2R^{13}$ [wherein, $R^{13}$ is lower alkoxy, $-NR^{13d}(C=O)R^{13e}$ (wherein, $R^{13d}$ represents a hydrogen atom and the like),

$R^{13e}$ represents lower alkyl and the like) and the like] and the like, and
$R^4$ represents a hydrogen atom and the like}, or a pharmaceutically acceptable salt thereof as an active ingredient, and the like.

**Description**

TECHNICAL FIELD OF THE INVENTION

The present invention relates to a chemokine receptor activity modulator containing a pyrazolopyrimidine derivative or a pharmaceutically acceptable salt thereof as an active ingredient and the like.

BACKGROUND OF THE INVENTION

Chemokine is a series of families of small inflammatory cytokines having a strong chemotactic activity and is composed of about 70 to 120 amino acids. It is a chemotactic cytokine released from a wide range of cells to lead various cells such as monocyte, macrophage, T cell, eosinophil, basophil, neutrophil and the like to inflammatory sites. The chemokine family was originally defined by 4 conserved cysteine residues in the amino acid sequence. That is, it was classified into 2 subfamilies of CXC-chemokine family and CC-chemokine family based on the sequence of the first cysteine pair (2 residues) from the N terminal side. In the CXC-chemokine family including CXCL1, Mig, CX3CL1, IL-8, Gro-α, NAP-2, IP-10 and the like, these two cysteine residues are divided by one of the amino acid residues different from cysteine and, on the other hand, these two cysteine residues are adjacent to each other in the CC-chemokine family including RANTES (CCL5), MCP-1 (CCL2), MCP-2 (CCL8), MCP-3 (CCL7), MCP-4 (CCL13), MIP-1α (CCL3), MIP-1β (CCL4), Eotaxin (CCL11), Eotaxin-2 (CCL24), Eotaxin-3 (CCL26), PARC (CCL18), TARC (CCL17), MDC (CCL22), LARC (CCL20), ELC (CCL19), SLC (CCL21), I-309 (CCL1), TECK (CCL25), CTACK (CCL27), MEC (CCL28) and the like. Thereafter, the C chemokine family having, from the 4 cysteine residues to be originally present, only two cysteine residues corresponding to the second and the fourth residues from the N-terminal side, and the CX3C chemokine family having a sequence having 3 amino acid residues different from cysteine between the first two cysteine residues from the N-terminal side have been found.

As the chemokine receptor that CC-chemokine family binds, 10 kinds of receptors have been reported. That is, CCR1 (alias, CKR1 or CC-CKR-1) that binds MIP-1α, MIP-1β, MCP-3, RANTES and the like, CCR2A (alias, CKR2A or CC-CKR-2A) and CCR2B (alias, CKR2B or CC-CKR-2B) that bind MCP-1, MCP-2, MCP-3 and MCP-4 and the like, CCR3 (alias, CKR-3 or CC-CKR-3) that binds Eotaxin, Eotaxin-2, RANTES, MCP-2, MCP-3 and the like, CCR4 (alias, CKR4 or CC-CKR-4) that binds TARC, MDC and the like, CCR5 (alias, CKR-5 or CC-CKR-5) that binds MIP-1α, RANTES, MIP-1β and the like, CCR6 (alias, GPRCY4) that binds LARC and the like, CCR7 (alias, EBI-1) that binds ELC, SLC and the like, CCR8 that binds I-309 and the like, CCR9 (alias, GPR9-6) that binds TECK and the like, and CCR10 that binds CTACK, MEC and the like are known.

Chemokine receptors have different expression cells depending on the kind of the receptors. For example, CCR1 is expressed in various cells such as monocyte, T cell, mast cell, eosinophils, basophil and the like, and CCR2 is expressed in various cells such as dendritic cell, B cell, basophil, eosinophil, vascular endothelial cell, fibroblast, platelet, T cell and the like. On the other hand, some receptors are expressed only in some cells such as CCR3 expressed in eosinophil and basophil, and CCR9 expressed in T cell.

Since chemokine receptors have been reported to be involved in various diseases, a medicament that modulates a chemokine receptor activity is expected to be a therapeutic drug for various diseases. There have been used plural chemokine receptor activity modulators as therapeutic drugs. For example, it has been clarified that, when CD4+T cell is infected with HIV, HIV enters into the cell via CCR5. Therefore, CCR5 antagonists are used as therapeutic drugs for HIV infection. As a medicament for stem cell mobilization for autologous stem cell transplantation in patients with non-Hodgkin lymphoma or multiple myeloma, a combined use of an antagonist of CXCR4 which is a CXC-chemokine family receptor with G-CSF has been approved. In addition, CCR3 antagonist, CCR9 antagonist, antibody to CCR4 and the like are under clinical tests.

As diseases involving a chemokine receptor, inflammatory diseases such as asthma, rhinitis, dermatitis, allergic disease and the like, immunoregulatory disorders and diseases, autoimmune diseases such as rheumatoid arthritis, lupus erythematosus, systemic scleroderma, Sjogren's syndrome, Celiac disease and the like, and the like are known. It has been reported that the onset of these diseases involves chemokine receptors such as CCR1, CCR2A, CCR2B, CCR3, CCR4, CCR5, CCR6, CCR7, CCR8, CCR9 and CCR10, and CXCR3 and CXCR4, which are the receptors of CXC-chemokine family, and the like. Above all, as diseases involving CCR4, CCR8, CCR9 and CCR10 and the like, dermatic diseases and the like are known.

As dermatic diseases involving a chemokine receptor, acne vulgaris, drug eruption, contact dermatitis, pollinosis dermatitis, urticaria, psoriasis, atopic dermatitis, Candida dermatitis, seborrheic dermatitis, eczema, Stevens-Johnson syndrome, toxic epidermal necrosis, erythema multiforme, pityriasis rosea, lichen planus, lichen pilaris (keratosis pilaris), herpes simplex, lupus erythematosus, keloid, scabies, generalized scleroderma and dermatitis as a side effect of an anticancer agent, and the like are known. In these dermatic diseases, various chemokines are highly expressed in the skin. For example, in psoriasis, expression of chemokine such as MCP-1, RANTES, TARC, MDC, CTACK, CXCL1, Gro-α, IL-8, Mig, IP-10, CX3CL1 and the like increases at the dermatitis lesion, and T cell and neutrophil infiltrate into the skin via CCR4, CCR6 and CCR10, and CXCR1, CXCR2 and CXCR3, which are the receptors of CXC-chemokine family, and the like (non-patent document 1). And, in atopic dermatitis, expression of chemokine such as I-309, MCP-1, MIP-1α, MIP-1β, RANTES, Eotaxin, MCP-4, PARC, LARC, MDC, Eotaxin-3, CTACK and the like increases at the dermatitis lesion, and T cell, monocyte and eosinophil infiltrate into the skin via CCR1, CCR2A, CCR2B, CCR3, CCR4, CCR5, CCR6, CCR8 and CCR10, and CX3CR1 which is the receptor of CX3C chemokine family, and the like (non-patent document 2). It has been reported that an anti-CTACK antibody suppresses dermatitis in various dermatitis models and the like (for example, non-patent documents 3, 4, 5 and 6). A new medicament that controls chemokine receptor activity in dermatic diseases in which these chemokine receptors are involved and the like is needed.

Pyrazolo[1,5-a]pyrimidine derivatives are known to be useful as corticotrophin-releasing factor receptor antagonist (patent documents 1 and 2), adenosine enhancer (patent document 3), angiotensin II antagonist (patent document 4), tyrosine kinase inhibitor (patent documents 5 and 6), HMG-CoA inhibitor (patent document 7), NAD(H)oxidase inhibitor (patent document 8), adenosine $A_{2A}$ receptor agonist (patent documents 9 and 10), therapeutic drug for prostatic hyperplasia (patent document 11), therapeutic drug for cerebral circulatory disturbance (patent document 12), anti-obesity drug (patent document 13), antiinflammatory drug (patent document 14) and therapeutic drug for dermatic diseases (patent document 15).

[Prior Art Documents]

[patent documents]

patent document 1: WO 00/59908
patent document 2: JP-A-2000-38350
patent document 3: JP-A-10-101672
patent document 4: JP-A-7-157485
patent document 5: WO 00/53605
patent document 6: WO 98/54093
patent document 7: JP-A-4-270285
patent document 8: WO 03/091256
patent document 9: WO 02/40485
patent document 10: WO 2004/110454
patent document 11: JP-A-5-112571
patent document 12: EP-A-0328700
patent document 13: WO 00/44754
patent document 14: JP-A-9-169762
patent document 15: WO 2009/041663

[non-patent documents]

non-patent document 1: Clinical Dermatology, 2008, vol. 26, p. 539
non-patent document 2: Journal of Allergy and Clinical Immunology, 2006, vol. 118, p. 178
non-patent document 3: Nature Medicine, 2002, vol. 8, p. 157-165
non-patent document 4: International Immunology, 2006, vol. 18, p. 1233-1242
non-patent document 5: European Journal of Immunology, 2008, vol. 38, p. 647-657
non-patent document 6: Experimental Dermatology, 2007, vol. 17, p. 30-34

## DISCLOSURE OF THE INVENTION

### Problems to be Solved by the Invention

An object of the present invention is to provide a chemokine receptor activity modulator containing a pyrazolopyrimidine derivative or a pharmaceutically acceptable salt thereof as an active ingredient, and the like.

Means for Solving the Problems

The present invention relates to the following (1) to (48).

(1) A chemokine receptor activity modulator containing a pyrazolopyrimidine derivative represented by the formula (I)

[Chemical Formula 1]

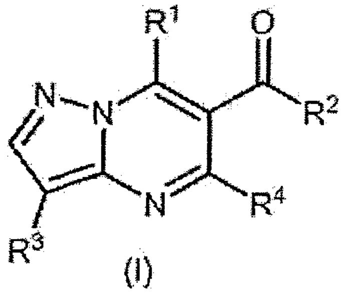

(I)

[wherein, $R^1$ represents $-NR^{1a}R^{1b}$ (wherein, $R^{1a}$ and $R^{1b}$ are the same or different and each represents a hydrogen atom, lower alkyl optionally having substituent(s), cycloalkyl optionally having substituent(s), lower alkoxy optionally having substituent(s), lower alkanoyl optionally having substituent(s), aryl optionally having substituent(s), aralkyl optionally having substituent(s), an aromatic heterocyclic group optionally having substituent(s) or an aliphatic heterocyclic group optionally having substituent(s), or $R^{1a}$ and $R^{1b}$ form, together with the adjacent nitrogen atom, a nitrogen-containing heterocyclic group optionally having substituent(s)), $-OR^{1c}$ (wherein, $R^{1c}$ represents a hydrogen atom, lower alkyl optionally having substituent(s), cycloalkyl optionally having substituent(s), aryl optionally having substituent(s), aralkyl optionally having substituent(s), an aromatic heterocyclic group optionally having substituent(s) or an aliphatic heterocyclic group optionally having substituent(s)) or $-SR^{1d}$ (wherein, $R^{1d}$ has the same definition as the above $R^{1c}$),
$R^2$ represents

[Chemical Formula 2]

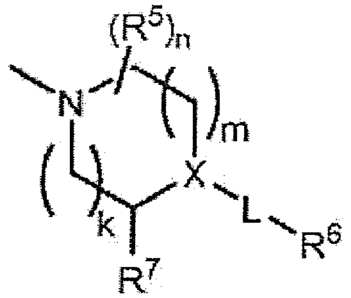

[wherein, k and m each represents an integer of 0 to 2 (provided that, the total of k and m is less than or equal to 3), n represents an integer of 0 to 4, and when n is 2, 3 or 4, respective $R^5$s may be the same or different,
L represents a single bond, alkylene, C(=O) or $SO_2$,
$R^5$ represents halogen, hydroxy, lower alkyl optionally having substituent(s) or lower alkoxy optionally having substituent(s), $R^6$ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
X represents a nitrogen atom or $-CR^8$ (wherein, $R^8$ represents a hydrogen atom, halogen, hydroxy, cyano, lower alkyl optionally having substituent(s) or lower alkoxy optionally having substituent(s), or forms a bond together with $R^7$), and $R^7$ forms a bond together with $R^8$, or represents a hydrogen atom, halogen, hydroxy, lower alkyl optionally having substituent(s) or lower alkoxy optionally having substituent(s)],

[Chemical Formula 3]

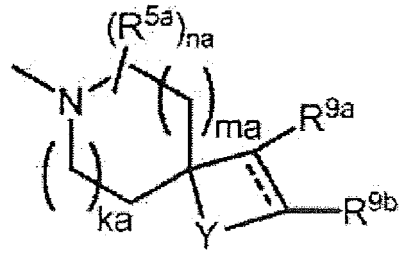

{wherein, ka, ma and na have the same definitions as the above k, m and n, respectively, $R^{5a}$ has the same definition as the above $R^5$, --- represents a single bond or a double bond,

$R^{9a}$ and $R^{9b}$ are the same or different and each represents a hydrogen atom or lower alkyl optionally having substituent(s), or $R^{9a}$ and $R^{9b}$ form, together with the respectively adjacent carbon atoms, an aliphatic ring optionally having substituent(s) or an aromatic ring optionally having substituent(s), and

Y represents $-CHR^{10a}-CHR^{10b}-$ (wherein, $R^{10a}$ and $R^{10b}$ are the same or different and each represents a hydrogen atom, hydroxy, lower alkyl optionally having substituent(s) or lower alkoxy optionally having substituent(s), or $R^{10a}$ and $R^{10b}$ form, together with the respectively adjacent carbon atoms, an aliphatic ring optionally having substituent(s)), $-CR^{10c}=CR^{10d}-$ (wherein, $R^{10c}$ and $R^{10d}$ are the same or different and each represents a hydrogen atom or lower alkyl optionally having substituent(s), or $R^{10c}$ and $R^{10d}$ form, together with the respectively adjacent carbon atoms, an aliphatic ring having at least one double bond and optionally having substituent(s) or an aromatic ring optionally having substituent(s)), $-Z^a-CR^{11a}R^{11b}-$ [wherein, $R^{11a}$ and $R^{11b}$ are the same or different and each represents a hydrogen atom or lower alkyl optionally having substituent(s), or $R^{11a}$ and $R^{11b}$ form carbonyl together with the adjacent carbon atom, and $Z^a$ represents C(=O), O, S, SO, $SO_2$ or $NR^{12}$ (wherein,

$R^{12}$ represents a hydrogen atom, lower alkyl optionally having substituent(s), lower alkanoyl optionally having substituent(s), lower alkoxycarbonyl optionally having substituent(s) or aralkyl optionally having substituent(s))], or $-CR^{11c}R^{11d}-Z^b-$(wherein, $R^{11c}$, $R^{11d}$ and $Z^b$ have the same definitions as the above $R^{11a}$, $R^{11b}$ and $Z^a$, respectively)}, or

[Chemical Formula 4]

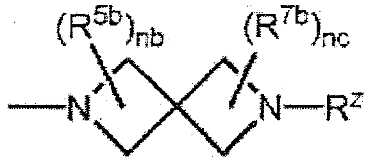

(wherein, $R^z$ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),

$R^{5b}$ and $R^{7b}$ are the same or different and each represents halogen, hydroxy, lower alkyl optionally having substituent(s) or lower alkoxy optionally having substituent(s),

nb represents an integer of 0 to 2, when nb is 2, respective $R^{5b}$s are the same or different, and

nc represents an integer of 0 to 2, when nc is 2, respective $R^{7b}$s are the same or different),

$R^3$ represents $-S(O)_2R^{13a}$ [wherein, $R^{13a}$ represents hydroxy, N,N-di-lower alkylaminomethyleneamino, lower alkoxy optionally having substituent(s), $-NR^{13b}R^{13c}$ (wherein, $R^{13b}$ and $R^{13c}$ are the same or different and each represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or $R^{13b}$ and $R^{13c}$ form, together with the adjacent nitrogen atom, a nitrogen-containing heterocyclic group optionally having substituent(s)), $-NR^{13d}C(=O)R^{13e}$ (wherein, $R^{13d}$ represents a hydrogen atom, lower alkyl optionally having substituent(s), cycloalkyl optionally having substituent(s), aralkyl optionally having substituent(s), aryl optionally having substituent(s), an aromatic heterocyclic group

optionally having substituent(s) or an aliphatic heterocyclic group optionally having substituent(s), and $R^{13e}$ represents a hydrogen atom, amino, lower alkyl optionally having substituent(s), cycloalkyl optionally having substituent(s), aralkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s), N,N-di-arylamino optionally having substituent(s), aryl optionally having substituent(s), an aromatic heterocyclic group optionally having substituent(s) or an aliphatic heterocyclic group optionally having substituent(s)), $-NR^{13f}C$ (=S) $R^{13g}$ (wherein, $R^{13f}$ and $R^{13g}$ have the same definitions as the above $R^{13d}$ and $R^{13e}$, respectively) or-$NR^{13h}S(O)_2R^{14}$ (wherein, $R^{13h}$ has the same definition as the above $R^{13d}$, and $R^{14}$ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s))], and

$R^4$ represents a hydrogen atom, halogen, lower alkyl optionally having substituent(s), aralkyl optionally having substituent(s), $-NR^{15a}R^{15b}$ (wherein, $R^{15a}$ and $R^{15b}$ have the same definitions as the above $R^{1a}$ and $R^{1b}$, respectively), $-OR^{15c}$ (wherein, $R^{15c}$ has the same definition as the above $R^{1c}$), or $-SR^{15d}$ (wherein, $R^{15d}$ has the same definition as the above $R^{1c}$)],

or a pharmaceutically acceptable salt thereof, as an active ingredient.

(2) A chemokine receptor activity modulator containing a pyrazolopyrimidine derivative represented by the formula (IA)

[Chemical Formula 5]

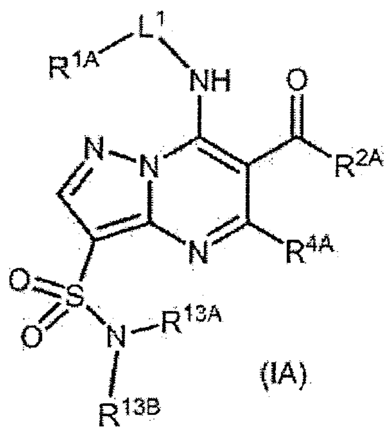

(IA)

[wherein,

$L^1$ represents a single bond or methylene,

$R^{1A}$ represents lower alkyl optionally having substituent(s), aralkyl optionally having substituent(s), cycloalkyl optionally having substituent(s), aryl optionally having substituent(s), an aromatic heterocyclic group optionally having substituent(s) or an aliphatic heterocyclic group optionally having substituent(s),

$R^{2A}$ represents

[Chemical Formula 6]

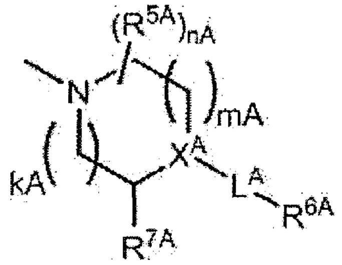

[wherein, nA represents an integer of 0 to 2, and when nA is 2, respective $R^{5A}$s may be the same or different, kA, mA and $L^A$ have the same definitions as the above k, m and l, respectively,
$R^{5A}$ represents halogen or lower alkyl,
$R^{6A}$ represents cycloalkyl optionally having substituent(s), aryl optionally having substituent(s), an aromatic heterocyclic group optionally having substituent(s) or an aliphatic heterocyclic group optionally having substituent(s),
$X^A$ represents a nitrogen atom or $-CR^{8A}$ (wherein, $R^{8A}$ represents a hydrogen atom, halogen or lower alkyl, or forms a bond together with $R^{7A}$), and
$R^{7A}$ forms a bond together with $R^{8A}$, or represents a hydrogen atom, halogen or lower alkyl],

[Chemical Formula 7]

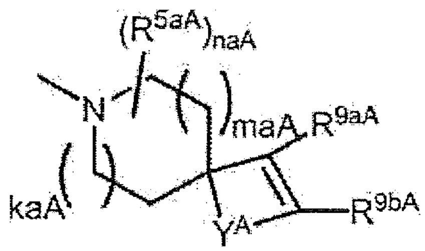

{wherein, naA and $R^{5aA}$ have the same definitions as the above nA and $R^{5A}$, respectively,
maA and kaA has the same definition as the above ma and ka, respectively,
$R^{9aA}$ and $R^{9bA}$ form, together with the respectively adjacent carbon atoms, an aromatic ring optionally having substituent(s), and
$Y^A$ represents $-CHR^{10aA}-CHR^{10bA}-$ (wherein, $R^{10aA}$ and $R^{10bA}$ are the same or different and each represents a hydrogen atom, hydroxy, lower alkyl optionally having substituent(s) or lower alkoxy optionally having substituent(s)), $-CR^{10cA}=CR^{10dA}-$ (wherein, $R^{10cA}$ and $R^{10dA}$ are the same or different and each represents a hydrogen atom or lower alkyl optionally having substituent(s)), $-Z^{aA}-CR^{11aA}R^{11bA}-$ [wherein, $R^{11aA}$ and $R^{11bA}$ are the same or different and each represents a hydrogen atom or lower alkyl optionally having substituent(s), or $R^{11aA}$ and $R^{11bA}$ form carbonyl together with the adjacent carbon atom, and $Z^{aA}$ represents C(=O), O, S, SO, $SO_2$ or $NR^{12A}$ (wherein, $R^{12A}$ represents a hydrogen atom, lower alkyl optionally having substituent(s), lower alkanoyl optionally having substituent(s), lower alkoxycarbonyl optionally having substituent(s) or aralkyl optionally having substituent(s))], or $-CR^{11ca}R^{11da}-Z^{bA}-$ (wherein, $R^{11cA}$, $R^{11dA}$ and $Z^{bA}$ has the same definition as the above $R^{11aA}$, $R^{11bA}$ and $Z^{aA}$, respectively)}, or

[Chemical Formula 8]

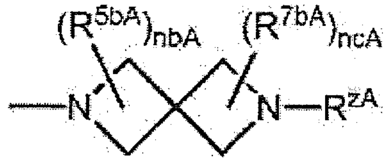

(wherein, $R^{zA}$ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s), $R^{5bA}$ and $R^{7bA}$ are the same or different and each represents halogen, hydroxy, lower alkyl optionally having substituent(s) or lower alkoxy optionally having substituent(s),

nbA represents an integer of 0 to 2, when nbA is 2, respective $R^{5bA}$s are the same or different, and

ncA represents an integer of 0 to 2, when ncA is 2, respective $R^{7bA}$s are the same or different),

$R^{13A}$ represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or $R^{13A}$ form, together with $R^{13B}$ and the adjacent nitrogen atom, a nitrogen-containing heterocyclic group optionally having substituent(s),

$R^{13B}$ represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s), an aliphatic heterocyclic group optionally having substituent(s) or $COR^{13e1}$ (wherein, $R^{13e1}$ has the same definition as the above $R^{13e}$), or $R^{13B}$ form, together with $R^{13A}$ and the adjacent nitrogen atom, a nitrogen-containing heterocyclic group optionally having substituent(s), and

$R^{4A}$ represents a hydrogen atom or lower alkyl optionally having substituent(s)],

or a pharmaceutically acceptable salt thereof, as an active ingredient.

(3) The chemokine receptor activity modulator containing the pyrazolopyrimidine derivative, or a pharmaceutically acceptable salt thereof, as an active ingredient, according to the above (2), wherein $L^1$ is a single bond.

(4) The chemokine receptor activity modulator containing the pyrazolopyrimidine derivative, or a pharmaceutically acceptable salt thereof, as an active ingredient, according to the above (2) or (3), wherein $R^{1A}$ is aryl optionally having substituent(s).

(5) The chemokine receptor activity modulator containing the pyrazolopyrimidine derivative, or a pharmaceutically acceptable salt thereof, as an active ingredient, according to the above (2) or (3), wherein $R^{1A}$ is phenyl optionally having substituent(s).

(6) The chemokine receptor activity modulator containing the pyrazolopyrimidine derivative, or a pharmaceutically acceptable salt thereof, as an active ingredient, according to any one of the above (2) to (5), wherein $R^{1A}$ is a hydrogen atom.

(7) The chemokine receptor activity modulator containing the pyrazolopyrimidine derivative, or a pharmaceutically acceptable salt thereof, as an active ingredient, according to any one of the above (2) to (6), wherein $R^{13A}$ is a hydrogen atom and $R^{13B}$ is lower alkyl optionally having substituent(s).

(8) The chemokine receptor activity modulator containing the pyrazolopyrimidine derivative, or a pharmaceutically acceptable salt thereof, as an active ingredient, according to any one of the above (2) to (6), wherein $R^{13A}$ is a hydrogen atom and $R^{13B}$ is $COR^{13e1}$ (wherein, $R^{13e1}$ has the same definition as described above).

(9) The chemokine receptor activity modulator containing the pyrazolopyrimidine derivative, or a pharmaceutically acceptable salt thereof, as an active ingredient, according to any one of the above (2) to (6), wherein $R^{13A}$ is a hydrogen atom and $R^{13B}$ is $COR^{13e2}$ (wherein, $R^{13e2}$ represents lower alkyl optionally having substituent(s), cycloalkyl optionally having substituent(s), N-mono-lower alkylamino optionally having substituent(s) or N-mono-arylamino optionally having substituent(s)).

(10) The chemokine receptor activity modulator containing the pyrazolopyrimidine derivative, or a pharmaceutically acceptable salt thereof, as an active ingredient, according to any one of the above (2) to (9), wherein $R^{2A}$ is

[Chemical Formula 9]

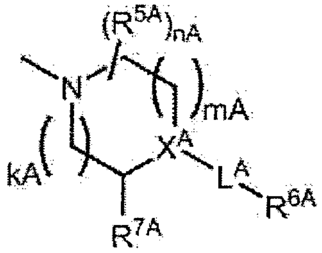

(wherein, nA, mA, kA, $R^{5A}$, $R^{6A}$, $R^{7A}$, $L^A$ and $X^A$ have the same definitions as described above, respectively).

(11) The chemokine receptor activity modulator containing the pyrazolopyrimidine derivative, or a pharmaceutically acceptable salt thereof, as an active ingredient, according to the above (10), wherein $R^{6A}$ is phenyl optionally having substituent(s).

(12) The chemokine receptor activity modulator containing the pyrazolopyrimidine derivative, or a pharmaceutically acceptable salt thereof, as an active ingredient, according to any one of the above (2) to (9), wherein $R^{2A}$ is

[Chemical Formula 10]

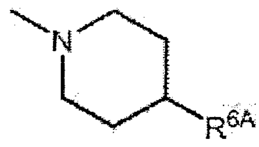

(wherein, $R^{6A}$ represents phenyl optionally having substituent(s)).

(13) The chemokine receptor activity modulator containing the pyrazolopyrimidine derivative, or a pharmaceutically acceptable salt thereof, as an active ingredient, according to any one of the above (2) to (9), wherein $R^{2A}$ is

[Chemical Formula 11]

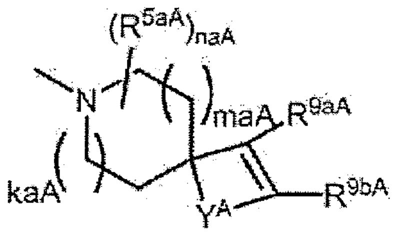

(wherein, naA, maA, kaA, $R^{5aA}$, $R^{9aA}$, $R^{9bA}$ and $Y^A$ have the same definitions as described above, respectively).

(14) The chemokine receptor activity modulator containing the pyrazolopyrimidine derivative, or a pharmaceutically acceptable salt thereof, as an active ingredient, according to the above (13), wherein $R^{9aA}$ and $R^{9bA}$ form, together with the respectively adjacent carbon atoms, a benzene ring optionally having substituent(s), and $Y^A$ is $-CHR^{10aA}-CHR^{10bA}-$ (wherein, $R^{10aA}$ and $R^{10bA}$ have the same definitions as described above, respectively), $-CR^{10cA}=CR^{10dA}-$ (wherein, $R^{10cA}$ and $R^{10dA}$ have the same definitions as described above, respectively), $-O-CR^{11aA}R^{11bA}-$ (wherein, $R^{11aA}$ and $R^{11bA}$ have the same definitions as described above, respectively) or $-CR^{11cA}R^{11dA}-O-$ (wherein, $R^{11cA}$ and $R^{11dA}$ have the same definitions as described above, respectively).

(15) The chemokine receptor activity modulator containing the pyrazolopyrimidine derivative, or a pharmaceutically acceptable salt thereof, as an active ingredient, according to any one of the above (2) to (9), wherein $R^{2A}$ is

[Chemical Formula 12]

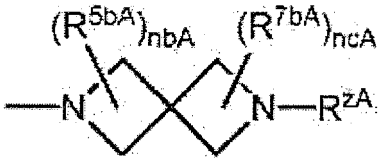

(wherein, $R^{zA}$, $R^{5bA}$, $R^{7bA}$, nbA and ncA have the same definitions as described above, respectively).

(16) The chemokine receptor activity modulator containing the pyrazolopyrimidine derivative, or a pharmaceutically acceptable salt thereof, as an active ingredient, according to the above (15), wherein $R^{zA}$ is phenyl optionally having substituent(s).

(17) The chemokine receptor activity modulator according to any one of the above (1) to (16), wherein the chemokine receptor activity modulator is a chemokine receptor antagonist.

(18) The chemokine receptor activity modulator according to any one of the above (1) to (17), wherein the chemokine receptor is that selected from CCR4, CCR8, CCR9 and CCR10.

(19) The chemokine receptor activity modulator according to any one of the above (1) to (17), wherein the chemokine receptor is CCR10.

(20) A preventive and/or therapeutic agent for a disease involving a chemokine receptor, containing the pyrazolopyrimidine derivative described in any one of the above (1) to (16) or a pharmaceutically acceptable salt thereof as an active ingredient.

(21) The preventive and/or therapeutic agent for a disease involving a chemokine receptor according to the above (20), wherein the chemokine receptor is that selected from CCR4, CCR8, CCR9 and CCR10.

(22) The preventive and/or therapeutic agent for a disease involving a chemokine receptor according to the above (20), wherein the chemokine receptor is CCR10.

(23) The preventive and/or therapeutic agent for a disease involving a chemokine receptor according to any one of the above (20) to (22), wherein the disease involving a chemokine receptor is a dermatic disease.

(24) The preventive and/or therapeutic agent according to the above (23), wherein the dermatic disease is that selected from acne vulgaris, drug eruption, contact dermatitis, lepidopteran dermatitis, pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis.

(25) The preventive and/or therapeutic agent according to the above (23), wherein the dermatic disease is dermatitis.

(26) The preventive and/or therapeutic agent according to the above (23), wherein the dermatic disease is that selected from contact dermatitis and atopic dermatitis.

(27) A method of modulating a chemokine receptor activity, comprising administering an effective amount of the pyrazolopyrimidine derivative described in any one of the above (1) to (16) or a pharmaceutically acceptable salt thereof.

(28) The method of modulating a chemokine receptor activity according to the above (27), wherein the method of modulating a chemokine receptor is a method of antagonizing a chemokine receptor.

(29) The method of modulating a chemokine receptor activity according to the above (27) or (28), wherein the chemokine receptor is that selected from CCR4, CCR8, CCR9 and CCR10.

(30) The method of modulating a chemokine receptor activity according to the above (27) or (28), wherein the chemokine receptor is CCR10.

(31) A method for preventing and/or treating a disease involving a chemokine receptor, comprising administering an effective amount of the pyrazolopyrimidine derivative described in any one of the above (1) to (16) or a pharmaceutically acceptable salt thereof.

(32) The method for preventing and/or treating a disease involving a chemokine receptor according to the above (31), wherein the chemokine receptor is that selected from CCR4, CCR8, CCR9 and CCR10.

(33) The method for preventing and/or treating a disease involving a chemokine receptor according to the above (31), wherein the chemokine receptor is CCR10.

(34) The method for preventing and/or treating a disease involving a chemokine receptor according to any one of the above (31) to (33), wherein the disease involving a chemokine receptor is a dermatic disease.

(35) The method for preventing and/or treating a disease involving a chemokine receptor according to the above (34), wherein the dermatic disease is that selected from acne vulgaris, drug eruption, contact dermatitis, lepidopteran dermatitis, pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis.

(36) The method for preventing and/or treating a disease involving a chemokine receptor according to the above (34), wherein the dermatic disease is dermatitis.

(37) The method for preventing and/or treating a disease involving a chemokine receptor according to the above (34), wherein the dermatic disease is that selected from contact dermatitis and atopic dermatitis.

(38) The pyrazolopyrimidine derivative described in any one of the above (1) to (16) or a pharmaceutically acceptable salt thereof for use in the modulation of chemokine receptor activity.

(39) The pyrazolopyrimidine derivative according to the above (38) or a pharmaceutically acceptable salt thereof, wherein the modulation of chemokine receptor activity is an antagaonism of chemokine receptor.

(40) The pyrazolopyrimidine derivative according to the above (38) or (39) or a pharmaceutically acceptable salt thereof, wherein the chemokine receptor is that selected from CCR4, CCR8, CCR9 and CCR10.

(41) The pyrazolopyrimidine derivative according to the above (38) or (39) or a pharmaceutically acceptable salt thereof, wherein the chemokine receptor is CCR10.

(42) The pyrazolopyrimidine derivative described in any one of the above (1) to (16) or a pharmaceutically acceptable salt thereof for use in the prevention and/or treatment of a disease involving a chemokine receptor.

(43) The pyrazolopyrimidine derivative according to the above (42) or a pharmaceutically acceptable salt thereof,

wherein the chemokine receptor is that selected from CCR4, CCR8, CCR9 and CCR10.
(44) The pyrazolopyrimidine derivative according to the above (42) or a pharmaceutically acceptable salt thereof, wherein the chemokine receptor is CCR10.
(45) The pyrazolopyrimidine derivative according to any one of the above (42) to (44) or a pharmaceutically acceptable salt thereof, wherein the disease involving a chemokine receptor is a dermatic disease.
(46) The pyrazolopyrimidine derivative according to the above (45) or a pharmaceutically acceptable salt thereof, wherein the dermatic disease is that selected from acne vulgaris, drug eruption, contact dermatitis, lepidopteran dermatitis, pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis.
(47) The pyrazolopyrimidine derivative according to the above (45) or a pharmaceutically acceptable salt thereof, wherein the dermatic disease is dermatitis.
(48) The pyrazolopyrimidine derivative according to the above (45) or a pharmaceutically acceptable salt thereof, wherein the dermatic disease is that selected from contact dermatitis and atopic dermatitis.

Effect of the Invention

According to the present invention, a chemokine receptor activity modulator containing a pyrazolopyrimidine derivative or a pharmaceutically acceptable salt thereof as an active ingredient and the like are provided.

Mode for Carrying Out the Invention

In the present specification, compounds represented by the formula (I) and the formula (IA) are referred to as compound (I) and compound (IA), respectively. The same applies to the compounds having other formula numbers.
In the definition of each group in the formula (I) and the formula (IA), examples of the lower alkyl, and the lower alkyl moiety of the lower alkoxy, the lower alkoxycarbonyl, the lower alkanoyl, the N,N-di-lower alkylaminomethyleneamino, the N-mono-lower alkylamino and the N,N-di-lower alkylamino include linear or branched alkyl having 1 to 10 carbon atoms, more specifically, methyl, ethyl, propyl, isopropyl, butyl, isobutyl, sec-butyl, tert-butyl, pentyl, isopentyl, neopentyl, hexyl, heptyl, octyl, nonyl, decyl and the like. Two lower alkyl moieties of the N,N-di-lower alkylamino and N,N-di-lower alkylaminomethyleneamino may be the same or different.
The alkylene has the same meaning as the group formed by removing one hydrogen atom from the above lower alkyl.
Examples of the cycloalkyl, and the cycloalkyl moiety of the N-cycloalkylamino include cycloalkyl having 3 to 8 carbon atoms, more specifically, cyclopropyl, cyclobutyl, cyclopentyl, cyclohexyl, cycloheptyl, cyclooctyl and the like.
Examples of the aryl, and the aryl moiety of the N-mono-arylamino and the N,N-diarylamino include aryl having 6 to 14 carbon atoms, more specifically, phenyl, naphthyl, azulenyl, anthryl, pentalenyl, indenyl, biphenylenyl and the like. The two aryl moieties of the N,N-diarylamino may be the same or different.
The alkylene moiety of the aralkyl has the same meaning as the group formed by removing one hydrogen atom from the above lower alkyl, and the aryl moiety of the aralkyl has the same meaning as the above aryl.
Examples of the aliphatic heterocyclic group include a 3-to 7-membered monocyclic aliphatic heterocyclic group containing at least one atom selected from a nitrogen atom, an oxygen atom and a sulfur atom, a bicyclic or tricyclic fused aliphatic heterocyclic group in which 3- to 8-membered rings are fused, containing at least one atom selected from a nitrogen atom, an oxygen atom and a sulfur atom, and the like, more specifically, aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxiranyl, tetrahydrofuranyl, tetrahydro-2H-pyranyl, 5,6-dihydro-2H-pyranyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolidinyl, dihydrobenzooxazolyl, dihydrobenzothioxazolyl, benzodioxolinyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl, dihydrobenzodioxanyl, oxetanyl and the like.
Examples of the aromatic heterocyclic group include a 5-membered or 6-membered monocyclic aromatic heterocyclic group containing at least one atom selected from a nitrogen atom, an oxygen atom and a sulfur atom, a bicyclic or tricyclic fused aromatic heterocyclic group in which 3- to 8-membered rings are fused, containing at least one atom selected from a nitrogen atom, an oxygen atom and a sulfur atom, and the like, more specifically, furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl, pyrrolopyrimidinyl, imidazopyridinyl, purinyl, quinolinyl, isoquinolinyl, cinnolinyl, phthalazinyl, quinazolinyl, quinoxalinyl, naphthyridinyl, azepinyl, diazepinyl, pyranyl, oxepinyl, thiopyranyl, thiepinyl, furazanyl, oxadiazolyl, oxazinyl, oxadiazinyl, oxazepinyl, oxadiazepinyl, thiazinyl, thiadiazinyl, thiazepinyl, thiadiazepinyl, indolizinyl, isobenzofuranyl, isobenzothiophenyl, dithianaphthalenyl, quinolizinyl, pteridinyl, benzoxazolidinyl, chromenyl, benzoxepinyl, benzoxadiazepinyl, benzothiepinyl, benzothiazepinyl, benzothiadiazepinyl,
benzoazepinyl, benzodiazepinyl, benzofurazanyl, benzothiadiazolinyl, carbazolyl, β-carbolinyl, acridinyl, phenazinyl, dibenzofuranyl, xanthenyl, dibenzothiophenyl, phenothiazinyl, phenoxazinyl, phenoxathinyl, thianthrenyl, phenanthridinyl, phenanthrolinyl, perimidinyl and the like.

Examples of the nitrogen-containing heterocyclic group formed together with the adjacent nitrogen atom thereto include a 5-membered or 6-membered monocyclic heterocyclic group containing at least one nitrogen atom (said monocyclic heterocyclic group may contain any other of a nitrogen atom, an oxygen atom or a sulfur atom), a bicyclic or tricyclic fused heterocyclic group in which 3- to 8-membered rings are fused, containing at least one nitrogen atom (said fused heterocyclic group may contain any other of a nitrogen atom, an oxygen atom or a sulfur atom), and the like, more specifically, aziridinyl, azetidinyl, pyrrolidinyl, piperidino, azepanyl, pyrrolinyl, imidazolidinyl, imidazolyl, pyrazolidinyl, pyrazolinyl, pyrazolyl, piperazinyl, homopiperazinyl, oxazolidinyl, 2H-oxazolyl, thioxazolidinyl, 2H-thioxazolyl, morpholino, thiomorpholinyl, dihydroindolyl, dihydroisoindolyl, indolyl, isoindolyl, tetrahydroquinolyl, tetrahydroisoquinolyl, dihydrobenzooxazolyl, dihydrobenzothioxazolyl, benzimidazolidinyl, benzimidazolyl, dihydroindazolyl, indazolyl, benzotriazolyl, pyrrolopyridinyl, pyrrolopyrimidinyl, imidazopyridinyl, purinyl and the like.

Examples of the aliphatic ring include aliphatic rings corresponding to the above cycloalkyl and aliphatic heterocyclic group.

Examples of the aliphatic ring having at least one double bond include aliphatic rings having one or more double bond from among the above aliphatic ring, and more specific examples thereof include 1,2,5,6-tetrahydropyridine, tetrahydro-2H-pyran, 2H-oxazone, 2H-thioxazone, dihydroindoline, dihydroisoindoline, dihydrobenzofuran, dihydrobenzooxazoline, dihydrobenzothioxazoline, dihydro-2H-chromane, dihydro-1H-chromane, dihydro-2H-thiochromane, dihydro-1H-thiochromane, dihydrobenzodioxane and the like.

Examples of the aromatic ring include aromatic rings corresponding to the above aryl and aromatic heterocyclic group.

The halogen means each atom of fluorine, chlorine, bromine or iodine.

The substituent(s) (substituent group-1) in the lower alkyl optionally having substituent(s), the lower alkoxy optionally having substituent(s), the N-mono-lower alkylamino optionally having substituent(s), the N,N-di-lower alkylamino optionally having substituent(s), the lower alkoxycarbonyl optionally having substituent(s) and the lower alkanoyl optionally having substituent(s) are the same or different and examples thereof include 1 to 3 substituents selected from the group consisting of halogen; sulfanyl; nitro; cyano; $C_{3-8}$ cycloalkyl optionally having 1 to 3 substituents selected from the following substituent group C; an aliphatic heterocyclic group optionally having 1 to 3 substituents selected from the following substituent group C; an aromatic heterocyclic group optionally having 1 to 3 substituents selected from the following substituent group B; $C_{1-10}$ alkylsulfanyl optionally having 1 to 3 substituents selected from the following substituent group A; $C_{6-14}$ arylsulfanyl optionally having 1 to 3 substituents selected from the following substituent group B; $C_{1-10}$ alkylsulfonyl optionally having 1 to 3 substituents selected from the following substituent group A; $C_{6-14}$ arylsulfonyl optionally having 1 to 3 substituents selected from the following substituent group B; $OR^{16}$ (wherein $R^{16}$ represents a hydrogen atom, $C_{1-10}$ alkyl optionally having 1 to 3 substituents selected from the following substituent group A, $C_{3-8}$ cycloalkyl optionally having 1 to 3 substituents selected from the following substituent group C, $C_{6-14}$ aryl optionally having 1 to 3 substituents selected from the following substituent group B, $C_{7-16}$ aralkyl optionally having 1 to 3 substituents selected from the following substituent group B, an aromatic heterocyclic group optionally having 1 to 3 substituents selected from the following substituent group B, $C_{2-11}$ alkanoyl optionally having 1 to 3 substituents selected from the following substituent group A, $C_{7-15}$ aroyl optionally having 1 to 3 substituents selected from the following substituent group B, $C_{1-10}$ alkylsulfonyl optionally having 1 to 3 substituents selected from the following substituent group A or $C_{6-14}$ arylsulfonyl optionally having 1 to 3 substituents selected from the following substituent group B); $C(=O)R^{17a}$ (wherein $R^{17a}$ represents amino, hydroxy, $C_{1-10}$ alkyl optionally having 1 to 3 substituents selected from the following substituent group A, $C_{3-8}$ cycloalkyl optionally having 1 to 3 substituents selected from the following substituent group C, $C_{6-14}$ aryl optionally having 1 to 3 substituents selected from the following substituent group B, an aliphatic heterocyclic group optionally having 1 to 3 substituents selected from the following substituent group C, an aromatic heterocyclic group optionally having 1 to 3 substituents selected from the following substituent group B, $C_{1-10}$ alkoxy optionally having 1 to 3 substituents selected from the following substituent group A, $C_{6-14}$ aryloxy optionally having 1 to 3 substituents selected from the following substituent group B, $C_{1-10}$ alkylamino optionally having 1 to 3 substituents selected from the following substituent group A, di-$C_{1-10}$ alkylamino optionally having 1 to 3 substituents selected from the following substituent group A or $C_{6-14}$ arylamino optionally having 1 to 3 substituents selected from the following substituent group B); and $-NR^{18a}R^{18b}$ (wherein $R^{18a}$ and $R^{18b}$ are the same or different and each represents a hydrogen atom, formyl, $C_{1-10}$ alkyl optionally having 1 to 3 substituents selected from the following substituent group A, $C_{3-8}$ cycloalkyl optionally having 1

to 3 substituents selected from the following substituent group C, $C_{6-14}$ aryl optionally having 1 to 3 substituents selected from the following substituent group B, an aliphatic heterocyclic group optionally having 1 to 3 substituents selected from the following substituent group C, an aromatic heterocyclic group optionally having 1 to 3 substituents selected from the following substituent group B, $C_{2-11}$ alkanoyl optionally having 1 to 3 substituents selected from the following substituent group A, $C_{7-15}$ aroyl optionally having 1 to 3 substituents selected from the following substituent group B, $C_{1-10}$ alkoxycarbonyl optionally having 1 to 3 substituents selected from the following substituent group A, $C_{1-10}$ alkylsulfonyl optionally having 1 to 3 substituents selected from the following substituent group A or $C_{6-14}$ arylsulfonyl optionally having 1 to 3 substituents selected from the following substituent group B). Examples of the substituent in the lower alkoxy optionally having substituent(s), the lower alkoxycarbonyl optionally having substituent(s) and the lower alkanoyl optionally having substituent (s) also include $C_{6-14}$ aryl optionally having 1 to 3 substituents selected from the following substituent group B, in addition to the above substituent group-1.

The substituent(s) in the aryl optionally having substituent(s), the N-mono-arylamino optionally having substituent(s), the N,N-diarylamino optionally having substituent(s), the phenyl optionally having substituent(s), the aralkyl optionally having substituent(s), the aromatic heterocyclic group optionally having substituent(s), the aromatic ring optionally having substituent(s) and the benzene ring optionally having substituent(s) are the same or different and examples thereof include 1 to 3 substituents selected from the group consisting of $C_{1-10}$ alkyl optionally having 1 to 3 substituents selected from the following substituent group A, $C_{6-14}$ aryl optionally having 1 to 3 substituents selected from the following substituent group B and the substituents of the aforementioned substituent group-1. The substituent in the aryl optionally having substituent(s) include a ring in which the aryl moiety is fused with $C_{4-8}$ cycloalkyl ring optionally having 1 to 3 substituents selected from the following substituent group C, or an aliphatic heterocyclic ring optionally having 1 to 3 substituents selected from the following substituent group C, in addition to the above substituent(s). The substituent in the phenyl optionally having substituent(s) include a ring in which the phenyl moiety is fused with $C_{4-8}$ cycloalkyl ring optionally having 1 to 3 substituents selected from the following substituent group C, or an aliphatic heterocyclic ring optionally having 1 to 3 substituents selected from the following substituent group C, in addition to the above substituent(s). The substituent in the aryl moiety of the aralkyl optionally having substituent(s) include a ring in which the aryl moiety is fused with $C_{4-8}$ cycloalkyl ring optionally having 1 to 3 substituents selected from the following substituent group C, or an aliphatic heterocycle ring optionally having 1 to 3 substituents selected from the following substituent group C, in addition to the above substituent(s).

The substituent(s) in the cycloalkyl optionally having substituent(s), the N-cycloalkylamino optionally having substituent(s), the aliphatic heterocyclic group optionally having substituent(s), the aliphatic ring having at least one double bond and optionally having substituent(s), the nitrogen-containing heterocyclic group optionally having substituent(s), which is formed together with the adjacent nitrogen atom, and the aliphatic ring optionally having substituent(s) are the same or different and examples thereof include 1 to 3 substituents selected from the group consisting of oxo, $C_{1-10}$ alkyl optionally having 1 to 3 substituents selected from the following substituent group A, $C_{6-14}$ aryl optionally having 1 to 3 substituents selected from the following substituent group B and the substituents of the above substituent group-1. The substituent in the cycloalkyl optionally having substituent(s) include a ring in which the cycloalkyl moiety is fused with a benzene ring optionally having 1 to 3 substituents selected from the following substituent group B, in addition to the above substituent(s).

The substituent group A means the group consisting of halogen; hydroxy; sulfanyl; nitro; cyano; carboxy; carbamoyl; $C_{3-8}$ cycloalkyl; $C_{6-14}$ aryl optionally having 1 to 3 substituents selected from the group consisting of halogen, hydroxy, amino, nitro, carboxy, $C_{1-10}$ alkoxycarbonyl, $C_{1-10}$ alkoxy and trifluoromethyl (substituent group a); an aliphatic heterocyclic group; an aromatic heterocyclic group; $C_{1-10}$ alkoxy optionally having 1 to 3 substituents selected from the group consisting of halogen, hydroxy, amino, carboxy, $C_{1-10}$ alkoxy, $C_{1-10}$ alkylamino, di-$C_{1-10}$ alkylamino and $C_{1-10}$ alkoxycarbonyl (substituent group b); $C_{3-8}$ cycloalkoxy; $C_{6-14}$ aryloxy optionally having 1 to 3 substituents selected from the above substituent group a; $C_{7-16}$ aralkyloxy optionally having 1 to 3 substituents selected from the above substituent group a; $C_{2-11}$ alkanoyloxy; $C_{7-15}$ aroyloxy; $C_{1-10}$ alkylsulfonyloxy; trifluoromethanesulfonyloxy; $C_{6-14}$ arylsulfonyloxy; p-toluenesulfonyloxy; $C_{1-10}$ alkylsulfanyl; $C_{6-14}$ arylsulfanyl; - $NR^{19a}R^{19b}$ (wherein $R^{19a}$ and $R^{19b}$ are the same or different and each represents a hydrogen atom; formyl; $C_{1-10}$ alkyl optionally having 1 to 3 substituents selected from the above substituent group b; $C_{3-8}$ cycloalkyl; $C_{6-14}$ aryl optionally having 1 to 3 substituents selected from the above substituent group a; an aromatic heterocyclic group; $C_{7-16}$ aralkyl optionally having 1 to 3 substituents selected from the above substituent group a; $C_{2-11}$ alkanoyl; $C_{7-15}$ aroyl; $C_{1-10}$ alkoxycarbonyl; $C_{7-16}$ aralkyloxycarbonyl; $C_{1-10}$ alkylsulfonyl; trifluoromethanesulfonyl; $C_{6-14}$ arylsulfonyl or p-toluenesulfonyl) ; $C_{2-11}$ alkanoyl; $C_{3-8}$ cycloalkylcarbonyl; $C_{7-15}$ aroyl; aliphatic heterocyclylcarbonyl; aromatic heterocyclylcarbonyl; $C_{1-10}$ alkoxycarbonyl; $C_{6-14}$ aryloxycarbonyl; $C_{7-16}$ aralkyloxycarbonyl; $C_{1-10}$ alkylcarbamoyl; di-$C_{1-10}$ alkylcarbamoyl and $C_{6-14}$ arylcarbamoyl.

The substituent group B means the group consisting of $C_{1-10}$ alkyl, trifluoromethyl and the substituents of the above substituent group A.

The substituent group C means the group consisting of oxo, $C_{1-10}$ alkyl, trifluoromethyl and the substituents of the above substituent group A.

Examples of the $C_{1-10}$ alkyl and the $C_{1-10}$ alkyl moiety of the $C_{1-10}$ alkoxy, the $C_{2-11}$ alkanoyloxy, the $C_{1-10}$ alkylsulfanyl, the $C_{2-11}$ alkanoyl, the $C_{1-10}$ alkoxycarbonyl, the $C_{1-10}$ alkylcarbamoyl, the di-$C_{1-10}$ alkylcarbamoyl, the $C_{1-10}$ alkylsulfonyl, the $C_{1-10}$ alkylsulfonyloxy, the $C_{1-10}$ alkylamino and the di-$C_{1-10}$ alkylamino shown here include the groups exemplified as the above lower alkyl. Two $C_{1-10}$ alkyl moieties of di-$C_{1-10}$ alkylcarbamoyl and di-$C_{1-10}$ alkylamino may be the same or different.

Examples of the $C_{3-8}$ cycloalkyl and the $C_{3-8}$ cycloalkyl moiety of the $C_{3-8}$ cycloalkoxy and the $C_{3-8}$ cycloalkylcarbonyl include the groups exemplified as the above cycloalkyl.

Examples of the aryl fused with a $C_{4-8}$ cycloalkyl ring and the moiety formed by removing the alkylene moiety from the aralkyl of which the aryl moiety is fused with a $C_{4-8}$ cycloalkyl ring include a cycloalkyl-fused aryl having 8 to 16 carbon atoms, more specifically, indanyl, 1,2,3,4-tetrahydronaphthalenyl and the like.

Examples of the phenyl fused with a $C_{4-8}$ cycloalkyl ring include a cycloalkyl-fused phenyl having 8 to 12 carbon atoms, more specifically, indanyl, 1,2,3,4-tetrahydronaphthalenyl and the like.

Examples of the cycloalkyl fused with a benzene ring include a benzene ring-fused cycloalkyl having 8 to 12 carbon atoms, more specifically, indanyl, 1,2,3,4-tetrahydronaphthalenyl and the like.

Examples of the $C_{6-14}$ aryl, and the aryl moiety of the $C_{6-14}$ aryloxy, the $C_{6-14}$ arylamino, the $C_{6-14}$ arylsulfanyl, the $C_{7-15}$ aroyl, the $C_{7-15}$ aroyloxy, the $C_{6-14}$ aryloxycarbonyl, the $C_{6-14}$ arylsulfonyl, the $C_{6-14}$ arylsulfonyloxy and the $C_{6-14}$ arylcarbamoyl include the groups exemplified as the above aryl.

Examples of the aryl moiety of the $C_{7-16}$ aralkyloxy, the $C_{7-16}$ aralkyl and the $C_{7-16}$ aralkyloxycarbonyl include the group exemplified as the above aryl, and examples of the alkylene moiety thereof include $C_{1-10}$ alkylene and the like, more specifically, the group formed by removing one hydrogen atom from the above lower alkyl and the like.

Examples of the aliphatic heterocyclic group and the aliphatic heterocyclic group moiety of the aliphatic heterocyclylcarbonyl include the groups exemplified as the above aliphatic heterocyclic group.

Examples of the aromatic heterocyclic group and the aromatic heterocyclic group moiety of the aromatic heterocyclylcarbonyl include the groups exemplified as the above aromatic heterocyclic group.

Examples of the aryl fused with a aliphatic heterocycle and the moiety formed by removing the alkylene moiety from the aralkyl of which the aryl moiety is fused with a aliphatic heterocycle include aryl fused with a 4- to 7-membered monocyclic aliphatic heterocyclic group containing at least one atom selected from a nitrogen atom, an oxygen atom and a sulfur atom, more specifically, dihydrobenzofuranyl, dihydroisobenzofuranyl, indolinyl, isoindolinyl, chromanyl, isochromanyl and the like.

Examples of the phenyl fused with a aliphatic heterocycle include phenyl fused with a 4- to 7-membered monocyclic aliphatic heterocyclic group containing at least one atom selected from a nitrogen atom, an oxygen atom and a sulfur atom, more specifically, dihydrobenzofuranyl, dihydroisobenzofuranyl, indolinyl, isoindolinyl, chromanyl, isochromanyl and the like.

Examples of the halogen include the atom exemplified as the above halogen.

In each group of compound (I), $R^1$ is preferably $NR^{1a}R^{1b}$ (wherein, $R^{1a}$ and $R^{1b}$ are each as defined above), more preferably, one of $R^{1a}$ and $R^{1b}$ is a hydrogen atom, and the other is lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and more preferably, one of $R^{1a}$ and $R^{1b}$ is a hydrogen atom, and the other is aralkyl optionally having substituent(s) or aryl optionally having substituent(s) (wherein, the substituent(s) in the aralkyl optionally having substituent(s) or the aryl optionally having substituent(s) is preferably lower alkyl or halogen, and the number of substituents(s) is preferably 2 or 3). In addition, it is also more preferable that one of $R^{1a}$ and $R^{1b}$ is a hydrogen atom, and the other is an aromatic heterocyclic group optionally having substituent(s).

When $R^2$ is

[Chemical Formula 13]

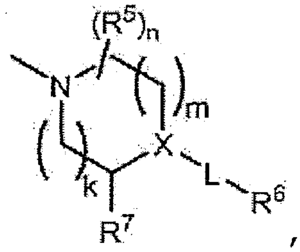

,

$R^5$ is preferably lower alkyl optionally having substituent(s), more preferably lower alkyl,

$R^6$ is preferably aryl optionally having substituent(s) or an aromatic heterocyclic group optionally having substituent(s), more preferably aryl optionally having substituent(s), still more preferably phenyl optionally having substituent(s),
$R^7$ is preferably a hydrogen atom or lower alkyl,
X is preferably a nitrogen atom or $CR^{8a}$ (wherein, $R^{8a}$ represents a hydrogen atom, halogen or lower alkyl), more preferably $CR^{8a}$ (wherein, $R^{8a}$ is as defined above),
L is preferably a single bond,
k and m are each preferably 1, and
n is preferably 0 or 1, more preferably 0.

When $R^2$ is

[Chemical Formula 14]

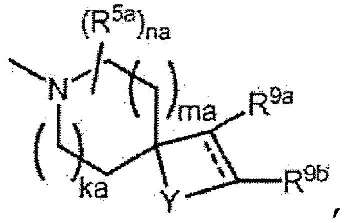

$R^{5a}$ is preferably lower alkyl optionally having substituent(s), more preferably lower alkyl.
$R^{9a}$ and $R^{9b}$ preferably form, together with the respectively adjacent carbon atoms, an aromatic ring optionally having substituent(s), more preferably form, together with the respectively adjacent carbon atoms, a benzene ring optionally having substituent(s),
Y is preferably $-CHR^{10a}-CHR^{10b}-$ (wherein, $R^{10a}$ and $R^{10b}$ are each as defined above), $-CR^{10c}=CR^{10d}-$ (wherein, $R^{10c}$ and $R^{10d}$ are each as defined above), $-O-CR^{11a}R^{11b}-$ (wherein, $R^{11a}$ and $R^{11b}$ are each as defined above) or $-CR^{11c}R^{11d}-O-$ (wherein, $R^{11c}$ and $R^{11d}$ are each as defined above), more preferably $-CHR^{10aa}-CHR^{10ba}-$(wherein, $R^{10aa}$ and $R^{10ba}$ are the same or different and each represents a hydrogen atom or lower alkyl), $-CR^{10ca}=CR^{10da}-$(wherein, $R^{10ca}$ and $R^{10da}$ are the same or different and each represents a hydrogen atom or lower alkyl), $-O-CR^{11aa}R^{11ba}-$(wherein, $R^{11aa}$ and $R^{11ba}$ are the same or different and each represents a hydrogen atom or lower alkyl), or $-CR^{11ca}R^{11da}-O-$(wherein, $R^{11ca}$ and $R^{11da}$ are the same or different and each represents a hydrogen atom or lower alkyl),
ka and ma are each preferably 1, and
na is preferably 0 or 1, more preferably 0.

Also, when $R^2$ is

[Chemical Formula 15]

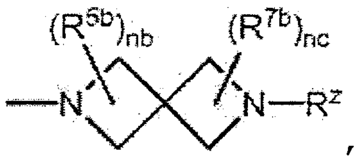

$R^z$ is preferably aryl optionally having substituent(s) or an aromatic heterocyclic group optionally having substituent(s), more preferably aryl optionally having substituent(s), still more preferably phenyl optionally having substituent(s),
$R^{5b}$ and $R^{7b}$ are the same or different and each is preferably halogen, hydroxy or lower alkyl optionally having substituent(s), more preferably halogen or lower alkyl optionally having substituent(s), still more preferably lower alkyl optionally having substituent(s), and
nb is preferably 0 and nc is preferably 0.
$R^3$ is preferably the following (A) or (B).
(A) $S(O)_2NR^{13b}R^{13c}$ (wherein, $R^{13b}$ and $R^{13c}$ are each as defined above), and it is more preferable when $R^{13b}$ is

a hydrogen atom and $R^{13c}$ is lower alkyl optionally having substituent(s), lower alkoxy optionally having substituent(s) or cycloalkyl optionally having substituent(s).

(B) $S(O)_2NR^{13d}C(=O)R^{13e}$ (wherein, $R^{13d}$ and $R^{13e}$ are each as defined above), and it is more preferabe when $R^{13d}$ is a hydrogen atom and $R^{13e}$ is lower alkyl optionally having substituent(s), cycloalkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s) or N,N-di-arylamino optionally having substituent(s).

$R^4$ is preferably a hydrogen atom.

As compound (I), a compound, wherein one or more of the above-mentioned preferable substituent embodiments are combined, is preferable.

In each group of compound (IA),

$R^{1A}$ is preferably aryl optionally having substituent(s) or an aromatic heterocyclic group optionally having substituent(s), more preferably aryl optionally having substituent(s), still more preferably phenyl optionally having substituent(s) (wherein, the substituent(s) in the aryl optionally having substituent(s) or the phenyl optionally having substituent(s) is preferably lower alkyl or halogen, and the number of substituents(s) is preferably 2 or 3), and it is also more preferable embodiment when $R^{1A}$ is an aromatic heterocyclic group optionally having substituent(s).

$L^1$ is preferably a single bond.

When $R^{2A}$ is

[Chemical Formula 16]

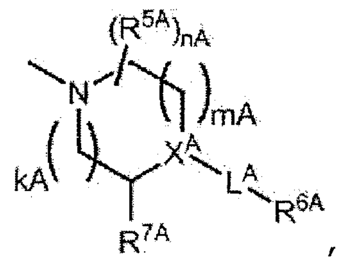

$R^{5A}$ is preferably lower alkyl,

$R^{6A}$ is preferably aryl optionally having substituent(s) or an aromatic heterocyclic group optionally having substituent(s), more preferably aryl optionally having substituent(s), still more preferably phenyl optionally having substituent(s),

$L^A$ is preferably a single bond,

$R^{7A}$ is preferably a hydrogen atom or lower alkyl,

$X^A$ is preferably a nitrogen atom or $-CR^{8Aa}$ (wherein, $R^{8Aa}$ represents a hydrogen atom, halogen or lower alkyl), more preferably $-CR^{8Aa}$ (wherein, $R^{8Aa}$ is as defined above),

mA and kA are each preferably 1, and

nA is preferably 0 or 1, more preferably 0.

Further, when $R^{2A}$ is

[Chemical Formula 17]

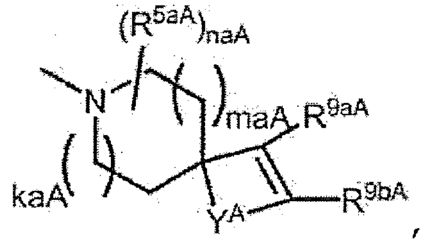

$R^{5aA}$ is preferably lower alkyl,

kaA and maA are each preferably 1,

$R^{9aA}$ and $R^{9bA}$ preferably form, together with the respectively adjacent carbon atoms, a benzene ring optionally

having substituent(s),
$Y^A$ is preferably $-CHR^{10aA}-CHR^{10bA}-$ (wherein, $R^{10aA}$ and $R^{10bA}$ are each as defined above), $-CR^{10cA}=CR^{10dA}-$ (wherein, $R^{10cA}$ and $R^{10dA}$ are each as defined above), $-O-CR^{11aA}R^{11bA}-$ (wherein, $R^{11aA}$ and $R^{11bA}$ are each as defined above) or $-CR^{11cA}R^{11dA}-O-$ (wherein, $R^{11cA}$ and $R^{11dA}$ are each as defined above), more preferably $-CHR^{10aAa}-CHR^{10bAa}-$ (wherein, $R^{10aAa}$ and $R^{10bAa}$ are the same or different and each represents a hydrogen atom or lower alkyl), $-CR^{10cAa}=CR^{10dAa}-$ (wherein, $R^{10cAa}$ and $R^{10dAa}$ are the same or different and each represents a hydrogen atom or lower alkyl), $-O-CR^{11aAa}R^{11bAa}-$ (wherein, $R^{11aAa}$ and $R^{11bAa}$ are the same or different and each represents a hydrogen atom or lower alkyl) or $-CR^{11cAa}R^{11aAa}-C-$ (wherein, $R^{11cAa}$ and $R^{11dAa}$ are the same or different and each represents a hydrogen atom or lower alkyl), and
naA is preferably 0 or 1, more preferably 0.

Also, when $R^{2A}$ is

[Chemical Formula 18]

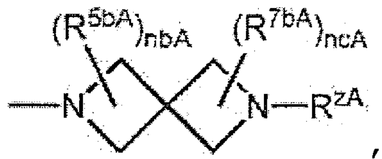

,

$R^{zA}$ is preferably aryl optionally having substituent(s) or an aromatic heterocyclic group optionally having substituent(s), more preferably aryl optionally having substituent(s), still more preferably phenyl optionally having substituent(s),
$R^{5bA}$ and $R^{7bA}$ are the same or different and each is preferably halogen, hydroxy or lower alkyl optionally having substituent(s), more preferably halogen or lower alkyl optionally having substituent(s), still more preferably lower alkyl optionally having substituent(s), and
nbA is preferably 0 and ncA is preferably 0.
$R^{13A}$ is preferably a hydrogen atom or lower alkyl optionally having substituent(s), more preferably a hydrogen atom.
$R^{13B}$ is preferably lower alkyl optionally having substituent(s), lower alkoxy optionally having substituent(s) or $COR^{13e1}$ (wherein, $R^{13e1}$ is as defined above), more preferably lower alkyl, halogen-substituted lower alkyl (wherein, the halogen moiety of the halogen-substituted lower alkyl is as defined for the above halogen and the lower alkyl moiety is as defined for the above alkylene) or $COR^{13e2}$ (wherein, $R^{13e2}$ is as defined above).
$R^{4A}$ is preferably a hydrogen atom.

As compound (IA), a compound, wherein one or more of the above-mentioned preferable substituent embodiments are combined, is preferable. In addition, compound (IA) described in (3)-(16) of [Means for Solving the Problems] of which substituents are limited according to the above-mentioned preferable embodiments of the substituents, are preferable. Furthermore, compound (IA) described in (3)-(16) of [Means for Solving the Problems] of which substituents are limited according to combinations of two or more above-mentioned preferable embodiments of the substituents, are more preferable.

The pharmaceutically acceptable salts of compound (I) and (IA) include, for example, pharmaceutically acceptable acid addition salts, metal salts, ammonium salts, organic amine addition salts, amino acid addition salts and the like. Examples of the pharmaceutically acceptable acid addition salts of compounds (I) and (IA) include inorganic acid salts such as hydrochloride, hydrobromide, nitrate, sulfate, phosphate and the like, organic acid salts such as acetate, oxalate, maleate, fumarate, citrate, benzoate, methanesulfonate and the like, and the like. Examples of the pharmaceutically acceptable metal salts include sodium salt, potassium salt, magnesium salt, calcium salt, aluminum salt, zinc salt and the like. Examples of the pharmaceutically acceptable ammonium salt include ammonium salt, tetramethylammonium salt and the like. Examples of the pharmaceutically acceptable organic amine addition salt include addition salts of morpholine, piperidine or the like. Examples of the pharmaceutically acceptable amino acid addition salt include addition salts of lysine, glycine, phenylalanine, aspartic acid, glutamic acid and the like. The pharmaceutically acceptable saltsof compound (I) and (II) are not limit to these examples.

Also, the pharmaceutically acceptable salts of compounds (I) and (IA) include quaternary ammonium salt. The quaternary ammonium salt means the salt wherein the nitrogen atom in the compound is quaternarized by Rx (Rx is lower alkyl or lower alkyl substituted by phenyl wherein the lower alkyl is as defined above).

Moreover, the pharmaceutically acceptable salts of compounds (I) and (IA) also include N-oxide. N-oxide is a compound wherein the nitrogen atom in the compound is oxidized. By using the compounds (I) and (IA) which is not N-oxide, and in the any oxidation method, for example, by using m-chloroperbenzoic acid, air oxidation, an oxidation reagent such as liver extract and the like, the N-oxide of compounds (I) and (IA) can be obtained.

In the present invention, the dermatic diseases refer to those where the lesion appears on the skin. Specific examples thereof include, but are not limited to, acne vulgaris, drug eruption, contact dermatitis, lepidopteran dermatitis, pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis and the like.

The chemokine receptor activity modulator refers to a medicament that acts on a ligand of a chemokine receptor (chemokine etc.) or a chemokine receptor, preferably, a chemokine receptor antagonist.

The chemokine receptor antagonist refers to, for example, a medicament that suppresses intercellular signaling signals produced at the downstream of the chemokine receptor by the interaction of ligand and chemokine receptor, specifically, a medicament that suppresses an increase in the intracellular calcium and the like.

Examples of the chemokine include CC-chemokine families such as RANTES (CCL5), MCP-1 (CCL2), MCP-2 (CCL8), MCP-3 (CCL7), MCP-4 (CCL13), MIP-1$\alpha$ (CCL3), MIP-1$\beta$ (CCL4), Eotaxin (CCL11), Eotaxin-2 (CCL24), Eotaxin-3 (CCL26), PARC (CCL18), TARC (CCL17), MDC (CCL22), LARC (CCL20), ELC (CCL19), SLC (CCL21), I-309 (CCL1), TECK (CCL25), CTACK (CCL27) and MEC (CCL28) and the like, preferably TARC, MDC, I-309, TECK, CTACK and MEC.

Examples of the chemokine receptor include CCR1 (alias, CKR1 or CC-CKR-1) that binds MIP-1$\alpha$, MIP-1$\beta$, MCP-3, RANTES and the like, CCR2A (alias, CKR2A or CC-CKR-2A) and CCR2B (alias, CKR2B or CC-CKR-2B) that bind MCP-1, MCP-2, MCP-3 and MCP-4 and the like, CCR3 (alias, CKR-3 or CC-CKR-3) that binds Eotaxin, Eotaxin-2, RANTES, MCP-2 and MCP-3 and the like, CCR4 (alias, CKR4 or CC-CKR-4) that binds TARC, MDC and the like, CCR5 (alias, CKR-5 or CC-CKR-5) that binds MIP-1$\alpha$, RANTES, MIP-1$\beta$ and the like, CCR6 (alias, GPRCY4) that binds LARC and the like, CCR7 (alias, EBI-1) that binds ELC, SLC and the like, CCR8 that binds I-309 and the like, CCR9 (alias, GPR9-6) that binds TECK and the like, CCR10 that binds CTACK, MEC and the like, and the like, preferably CCR4, CCR8, CCR9 and CCR10.

Examples of the disease involving a chemokine receptor include a disease involving at least one of CCR1, CCR2A, CCR2B, CCR3, CCR4, CCR5, CCR6, CCR7, CCR8, CCR9 and CCR10, preferably a disease involving CCR4, CCR8, CCR9 and CCR10 singly or two or more in combination. Examples of these diseases involving a chemokine receptor include inflammatory diseases and/or dermatic diseases such as asthma, rhinitis, dermatitis, allergic disease and the like, HIV infectious disease, immunoregulatory disorder and disease, autoimmune diseases such as rheumatoid arthritis, Sjogren's syndrome, Celiac disease and the like, and the like, preferably dermatic diseases, more preferably dermatic diseases involving CCR10. The disease involving a chemokine receptor in the present invention is not limited to these.

The dermatitis in the present invention refers to, from among the above dermatic diseases, one wherein immune system is endogenously or exogenously activated to cause skin symptoms. Specific examples thereof include, but are not limited to, acne vulgaris, contact dermatitis, atopic dermatitis, pollen dermatitis, psoriasis, drug eruption, lupus erythematosus, seborrheic dermatitis, eczema, Stevens-Johnson syndrome, toxic epidermal necrosis and the like.

The treatment in the present invention refers to reversing, mitigating or inhibiting of the progression of the disease or condition to be applied, or one or more symptoms of such disease or condition. Moreover, it includes application to inhibit progression before remission of disease, or when the symptom is mild. In dermatic diseases, aggravation and remission may be repeated regularly or chronically. The agent for prevention and/or treatment is also used for extending the remission period and preventing aggravation. The agent for prevention is also used for preventing the onset of a disease.

The aggravation in the present specification refers to aggravation of the symptoms of a disease.

The remission in the present specification refers to a temporary or permanent reduction or disappearance of the symptoms of a disease. In addition, the remission phase refers to a condition in remission, and the remission duration refers to duration of sustained remission.

Compounds (I) and (IA) used in the present invention include a prodrugs thereof. The prodrugs of compounds (I) and (IA) are compounds that can be converted to compound (I) or (IA) by a reaction with an enzyme, gastric acid and the like in the body. As the prodrug, many kinds thereof are known, and a suitable prodrug may be selected from a known literature (for example, Iyakuhin no Kaihatsu, Hirokawa Shoten, 1990, vol. 7, p. 163) and synthesized by a known method. For example, as a prodrug of compounds (I) and (IA), when compounds (I) and (IA) have amino, a compound wherein the amino is acylated, alkylated or phosphorylated, when compounds (I) and (IA) have hydroxy, a compound wherein the hydroxy is acylated, alkylated, phosphorylated or borated, when compounds (I) and (IA) have carboxy, a

compound wherein the carboxy is esterified or amidated, and the like can be exemplified. Also, the prodrug of compounds (I) and (IA) may be any of hydrate, non-hydrate and solvate, and may form a salt with a pharmaceutically acceptable acid or base, in the same manner as in the case of compounds (I) and (IA).

A preferable compound used in the present specification is a compound having desirable properties in one or more items from various evaluation items required for an agent for the prevention and/or treatment of dermatic diseases, or pharmaceutical products, such as pharmacological activity, as well as physical stability, stability under physiological conditions, safety to the body, and the like.

Compounds (I) and (IA), and pharmaceutically acceptable salts thereof to be used in the present invention may show an unpreferable action on living organisms. Even in such a case, the usefulness of the agent for the prevention and/or treatment of dermatic diseases, and the pharmaceutical product can be exhibited while reducing an unpreferable action by using appropriate dose and administration method.

Compounds (I) and (IA) to be used in the present invention include those possibly having a geometric isomer, a stereoisomer such as optical isomer and the like, tautomer and the like. The present invention encompasses all possible isomers and mixtures thereof including them, and the mixing ratio thereof may be arbitrary ratio.

Compounds (I) and (IA), and pharmaceutically acceptable salts thereof to be used in the present invention may be present as adducts with water or various solvents, and the present invention also encompasses such adducts.

A part or all of the respective atoms in compound (I) and compound (IA) may be replaced by corresponding isotope atom(s), and the present invention also encompasses such compounds replaced by isotope atom(s). For example, a part or all of hydrogen atoms in compound (I) may be a hydrogen atom having an atomic weight of 2 (deuterium atom). A compound incorporating a radioisotope such as $^{3}H$ (tritium) or $^{14}C$ from among the isotopes is useful for examining the tissue distribution of a compound and screening for an agent for the prevention and/or treatment of dermatic diseases.

For example, a compound wherein a part or all of the respective atoms in compound (I) is/are replaced by corresponding isotope atom(s) can be produced using a commercially available building block and in the same manner as in each of the above-mentioned production methods. In addition, a compound wherein a part or all of the hydrogen atoms in compound (I) is/are replaced by deuterium atom(s) can be synthesized by, for example, 1) a method using deuterium peroxide, to deuterate carboxylic acid and the like under basic conditions (see US Patent No. 3849458), 2) a method using an iridium complex as a catalyst, to deuterate alcohol, carboxylic acid and the like by using deuterium oxide as a deuterium source [see Journal of the American Chemical Society (J. Am. Chem. Soc.), Vol. 124, No. 10, 2092 (2002)], 3) a method using a palladium carbon as a catalyst, to deuterate fatty acid by using only a deuterium gas as a deuterium source [see LIPIDS, Vol. 9, No. 11, 913(1974)], 4) a method using a metal such as platinum, palladium, rhodium, ruthenium, iridium and the like as a catalyst, to deuterate acrylic acid, methyl acrylate, methacrylic acid, methyl methacrylate and the like by using deuterium oxide, or deuterium oxide and deuterium gas, as a deuterium source (see JP-B-5-19536, JP-A-61-277648 and JP-A-61-275241), 5) a method using a catalyst such as palladium, nickel, copper or chromite copper and the like, to deuterate acrylic acid, methyl methacrylate and the like by using deuterium oxide as deuterium source (see JP-A-63-198638) and the like.

The isotope atom used in the present specification refers to an atom having an atomic value or a mass number different from those generally found naturally. Examples of the isotope in the compound of the present invention include $^{2}H$, $^{3}H$, $^{13}C$, $^{14}C$, $^{15}N$, $^{17}O$, $^{18}O$, $^{31}P$, $^{32}P$, $^{35}S$, $^{18}F$, $^{36}Cl$, and the like.

The inert solvent used in the present specification is an organic or inorganic solvent, which is liquid at room temperature or reaction temperature, and shows no change in the chemical structure after the reaction. Specific examples thereof include, but are not limited to, tetrahydrofuran (THF), dioxane, carbon tetrachloride, acetone, ethyl acetate, methyl acetate, isopropyl acetate, diethyl ether, ethylene glycol, triethylene glycol, glyme, diglyme, 1,2-dimethoxyethane (DME), acetonitrile, methanol, ethanol, butanol, 2-propanol, methylene chloride, chloroform, benzene, water, toluene, pyridine, N,N-dimethylformamide (DMF), dimethylimidazole, N-methylpyrrolidine, N-methylpyrrolidinone, dimethylpropyleneurea, hexane, pentane, nitrobenzene, dimethyl sulfoxide (DMSO), diphenyl ether, Dowtherm A (registered trade mark), polychlorinated diphenyl, tetralin, heptane, octane, xylene, methyl ethyl ketone, methyl isobutyl ketone, N,N-dimethylacetamide, sulfolane, 1,2-dichloroethane and the like.

Next, production methods of compound (I) are explained in the following.

In the production methods shown below, when the defined groups change under the conditions of the production methods or are inappropriate for performing the production methods, the desired compound can be produced by using methods for introducing and removing of protecting groups conventionally used in the synthetic organic chemistry (for example, methods described in Protective Groups in Organic Synthesis, third edition, T.W. Greene, John Wiley & Sons Inc., 1999, and the like) and the like. If necessary, the order of the reaction steps can also be changed.

Production method 1

[Chemical Formula 19]

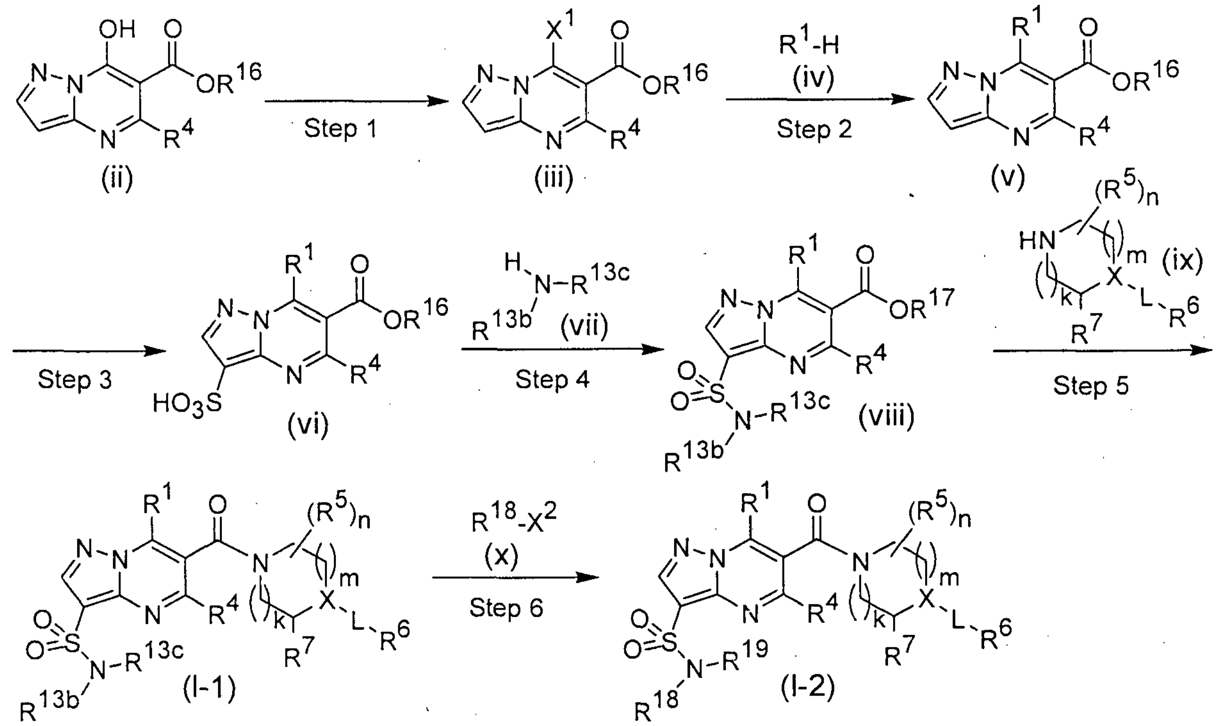


[wherein, $R^1$, $R^4$, $R^5$, $R^6$, $R^7$, $R^{13b}$, $R^{13c}$, X, L, k, m and n are each as defined above, $R^{16}$ represents lower alkyl (the lower alkyl is as defined above), $R^{17}$ represents a hydrogen atom or lower alkyl (the lower alkyl is as defined above), $R^{18}$ represents -C(=O)$R^{13e}$ (wherein $R^{13e}$ is as defined above), - C(=S)$R^{13g}$ (wherein $R^{13g}$ is as defined above) or -$S(O)_2R^{14}$ (wherein $R^{14}$ is as defined above), $R^{19}$ is as defined for the above $R^{13d}$, $X^2$ represents a chlorine atom, a bromine atom or hydroxy, and $X^1$ represents a leaving group. Examples of the leaving group include sulfonyloxy such as benzenesulfonyloxy, methanesulfonyloxy, p-toluenesulfonyloxy and the like, a group formed by removing one hydrogen atom from carboxylic acid such as lower alkanoyloxy (the lower alkanoyl moiety of said lower alkanoyloxy is as defined for the above lower alkanoyl), arylcarbonyloxy (the aryl moiety of said arylcarbonyloxy is as defined for the above aryl), aromatic heterocyclylcarbonyloxy (the aromatic heterocyclyl moiety of said aromatic heterocyclylcarbonyloxy is as defined for the above aromatic heterocyclic group) and the like, a chlorine atom, a bromine atom, an iodine atom, a fluorine atom and the like]

A conversion reaction for $R^1$, $R^4$, $R^5$, $R^6$, $R^7$, $R^{13b}$, $R^{13c}$, $R^{16}$, $R^{17}$, $R^{18}$, $R^{19}$, $X^1$ and $X^2$ can be performed during any step of steps 1 to 6 in the present production method.

(step 1)

Compound (iii) can be obtained by reacting compound (ii) in an inert solvent or without solvent with 0.5 to 50 equivalents, preferably a solvent amount, of a leaving group-introducing reagent at a temperature between -30°C and 150°C for 15 min to 24 hr.

As the leaving group-introducing reagent, a suitable reagent can be selected according to the leaving group to be used. For example, when $X^1$ is a halogen atom such as a chlorine atom, a bromine atom and the like, phosphorus oxychloride, phosphorus oxybromide, phosphorus pentachloride, trichloroacetyl chloride, phosphorus trichloride, phosphorus tribromide, acetyl chloride, thionyl chloride and the like can be used. In this case, 0.5 to 3 equivalents of, for example, N,N-diisopropylethylamine, N,N-diethylaniline and the like may be added.

Preferably, the reaction is performed using phosphorus oxychloride without solvent at a temperature between 80°C and 120°C.

Compound (ii) can be synthesized by a known method [for example, the method described in Journal of Medicinal Chemistry, vol. 49, page 2526, 2006] or modified methods thereto.

(step 2)

Compound (v) can be obtained by reacting compound (iii) with 0.5 to 10 equivalents, preferably 1 to 3 equivalents,

of compound (iv) in an inert solvent or without solvent at a temperature between the melting point of the solvent to be used and the boiling point thereof for 15 min to 24 hr, and if necessary, by useing a base, and if further necessary, by irradiating a microwave having an energy of 100 to 500 (W) under pressurization conditions of 1 to 20 atm.

Examples of the base include sodium hydroxide, potassium carbonate, sodium hydrogen carbonate, barium hydroxide, cesium carbonate, potassium hydroxide, sodium methoxide, potassium ethoxide, lithium hydroxide, lithium hexamethyldisilasane, sodium hydride, potassium hydride, n-butyllithium, lithium diisopropylamide, potassium tert-butoxide, triethylamine, N,N-diisopropylethylamine, tributylamine, dicyclohexyl methyl amine, N-methylmorpholine, pyridine, N-methylpiperidine, 2,6-di-tert-butylpyridine, 1,8-diazabicyclo[5.4.0]undec-7-ene (DBU), 1,5-diazabicyclo[4.3.0]non-5-ene (DBN), 4-(dimethylamino)pyridine, Amberlyst A-21 (manufactured by ROHM AND HAAS), AG 1-X8 (manufactured by Bio-Rad), polyvinyl pyridine, morpholinomethylpolystyrene and the like.

The microwave refers to 1 GHz - 1 THz electromagnetic wave, and 2450 MHz is preferably used.

The irradiation energy is more preferably 300 W.

Preferably, DMF, N,N-dimethylacetamide or acetonitrile is used as an inert solvent, potassium carbonate, triethylamine or N,N-diisopropylethylamine is used as a base, and the reaction is performed at a temperature between 50°C and 120°C for 1 to 24 hr.

(step 3)

Compound (vi) can be obtained by reacting compound (v) in an inert solvent or without solvent with 0.5 to 10 equivalents, preferably 1 to 3 equivalents, of a sulfonylating agent at a temperature between -30°C and the boiling point of the solvent to be used for 15 min to 48 hr.

Examples of the inert solvent include 1,2-dichloroethane, chloroform, methylene chloride, sulfolane, ethyl acetate and the like, and these can be used singly or as a mixture thereof. Among these, 1,2-dichloroethane or methylene chloride is preferable.

Examples of the sulfonylating agent include chlorosulfonic acid, fuming sulfuric acid, sulfur trioxide, sulfur dioxide and the like.

(step 4)

Compound (viii) can be obtained from compound (vi) by the following method.

Compound (vi) is treated in an inert solvent or without solvent with 1 to 20 equivalents, preferably 1 to 5 equivalents, of an acid halogenating agent at a temperature between -20°C and the boiling point of the solvent to be used for 15 min to 48 hr, preferably 1 to 18 hr, to give a sulfonyl halide of compound (vi). In this case, if necessary, 0.01 to 0.5 equivalents of DMF, pyridine and the like may be added. The obtained sulfonyl halide is reacted with 0.5 to 5 equivalents, preferably 1 to 2.5 equivalents, of compound (vii) in an inert solvent in the presence of 1 to 10 equivalents, preferably 1 to 5 equivalents, of a base at a temperature between -20°C and the boiling point of the solvent to be used, preferably between - 10°C and 30°C, for 15 min to 48 hr, preferably 1 to 10 hr, to give compound (viii-b) wherein $R^{17}$ is lower alkyl.

Examples of the inert solvent to be used for the reaction to give a sulfonyl halide include 1,2-dichloroethane, chloroform, methylene chloride, pyridine, THF, DME, toluene, DMF, dioxane, ethyl acetate and the like, and these can be used singly or as a mixture thereof. Among these, 1,2-dichloroethane, methylene chloride or toluene is preferable.

Examples of the acid halogenating agent include thionyl chloride, oxalyl chloride, phosphorus oxychloride and the like.

Examples of the inert solvent to be used for the reaction of sulfonic acid halide and compound (vii) include 1,2-dichloroethane, chloroform, methylene chloride, pyridine, THF, DME, toluene, DMF, dioxane, ethyl acetate and the like, and these can be used singly or as a mixture thereof. Among these, 1,2-dichloroethane, methylene chloride or DMF is preferable.

Examples of the base include pyridine, triethylamine, 4-(dimethylamino)pyridine, N,N-diisopropylethylamine, aqueous sodium hydrogen carbonate solution, aqueous sodium hydroxide solution and the like. Among these, triethylamine is preferable.

Compound (viii-a) wherein $R^{17}$ is a hydrogen atom can be obtained by treating compound (viii-b) wherein $R^{17}$ is lower alkyl in a solvent or without solvent with 0.5 to 50 equivalents of a suitable base at a temperature between -30°C and the boiling point of the solvent to be used, preferably 0°C and the boiling point of the solvent to be used for 5 min to 48 hr.

Examples of the solvent include water, methanol, ethanol, THF, DME, DMF, DMSO, dioxane, acetonitrile, pyridine and the like, and these can be used singly or as a mixture thereof.

Examples of the base include sodium hydroxide, potassium carbonate, sodium hydrogen carbonate, potassium hydroxide, lithium hydroxide, sodium methoxide, potassium ethoxide, lithium iodide/pyridine and the like. Among these, lithium hydroxide or lithium iodide/pyridine is preferable.

Also, for example, compound (I-a) which is compound (I) wherein $R^3$ is $-SO_3H$, and compound (I-b) which is compound (I) wherein $R^3$ is $-S(O)_2R^{13j}$ (wherein, $R^{13j}$ represents lower alkoxy optionally having substituent(s), the lower alkoxy is as defined for the above lower alkoxy, and the substituent of the substituted lower alkoxy is as defined above) can be produced according to the following reaction.

After compound A, which the 3-position substituent ($-SO_3H$) of compound (vi) is protected, is produced, compound A is reacted in the same manner as in step 5 to give compound B wherein the 6-position substituent ($COOR^{16}$) of compound A is conversed into amide, and then, the substituent of 3-position of compound B (protected $-SO_3H$ group) is deprotected to give compound (I-a).

According to the former paragraph of step 4, a sulfonyl halide of compound (vi) is obtained and reacted with lower alcohol optionally having substituent(s) to give compound C which is compound (vi) wherein the substituent of 3-position is $-S(O)_2R^{13j}$ (wherein, $R^{13j}$ is as defined above), and then, compound C is reacted in the same manner as in step 5 to give compound (I-b).

In the production of compound (I-a), the protection and deprotection of the substituent of 3-position can be performed by, for example, the method described in the above "Protective groups in Organic Synthesis, third edition, T. W. Greene, John Wiley& Sons Inc. 1999" and the like, and the like.

(step 5)

Compound (I-1) can be obtained by reacting compound (viii-a) or compound (viii-b) with compound (ix) according to the following <Method 1> - <Method 4>.

<Method 1>

Compound (viii-a) wherein $R^{17}$ is a hydrogen atom is treated in an inert solvent or without solvent with 1 to 20 equivalents, preferably 1 to 5 equivalents, of an acid halogenating agent at a temperature between -20°C and the boiling point of the solvent to be used, preferably between - 10°C and 30°C, for 15 min to 48 hr, preferably 1 to 18 hr, to give an acid halide of compound (viii-a). In this case, if necessary, 0.01 to 0.5 equivalent of DMF, pyridine and the like may be added. The obtained acid halide is reacted in an inert solvent in the presence of 1 to 5 equivalents, preferably 1 to 3 equivalents, of a base with 0.5 to 2 equivalents, preferably 1 to 1.2 equivalents, of compound (ix) at a temperature between -20°C and the boiling point of the solvent to be used, preferably between -10°C and 30°C, for 15 min to 48 hr, preferably 1 to 10 hr, to give compound (I-1).

Examples of the inert solvent to be used in the reaction to obtain acid halide include chloroform, methylene chloride, pyridine, THF, DME, toluene, DMF, dioxane, ethyl acetate and the like, and these can be used singly or as a mixture thereof. Among these, methylene chloride or toluene is preferable.

Examples of the acid halogenating agent include thionyl chloride, oxalyl chloride and the like.

Examples of the inert solvent to be used for the reaction of acid halide with compound (ix) include chloroform, methylene chloride, pyridine, THF, DME, toluene, DMF, dioxane, ethyl acetate and the like, and these can be used singly or as a mixture thereof. Among these, methylene chloride or DMF is preferable.

Examples of the base include pyridine, triethylamine, 4-(dimethylamino)pyridine, N,N-diisopropylethylamine, aqueous sodium hydrogen carbonate solution, aqueous sodium hydroxide solution and the like. Among these, triethylamine is preferable.

<Method 2>

Compound (viii-a) wherein $R^{17}$ is a hydrogen atom is reacted in an inert solvent or without solvent in the presence of 1 to 5 equivalents, preferably 1 to 3 equivalents, of a base with 1 to 20 equivalents, preferably 1 to 5 equivalents, of a reagent for synthesizing mixed acid anhydride at a temperature between -30°C and 40°C, preferably between -30°C and 0°C, for 5 min to 24 hr, preferably 10 min to 2 hr, to give a mixed acid anhydride of compound (viii-a). The obtained mixed acid anhydride is reacted with 0.5 to 2 equivalents, preferably 1 to 1.2 equivalents, of compound (ix) at a temperature between - 30°C and 40°C, preferably between -30°C and 30°C, for 5 min to 24 hr, preferably 10 min to 2 hr, to give compound (I-1).

Examples of the inert solvent include chloroform, methylene chloride, pyridine, THF, DME, toluene, DMF, dioxane, acetonitrile, ethyl acetate and the like, and these can be used singly or as a mixture thereof. Among these, THF, DMF or acetonitrile is preferable.

Examples of the base include pyridine, triethylamine, 4-(dimethylamino)pyridine, N-methylmorpholine, N,N-diisopropylethylamine and the like. Among these, N-methylmorpholine or triethylamine is preferable.

Examples of the reagent for synthesizing mixed acid anhydride include isobutyl chloroformate, ethyl chloroformate, pivaloyl chloride, tosyl chloride, mesyl chloride and the like. Among these, isobutyl chloroformate or mesyl chloride

is preferable.

<Method 3>

Compound (viii-a) wherein $R^{17}$ is a hydrogen atom is reacted in an inert solvent or without solvent, in the presence or absence of 1 to 20 equivalents, preferably 1 to 10 equivalents of a base, in the presence of 1 to 20 equivalents, preferably 1 to 5 equivalents, of a condensing agent, with 0.5 to 10 equivalents, preferably 0.5 to 5 equivalents, of compound (ix) at a temperature between -30°C and 60°C, preferably between -30°C and 40°C, for 30 min to 72 hr, preferably 1 to 18 hr to give compound (I-1). In this case, 0.5 to 2 equivalents of, for example, 1-hydroxybenzotriazole (HOBt) or hydrate thereof ($HOBt{\cdot}H_2O$), or 3-hydroxy-4-oxo-3,4-dihydro-1,2,3- benzotriazine or the like may be added.
Examples of the inert solvent include chloroform, methylene chloride, pyridine, THF, DME, toluene, DMF, DMSO, dioxane, acetonitrile, ethyl acetate and the like, and these can be used singly or as a mixture thereof. Among these, methylene chloride, DMF, THF or acetonitrile is preferable.
Examples of the base include pyridine, triethylamine, 4-(dimethylamino)pyridine, N-methylmorpholine, N,N-diisopropylethylamine and the like. Among these, N,N-diisopropylethylamine or triethylamine is preferable.
Examples of the condensing agent include 1,3-dicyclohexylcarbodiimide, 1-ethyl-3-[3-(dimethylamino)propyl]carbodiimide or hydrochloride thereof, 1,1'-carbonyldiimidazole, 1,3-diisopropylcarbodiimide, diphenylphosphoryl azide, 2-chloro-1-methylpyridinium iodide, 1-benzotriazolyl mesylate, 1-benzotriazolyl tosylate, 1-benzotriazolyl benzenesulfonate, benzotriazol-1-yloxytripyrrolidinophosphonium hexafluorophosphate, 1-propylphosphonic anhydride cyclic trimer and the like. Among these, 1,1'-carbonyldiimidazole or benzotriazol-1-yloxytripyrrolidinophosphonium hexafluorophosphate is preferable.

<Method 4>

Compound (viii-b) wherein $R^{17}$ is lower alkyl is reacted in an inert solvent or without solvent, with 1 to 20 equivalents of compound (ix) in the presence of 1 to 10 equivalents of an organometallic compound at a temperature between -78°C and the boiling point of the solvent to be used, preferably between -30°C and the boiling point of the solvent to be used for 15 min to 48 hr, preferably 1 to 18 hr, to give compound (I-1).
Examples of the inert solvent include THF, diethyl ether, toluene, benzene, hexane, pentane and the like, and these can be used singly or as a mixture thereof.
Examples of the organometallic compound include n-butyllithium, sec-butyllithium, trimethylaluminum and the like. Among these, n-butyllithium or trimethylaluminum is preferable.
Compound (I-1c) wherein $R^{13b}$ is a hydrogen atom can also be obtained by treating compound (I-1b) wherein $R^{13b}$ is tert-butyl in an inert solvent or without solvent, with 0.5 to 50 equivalents or a solvent amount of an acid at a temperature between -30°C and the boiling point of the solvent to be used, preferably between 0°C and the boiling point of the solvent to be used, for 5 min to 48 hr. In this case, 0.5 to 3 equivalents of p-anisole or triethylsilane may be added.
Examples of the inert solvent include chloroform, methylene chloride, 1,2-dichloroethane, toluene, dioxane, ethyl acetate and the like, and these can be used singly or as a mixture thereof. Among these, methylene chloride or 1,2-dichloroethane is preferable.
Examples of the acid include hydrochloric acid, sulfuric acid, trifluoroacetic acid, acetic acid and the like. Among these, trifluoroacetic acid is preferable.

(step 6)

Compound (I-2) can be obtained by reacting compound (I-1c) wherein $R^{13b}$ is a hydrogen atom with compound (x) (the following <Method 1> - <Method 4>).

<Method 1>

Compound (I-1c) is reacted in an inert solvent or without solvent, in the presence of 1 to 20 equivalents, preferably 1 to 5 equivalents, of a base, with 1 to 20 equivalents, preferably 1 to 5 equivalents, of compound (x) wherein $X^2$ is a chlorine atom or a bromine atom, at a temperature between -20°C and the boiling point of the solvent to be used, preferably between -10°C and 50°C, for 15 min to 48 hr, preferably 1 to 18 hr, to give compound (I-2).
Examples of the inert solvent include chloroform, methylene chloride, pyridine, THF, DME, toluene, DMF, dioxane, ethyl acetate and the like, and these can be used singly or as a mixture thereof. Among these, pyridine, methylene chloride or DMF is preferable.
Examples of the base include pyridine, triethylamine, 4-(dimethylamino)pyridine, 2,6-lutidine, N,N-diisopropylethylamine, aqueous sodium hydrogen carbonate solution, aqueous sodium hydroxide solution and the like. Among

these, triethylamine, pyridine or 4-(dimethylamino)pyridine is preferable.

<Method 2>

Compound (x) wherein $X^2$ is hydroxy is treated in an inert solvent or without solvent, with 1 to 20 equivalents, preferably 1 to 5 equivalents, of an acid halogenating agent at a temperature between -20°C and the boiling point of the solvent to be used, preferably between -10°C and 30°C, for 15 min to 48 hr, preferably 1 to 18 hr to give acid halide of compound (x). In this case, if necessary, 0.01 to 0.5 equivalents of DMF, pyridine and the like may be added. The obtained acid halide is reacted in an inert solvent, in the presence of 1 to 20 equivalents, preferably 1 to 5 equivalents, of a base, with 0.5 to 2 equivalents, preferably 1 to 1.2 equivalents, of compound (I-1c) at a temperature between -20°C and the boiling point of the solvent to be used, preferably between -10°C and 50°C, for 15 min to 48 hr, preferably 1 to 18 hr, to give compound (I-2).

Examples of the inert solvent to be used for the reaction to obtain acid halide include chloroform, methylene chloride, pyridine, THF, DME, toluene, DMF, dioxane, ethyl acetate and the like, and these can be used singly or as a mixture thereof. Among these, methylene chloride or toluene is preferable.

Examples of the acid halogenating agent include thionyl chloride, oxalyl chloride and the like.

Examples of the inert solvent to be used for the reaction of acid halide and compound (I-1c) include chloroform, methylene chloride, pyridine, THF, DME, toluene, DMF, dioxane, ethyl acetate and the like, and these can be used singly or as a mixture thereof. Among these, pyridine, methylene chloride or DMF is preferable.

Examples of the base include pyridine, triethylamine, 4-(dimethylamino)pyridine, 2,6-lutidine, N,N-diisopropylethylamine, aqueous sodium hydrogen carbonate solution, aqueous sodium hydroxide solution and the like. Among these, triethylamine, pyridine or 4-(dimethylamino)pyridine is preferable.

<Method 3>

Compound (x) wherein $X^2$ is hydroxy is reacted in an inert solvent or without solvent, in the presence of 1 to 5 equivalents, preferably 1 to 3 equivalents, of a base, with 1 to 20 equivalents, preferably 1 to 5 equivalents, of a reagent for synthesizing mixed acid anhydride at a temperature between -30°C and 40°C, preferably between -30°C and 0°C for 5 min to 24 hr, preferably 10 min to 2 hr, to give a mixed acid anhydride of compound (x). The obtained mixed acid anhydride is reacted with 0.5 to 2 equivalents, preferably 1 to 1.2 equivalents, of compound (I-1c) at a temperature between -30°C and 40°C, preferably between -30°C and 30°C, for 5 min to 24 hr, preferably 10 min to 2 hr, to give compound (I-2).

Examples of the inert solvent include chloroform, methylene chloride, pyridine, THF, DME, toluene, DMF, dioxane, acetonitrile, ethyl acetate and the like, and these can be used singly or as a mixture thereof. Among these, THF, DMF or acetonitrile is preferable.

Examples of the base include pyridine, triethylamine, 4-(dimethylamino)pyridine, N-methylmorpholine, N,N-diisopropylethylamine and the like. Among these, N-methylmorpholine or triethylamine is preferable.

Examples of the reagent for synthesizing mixed acid anhydride include isobutyl chloroformate, ethyl chloroformate, pivaloyl chloride, tosyl chloride, mesyl chloride and the like. Among these, isobutyl chloroformate or mesyl chloride is preferable.

<Method 4>

Compound (x) wherein $X^2$ is hydroxy is reacted in an inert solvent or without solvent in the presence or absence of 1 to 20 equivalents, preferably 1 to 10 equivalents, of a base, in the presence of 1 to 20 equivalents, preferably 1 to 5 equivalents, of a condensing agent, with 0.3 to 20 equivalents, preferably 0.1 to 10 equivalents, of compound (I-1c) at a temperature between -30°C and 100°C, preferably between 0°C and 80°C, for 30 min to 72 hr, preferably 1 to 30 hr, to give compound (I-2). In this case, 0.5 to 2 equivalents of, for example, HOBt, $HOBt{\cdot}H_2O$, 3-hydroxy-4-oxo-3,4-dihydro-1,2,3-benzotriazine and the like may be added.

Examples of the inert solvent include chloroform, methylene chloride, pyridine, THF, DME, toluene, DMF, DMSO, dioxane, acetonitrile, ethyl acetate and the like, and these can be used singly or as a mixture thereof. Among these, methylene chloride, DMF, THF or acetonitrile is preferable.

Examples of the base include pyridine, triethylamine, 4-(dimethylamino)pyridine, N-methylmorpholine, N,N-diisopropylethylamine, DBU, DBN and the like. Among these, DBU, N,N-diisopropylethylamine or triethylamine is preferable.

Examples of the condensing agent include 1,3-dicyclohexylcarbodiimide, 1-ethyl-3-[3-(dimethylamino)propyl]carbodiimide or hydrochloride thereof, 1,1'-carbonyldiimidazole, 1,3-diisopropylcarbodiimide, diphenylphosphoryl azide, 2-chloro-1-methylpyridinium iodide, 1-benzotriazolyl mesylate, 1-benzotriazolyl tosylate, 1-benzotriazolyl benzenesulfonate, benzotriazol-1-yloxytripyrrolidinophosphonium hexafluorophosphate, 1-propylphosphonic anhydride cyclic trimer and the like. Among these, 1-ethyl-3-[3-(dimethylamino)propyl]carbodiimide or hydrochloride thereof or 1,1'-carbonyldiimidazole is preferable.

Production method 2

[Chemical Formula 20]

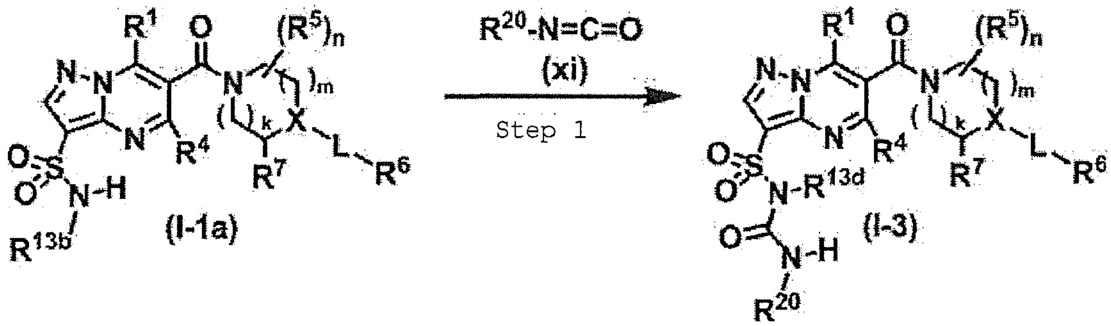


[wherein, $R^1$, $R^4$, $R^5$, $R^6$, $R^7$, $R^{13b}$, $R^{13d}$, X, L, k, m and n are each as defined above, $R^{20}$ represents lower alkyl optionally having substituent(s) (the lower alkyl is as defined above, and the substituent of the substituted lower alkyl is as defined above) or aryl optionally having substituent(s) (the aryl is as defined above, and the substituent of the substituted aryl is as defined above)]

The conversion reaction for each of $R^1$, $R^4$, $R^5$, $R^6$, $R^7$, $R^{13b}$, $R^{13d}$ and $R^{20}$ can be performed during step 1 of this production method.

(step 1)

Compound (I-3) can be obtained by reacting compound (I-1a) which is compound (I-1) wherein $R^{13c}$ is a hydrogen atom in an inert solvent or without solvent, in the presence of 1 to 20 equivalents, preferably 1 to 5 equivalents, of a base, with 0.5 to 50 equivalents, preferably 3 to 20 equivalents, of compound (xi) or an equivalent thereof at a temperature between -30°C and 150°C for 15 min to 48 hr.

Examples of the inert solvent include chloroform, methylene chloride, THF, DME, toluene, DMF, dioxane, ethyl acetate, acetone, methyl ethyl ketone and the like, and these can be used singly or as a mixture thereof. Among these, dioxane, acetone or methylethyl ketone is preferable.

Examples of the base include pyridine, triethylamine, 4-(dimethylamino)pyridine, 2,6-lutidine, N,N-diisopropylethylamine, sodium carbonate, potassium carbonate and the like. Among these, potassium carbonate or N,N-diisopropylethylamine is preferable.

The following compounds (I-4), (I-5) and (I-6) can be obtained using compound (XX), which is the corresponding cyclic amine

[Chemical Formula 21]

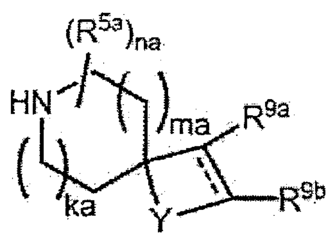

(XX)

(wherein, ---, $R^{5a}$, $R^{9a}$, $R^{9b}$, Y, ka, ma and na are each as defined above), and by a method similar to the above-mentioned production methods 1 and 2.

[Chemical Formula 22]

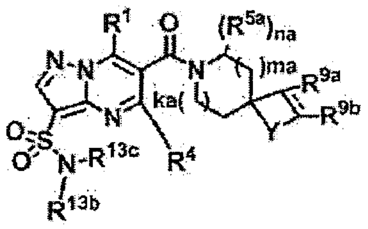

(I-4)

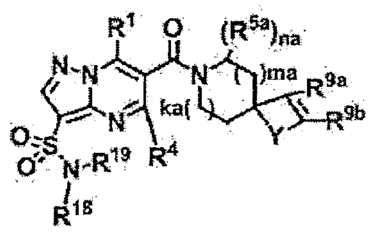

(I-5)

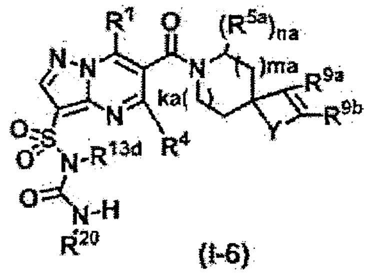

(I-6)

(wherein ---, $R^1$, $R^4$, $R^{5a}$, $R^{9a}$, $R^{9b}$, $R^{13b}$, $R^{13c}$, $R^{13d}$, $R^{18}$, $R^{19}$, $R^{20}$, Y, ka, ma and na are each as defined above)

The following Compounds (I-9), (I-10) and (I-11) can be obtained using compound (XXX), which is the corresponding amine

[Chemical Formula 23]

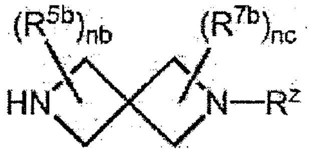

(XXX)

(wherein, $R^z$, $R^{5b}$, $R^{7b}$, nb and nc are each as defined above), and by a method similar to the above-mentioned production methods 1 and 2.

[Chemical Formula 24]

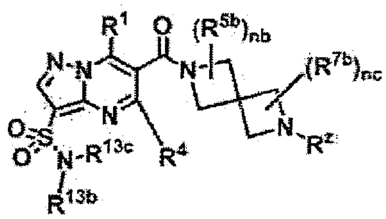

(I-9)

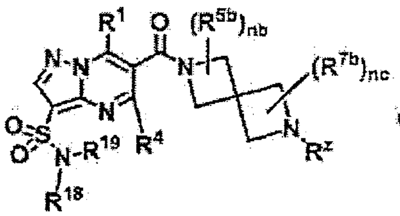

(I-10)

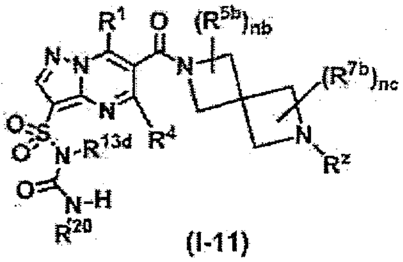

(I-11)

(wherein, $R^1$, $R^4$, $R^{5b}$, $R^{7b}$, $R^z$, $R^{13b}$, $R^{13c}$, $R^{13d}$, $R^{18}$, $R^{19}$, $R^{20}$, nb and nc are each as defined above)

Each functional group in compound (I) and (IA) can also be converted by a known method [for example, the method described in Comprehensive Organic Transformations 2nd edition, R.C. Larock, Vch Verlagsgesellschaft Mbh, 1999 and the like] or modified methods thereto.

The intermediates and the desired compounds in the above-mentioned respective production methods can be isolated and purified by applying separation and purification methods usually used in the synthetic organic chemistry such as filtration, extraction, washing, drying, concentration, recrystallization, various chromatographies and the like. In addition, intermediates can also be subjected to a next reaction without particular purification.

In order to obtain a salt of compound (I) and (IA), when compound (I) or (IA) are obtained in the form of a salt, it can be directly purified. When it is obtained in a free form, compound (I) or (IA) may be dissolved or suspended in a suitable solvent, and an acid or base is added thereto to form a salt, which may be isolated and purified.

Specific examples of compounds (I) and (IA) used by the present invention are shown in Table 1-1 to 1-5, 2-1 to 2-2, 3, 4-1 to 4-2 and 5. However, the compounds of the present invention are not limited thereto.

[Table 1-1]

Table 1-1

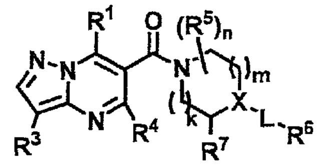

(I-7)

| Comound No. | ●-$R^1$ | 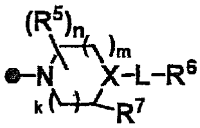 | ●-$R^4$ | ●-$R^3$ |
|---|---|---|---|---|
| a-1 | 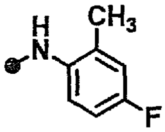 | 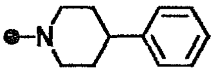 | ●-H | 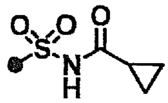 |
| a-2 | 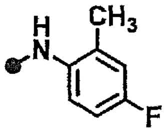 | 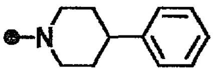 | ●-H | 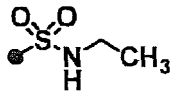 |
| a-3 | 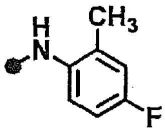 | 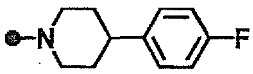 | ●-H | 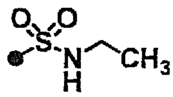 |
| a-4 | 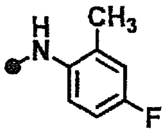 | 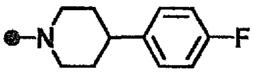 | ●-H | 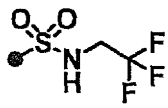 |
| a-5 | 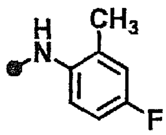 | 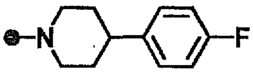 | ●-H | 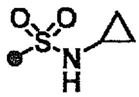 |
| a-6 | 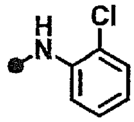 | 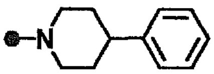 | ●-H | 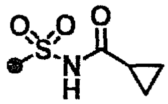 |
| a-7 | 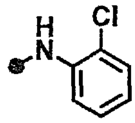 | 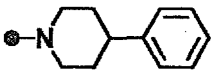 | ●-H | 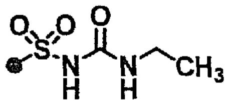 |
| a-8 | 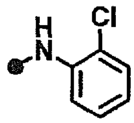 | 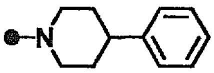 | ●-H | 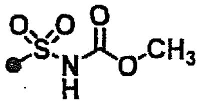 |
| a-9 | 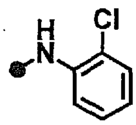 | 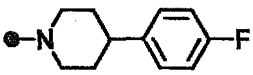 | ●-H | 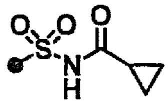 |
| a-10 | 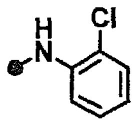 | 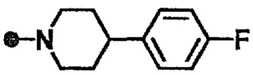 | ●-H | 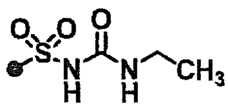 |

[Table 1-2]

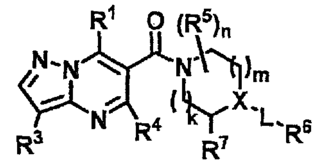

(I-7)

Table 1-2

| Comound No. | ●-$R^1$ | 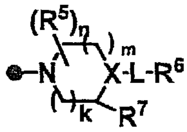 | ●-$R^4$ | ●-$R^3$ |
|---|---|---|---|---|
| **a-11** | 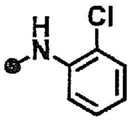 | 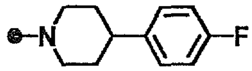 | **●-H** | 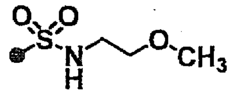 |
| **a-12** | 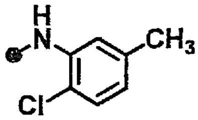 | 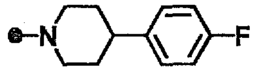 | **●-H** | 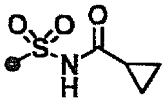 |
| **a-13** | 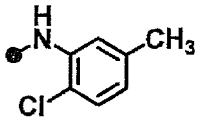 | 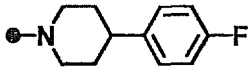 | **●-H** | 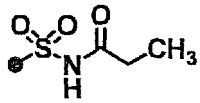 |
| **a-14** | 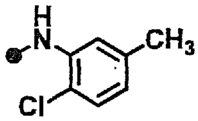 | 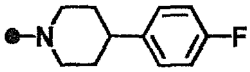 | **●-H** | 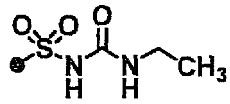 |
| **a-15** | 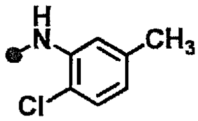 | 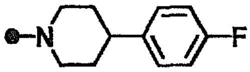 | **●-H** | 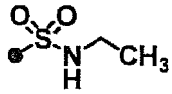 |
| **a-16** | 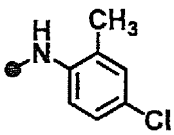 | 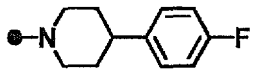 | **●-H** | 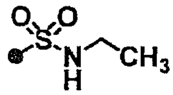 |
| **a-17** | 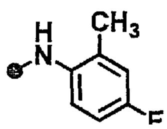 | 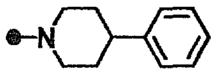 | **●-H** | 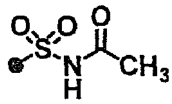 |
| **a-18** | 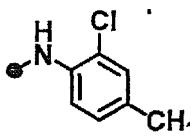 | 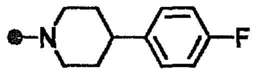 | **●-H** | 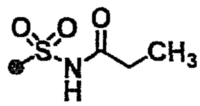 |
| **a-19** | 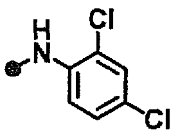 | 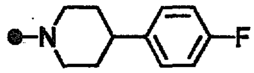 | **●-H** | 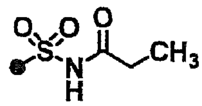 |
| **a-20** | 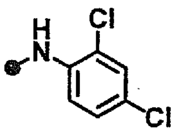 | 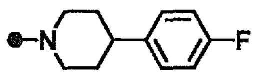 | **●-H** | 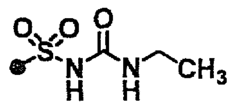 |

[Table 1-3]

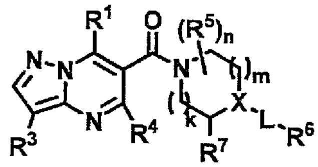

(I-7)

Table 1-3

| Comound No. | ●-$R^1$ | 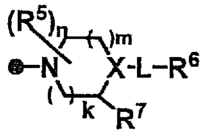 | ●-$R^4$ | ●-$R^3$ |
|---|---|---|---|---|
| **a-21** | 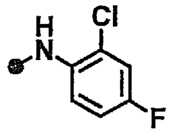 | 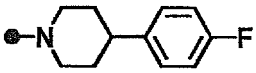 | ●-H | 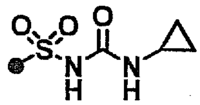 |
| **a-22** | 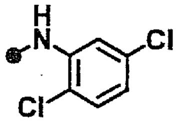 | 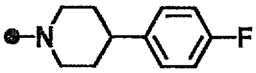 | ●-H | 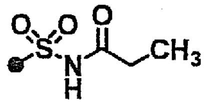 |
| **a-23** | 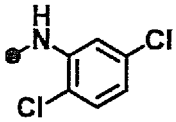 | 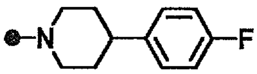 | ●-H | 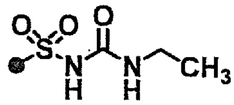 |
| **a-24** | 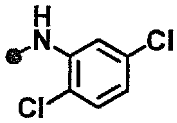 | 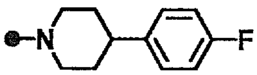 | ●-H | 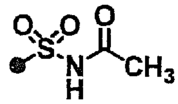 |
| **a-25** | 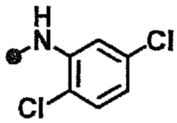 | 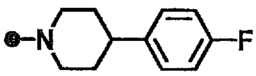 | ●-H | 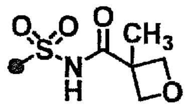 |
| **a-26** | 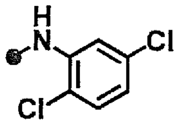 | 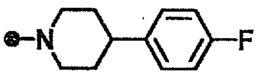 | ●-H | 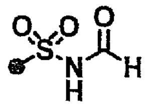 |
| **a-27** | 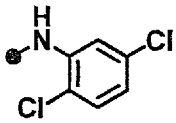 | 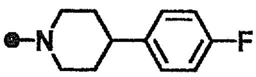 | ●-H | 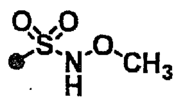 |
| **a-28** | 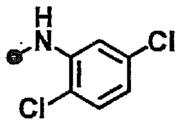 | 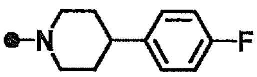 | ●-H | 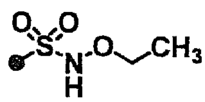 |
| **a-29** | 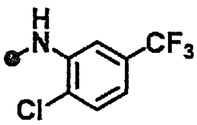 | 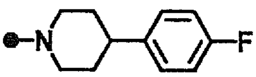 | ●-H | 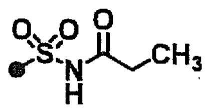 |
| **a-30** | 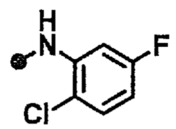 | 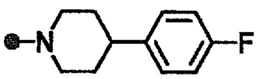 | ●-H | 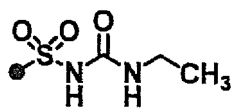 |

[Table 1-4]

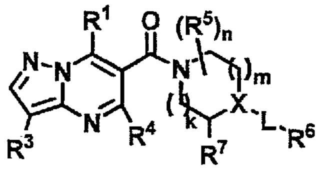

(I-7)

Table 1-4

| Comound No. | ●-$R^1$ | 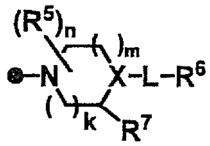 | ●-$R^4$ | ●-$R^3$ |
|---|---|---|---|---|
| a-31 | 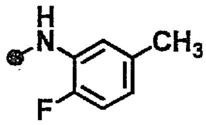 | 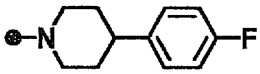 | ●-H | 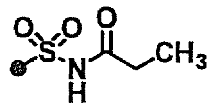 |
| a-32 | 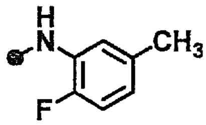 | 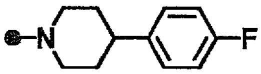 | ●-H | 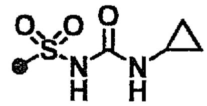 |
| a-33 | 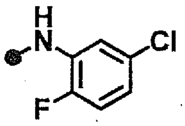 | 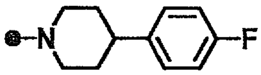 | ●-H | 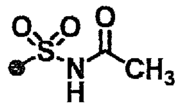 |
| a-34 | 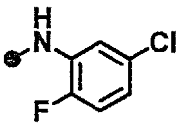 | 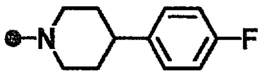 | ●-H | 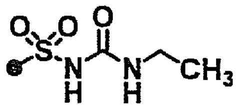 |
| a-35 | 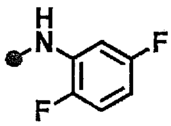 | 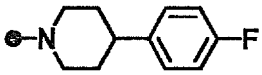 | ●-H | 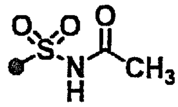 |
| a-36 | 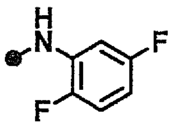 | 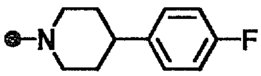 | ●-H | 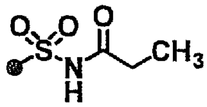 |
| a-37 | 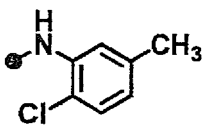 | 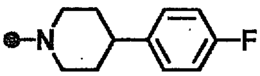 | ●-H | 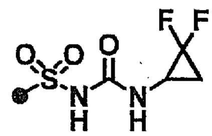 |
| a-38 | 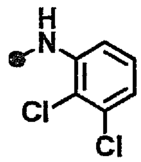 | 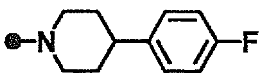 | ●-H | 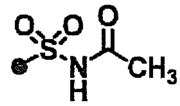 |
| a-39 | 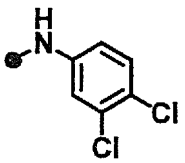 | 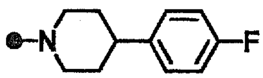 | ●-H | 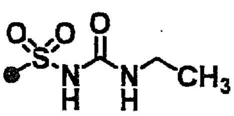 |

[Table 1-5]

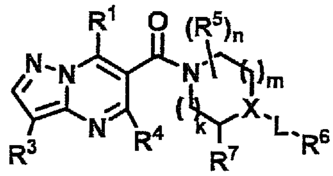

(I-7)

Table 1-5

| Comound No. | ●-$R^1$ | 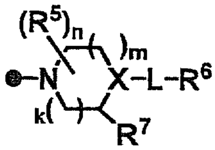 | ●-$R^4$ | ●-$R^3$ |
|---|---|---|---|---|
| **a-40** | 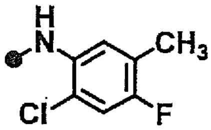 | 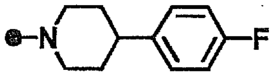 | ●-H | 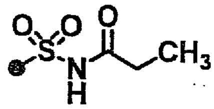 |
| **a-41** | 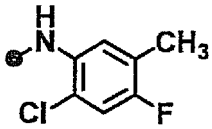 | 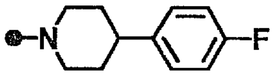 | ●-H | 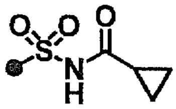 |
| **a-42** | 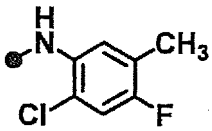 | 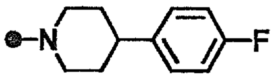 | ●-H | 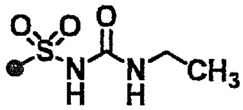 |

[Table 2-1]

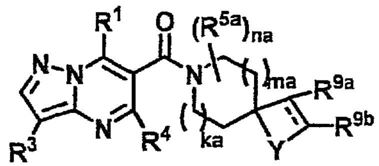

(I-8) (I-8)

Table 2-1

| Comound No. | ●-R$^1$ | 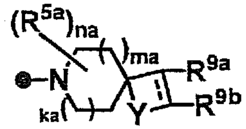 | ●-R$^4$ | ●-R$^3$ |
|---|---|---|---|---|
| a-43 | 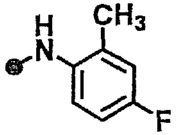 | 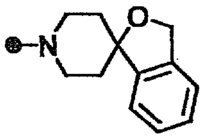 | ●-H | 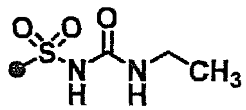 |
| a-44 | 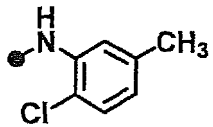 | 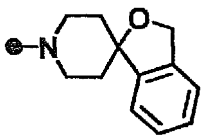 | ●-H | 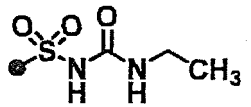 |
| a-45 | 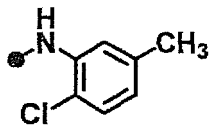 | 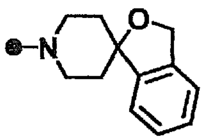 | ●-H | 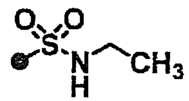 |
| a-46 | 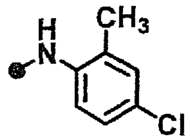 | 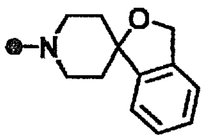 | ●-H | 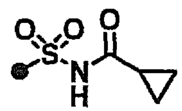 |
| a-47 | 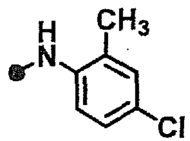 | 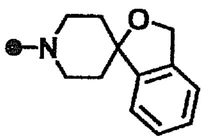 | ●-H | 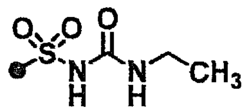 |

(continued)

| Comound No. | ●-R1 | 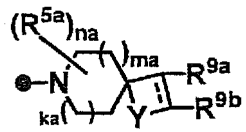 | ●-R4 | ●-R3 |
|---|---|---|---|---|
| a-48 | 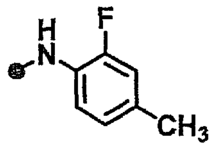 | 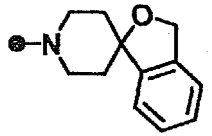 | ●-H | 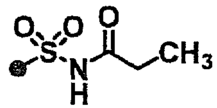 |
| a-49 | 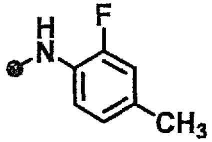 | 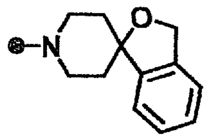 | ●-H | 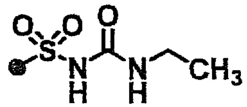 |
| a-50 | 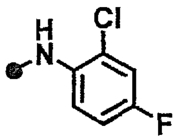 | 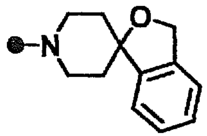 | ●-H | 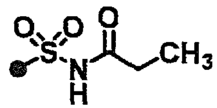 |

[Table 2-2]

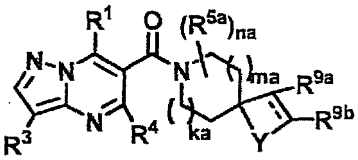

(I-8)

Table 2-2

| Comound No. | ●-$R^1$ | 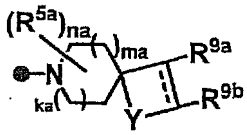 | ●-$R^4$ | ●-$R^3$ |
|---|---|---|---|---|
| a-51 | 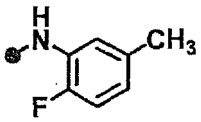 | 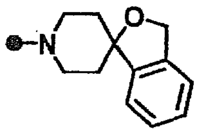 | ●-H | 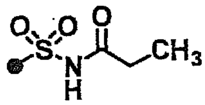 |
| a-52 | 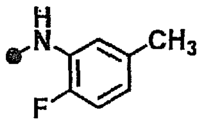 | 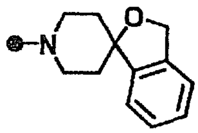 | ●-H | 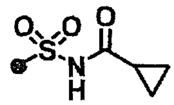 |
| a-53 | 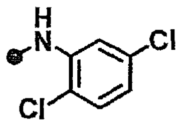 | 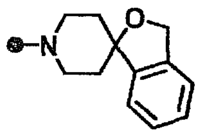 | ●-H | 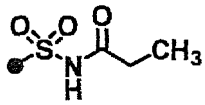 |
| a-54 | 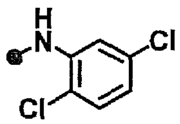 | 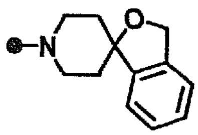 | ●-H | 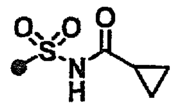 |
| a-55 | 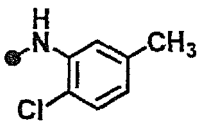 | 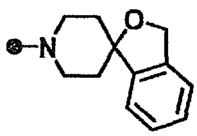 | ●-H | 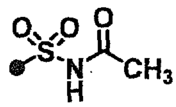 |
| a-56 | 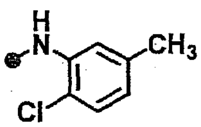 | 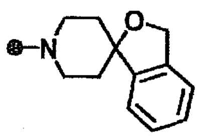 | ●-H | 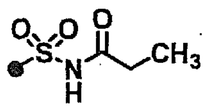 |
| a-57 | 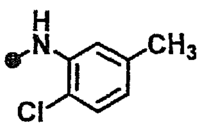 | 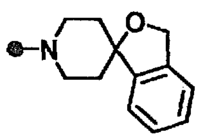 | ●-H | 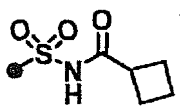 |
| a-58 | 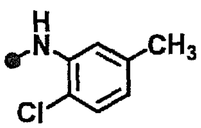 | 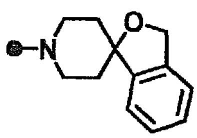 | ●-H | 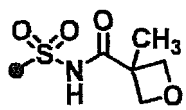 |
| a-59 | 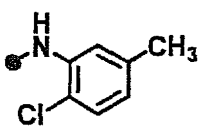 | 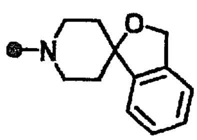 | ●-H | 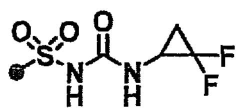 |

[Table 3]

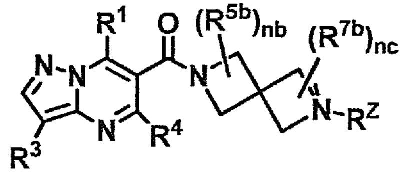

(I-12)

Table 3

| Comound No. | ●-$R^1$ | 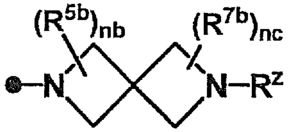 | ●-$R^4$ | ●-$R^3$ |
|---|---|---|---|---|
| **a-60** | 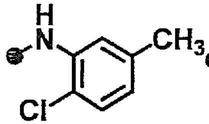 | 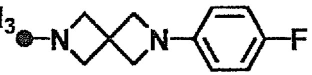 | ●-H | 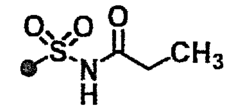 |
| **a-61** | 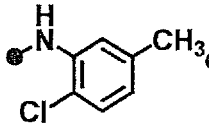 | 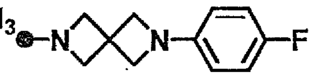 | ●-H | 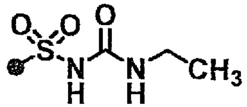 |

[Table 4-1]

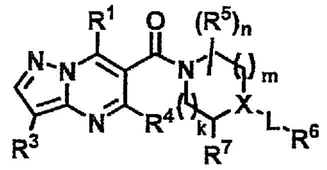

(I-7)

Table 4-1

| Comound No. | ●-$R^1$ | 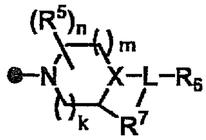 | ●-$R^4$ | ●-$R^3$ |
|---|---|---|---|---|
| a-62 | 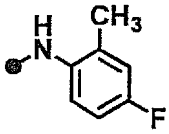 | 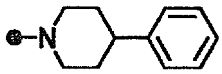 | ●-H | 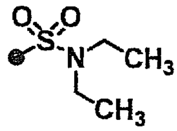 |
| a-63 | 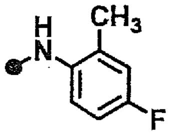 | 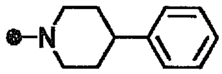 | ●-H | 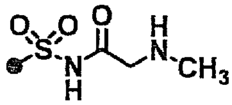 |
| a-64 | 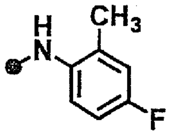 | 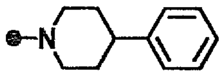 | ●-H | 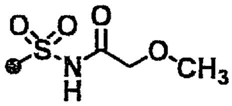 |
| a-65 | 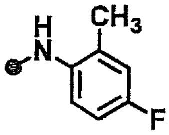 | 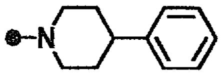 | ●-H | 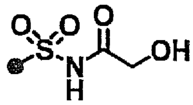 |
| a-66 | 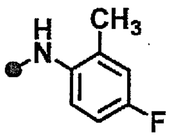 | 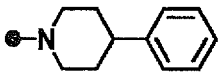 | ●-H | 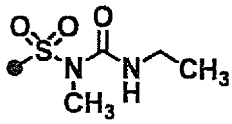 |
| a-67 | 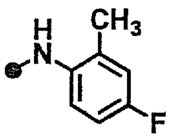 | 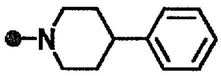 | ●-H | 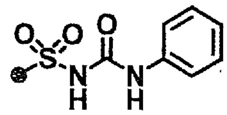 |
| a-68 | 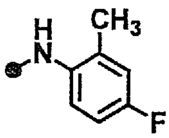 | 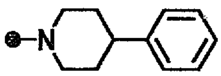 | ●-H | 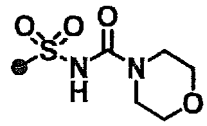 |
| a-69 | 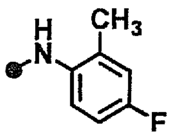 | 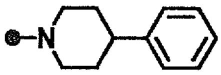 | ●-H | 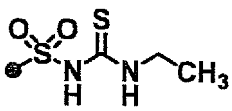 |
| a-70 | 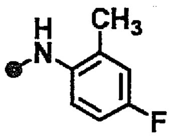 | 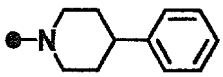 | ●-H | 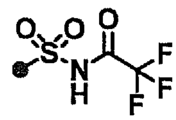 |

(continued)

| Comound No. | ●-$R^1$ | 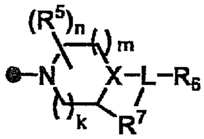 | ●-$R^4$ | ●-$R^3$ |
|---|---|---|---|---|
| a-71 | 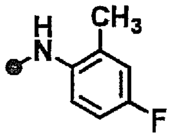 | 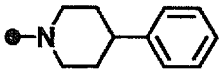 | ●-H | 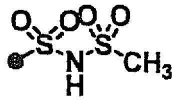 |

[Table 4-2]

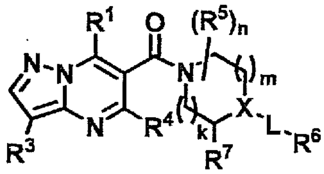

(I-7)

Table 4-2

| Comound No. | ●-$R^1$ | 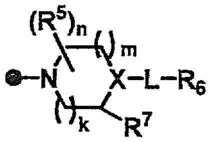 | ●-$R^4$ | ●-$R^3$ |
|---|---|---|---|---|
| a-72 | 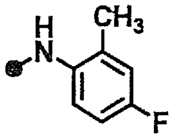 | 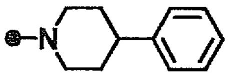 | ●-H | 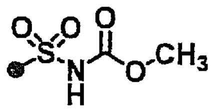 |
| a-73 | 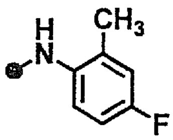 | 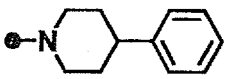 | ●-H | 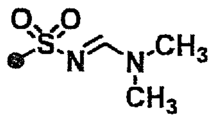 |
| a-74 | 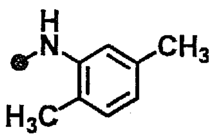 | 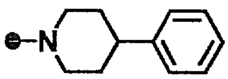 | ●-H | 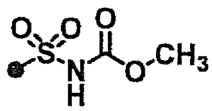 |
| a-75 | 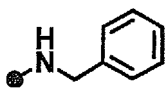 | 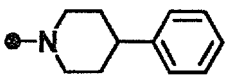 | ●-H | 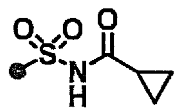 |
| a-76 | 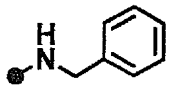 | 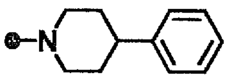 | ●-H | 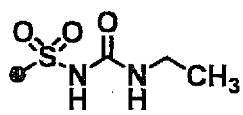 |
| a-77 | 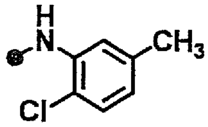 | 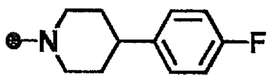 | ●-H | 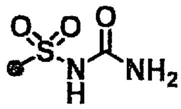 |
| a-78 | 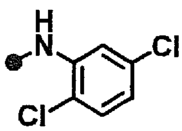 | 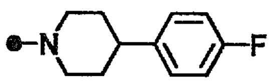 | ●-H | 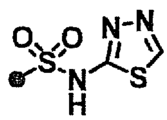 |

(continued)

| Comound No. | ●-$R^1$ | 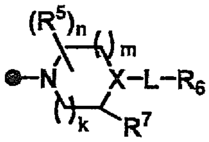 | ●-$R^4$ | ●-$R^3$ |
|---|---|---|---|---|
| a-79 | 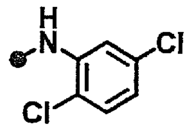 | 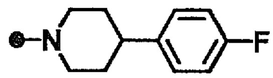 | ●-H | 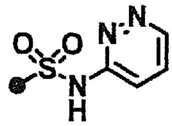 |
| a-80 | 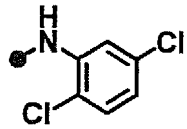 | 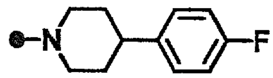 | ●-H | 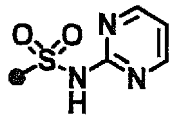 |

[Table 5]

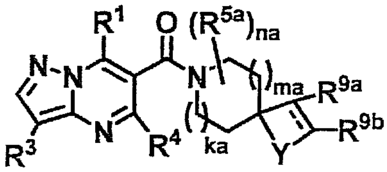 (I-8)

Table 5

| Comound No. | ●-$R^1$ | 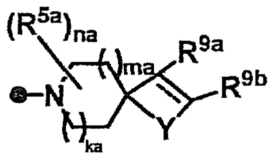 | ●-$R^4$ | ●-$R^3$ |
|---|---|---|---|---|
| a-81 | 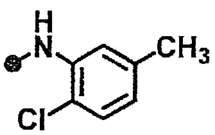 | 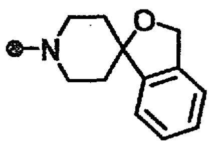 | ●-H | 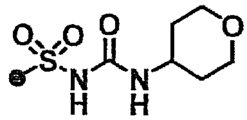 |
| a-82 | 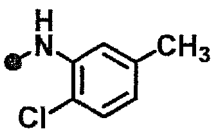 | 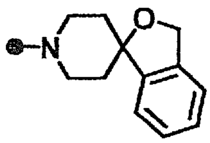 | ●-H | 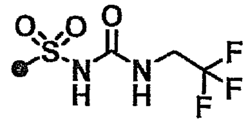 |

Next, the pharmacological effect of the representative compounds is specifically explained by Experimental Examples.

Experimental Example 1: Chemokine receptor activity modulating effect (1)

The antagonistic activity of the test compound for CCR4 is examined by the method of measuring an inhibitory effect on the binding of [$^{125}$I]-TARC to Hut78 cells.

RPMI-1640 medium (manufactured by Sigma-Aldrich Japan) containing 20 mmol/L of 4-(2-hydroxyethyl)-1-piperazineethanesulfonic acid (HEPES; manufactured by NACALAI TESQUE, INC.) and 0.1% [weight (w)/volume (v)%] bovine serum albumin (manufactured by SEIKAGAKU CORPORATION) is adjusted to pH=7.0 with an aqueous sodium hydrogen carbonate solution (manufactured by Wako Pure Chemical Industries, Ltd.) (binding/wash buffer solution). Hut78 cells (ATCC No. TIB-161, purchased from ATCC) are suspended in the obtained binding/wash buffer solution, the suspension (60 μL, 3×10$^5$ cells) is added to each well of a round-bottomed 96 well plate (manufactured by Corning Costar), (1) a test compound solution (20 μL) containing a test compound at each concentration diluted from 10 mmol/L DMSO solution with the binding/wash buffer solution and (2) 810 Bq [$^{125}$I]-TARC (manufactured by Amersham Bioscience) (20 μL) diluted with the binding/wash buffer solution are added to the total amount of 100 μL, and the mixture is reacted

at room temperature for 2 hr.

The reaction products in each well containing the above-mentioned Hut-78 cells are added to each well of a glass filter plate (unifilter GF/B96, manufactured by Packard Bioscience), and are rapidly filtered using Filtermate 196 (manufactured by Packard Bioscience). 0.3% [weight (w)/volume (v)%] polyethyleneimine solution diluted with the binding/wash buffer solution (manufactured by NACALAI TESQUE, INC.) (50 μL) is added to each well of the glass filter plate before use. The glass filter in each well is washed with the binding/wash buffer solution at 4°C, added MicroScinti 20 (manufactured by Packard Bioscience) (50 μL) thereto, and the radioactivity level ([$^{125}$I]-TARC-bound radioactivity level) on the glass filter is measured by Topcount NXT™ (manufactured by Packard Bioscience).

The binding inhibitory rate (%) of the test compound is calculated according to the following formula.

[Formula 1]

$$\text{binding inhibitory rate (\%)} = \frac{\text{total binding amount} - \text{binding amount with addition of test compound}}{\text{total binding amount} - \text{non-specific binding amount}} \times 100$$

total binding amount: [$^{125}$I]-TARC binding radioactivity level when experiment is performed in the absence of test compound. binding amount with addition of test compound: [$^{125}$I]-TARC binding radioactivity level in the presence of test compound. non-specific binding amount: [$^{125}$I]-TARC binding radioactivity level when binding experiment is performed with addition of sufficient amount of unlabeled TARC together with [$^{125}$I]-TART.

As for other chemokine receptors, for example, CCR8, CCR9, CCR10, the antagonistic activity of a test compound for each receptor is measured using each radiolabeled selective ligand.

By the above-mentioned method, it is suggested that a test compound showing an antagonistic activity for chemokine receptors, for example, CCR4, CCR8, CCR9, CCR10, is useful as a prophylactic and/or therapeutic agent for diseases involving each chemokine receptor.

Experimental Example 2: Chemokine receptor activity modulating effect (2)

(1) Preparation of human CCR10-inducible expression plasmid

A CCR10-inducible expression plasmid was produced according to a known method ("Analytical Biochemistry", 2006, vol. 400, page 163). A DNA encoding human CCR10 was obtained by PCR. Using human chromosomal DNA (100 ng; manufactured by Clontech Laboratories, Inc.) as a template, a synthetic DNA having the sequences shown by SEQ ID NOs: 1 and 2 as a human CCR10 cDNA specific primer, and Pyrobest DNA Polymerase (manufactured by TAKARA SHUZO CO. LTD.) as an enzyme, a DNA encoding human CCR10 was obtained by PCR. As a buffer solution for PCR, the buffer solution attached to the enzyme was diluted 10-fold with deionized water and used. Using Thermal Cycler DNA Engine (manufactured by MJ Research), PCR was performed by 35 cycles of reactions composed of an incubation at 90°C for 2 min, at 94°C for 30 seconds, at an anneal temperature of 58°C for 30 seconds, and at 72°C for 1 min.

The amplified PCR fragment was cleaved with HindIII and NotI, then a human CCR10 DNA fragment was recovered by an agarose gel electrophoresis method. The fragment was incorporated in between the corresponding restriction enzyme sites (HindIII-NotI) of an inducible expression vector, whereby a human CCR10-inducible expression plasmid were prepared.

Using a primer specific to the sequence of the plasmid (synthetic DNA having the sequences shown by SEQ ID NOs: 3 and 4), the sequence of human CCR10 DNA region was determined. For the determination of the base sequence, DNA sequencer 377 (manufactured by PerkinElmer) and a reaction kit (ABI Prism (registered trade mark) BigDye (registered trade mark) Terminator Cycle Sequencing Ready Reaction kit: manufactured by Applied Biosystems) were used. The sequence of human CCR10 DNA matched with the sequence (NM_016602) registered in GenBank.

(2) Preparation of human CCR10-expressing cell for calcium assay

Cells for the detection of the signal from human CCR10 by a calcium assay were prepared. Human CCR10-inducible expressing cells whose host cell is KJMGER8 cell (cell line derived from Namalwa cell) were prepared according to a known method ("Analy Biochem", 2006, vol. 400, page 163). Human CCR10-inducible expression plasmid produced

above and Gα16 expression plasmid were co-transfected into KJMGER8 cell by an electroporation method ("Cytotechnology", 1990, vol. 3, page 133), whereby a signal from human CCR10 could be detected by a calcium assay (hereinafter to be referred to as hCCR10G16 cell). Gα16 expression plasmid was produced by incorporating human Gα16 DNA into expression vector pAMoh (WO03/087366). Induced expression of human CCR10 was performed by cultivating hCCR10G16 cells in the presence of 10 nmol/L β-estradiol (manufactured by Sigma Ltd.) for 24 hr.

(3) Calcium assay of human CCR10

Cells expressing human CCR10 induced by the above-mentioned method were suspended in RPMI1640 medium and adjusted to cell density of $1.25\times10^6$ cells/mL. This mixture was dispensed to a 384 well clear bottom plate (manufactured by Corning Incorporated) at 20 μL/well, and these were incubated at 37°C for several dozen minutes. A loading buffer prepared according to the protocol attached to FLIPR Calcium 3 assay kit (manufactured by Molecular Device) was dispensed to this plate at 20 μL/well, and the plate was incubated at 37°C for 60 min. A 33 μmol/L DMSO solution of the test compound was diluted 37-fold with RPMI1640 medium and the resulting solution was added at 5 μL/well, and the mixture was incubated at 37°C for 30 min. 300 nmol/L human recombinant CTACK (manufactured by R and D Systems) diluted with RPMI1640 medium containing 1 w/v% bovine serum albumin was added at 5 μL/well. Change of the intracellular calcium ion concentration for about 5 min after the addition was measured by FLIPR (manufactured by Molecular Device) or FDSS (manufactured by Hamamatsu Photonics K.K.). The difference between the maximum fluorescence intensity and the minimum fluorescence intensity for 5 min was calculated, and taken as the measurement value (maximum fluorescence intensity - minimum fluorescence intensity).

The inhibitory rate of the test compound for increase in calcium ion concentration was calculated according to the following formula.

[Formula 2]

$$\text{inhibitory rate of test compound for increase in calcium ion concentration (\%)} = 1 - \left(\frac{\text{test compound addition group} - \text{blank}}{\text{control} - \text{blank}}\right) \times 100$$

test compound addition group: average measurement value of test compound addition group

control: Average measurement value of group treated as follows. DMSO buffer diluted 37-fold with RPMI1640 medium was added instead of test compound solution.Subsequently, 300 nmol/L CTACK diluted with RPMI1640 medium containing 1 w/v% bovine serum albumin was added, and change in intracellular calcium ion concentration was measured.

blank: Average measurement value of group treated as follows. DMSO diluted 37-fold with RPMI1640 medium was added instead of test compound solution. Subsequently, RPMI1640 medium containing 1 w/v% bovine serum albumin was added instead of medium containing CTACK, and change in intracellular calcium ion concentration was measured.

A test using human recombinant CTACK was performed by the above-mentioned method. Compounds a-1 to a-82 showed inhibitory rate for an increase in calcium ion concentration at a concentration of 100 nmol/L, as shown in Table 6, and showed an antagonistic effect against CCR10.

[Table 6]

| Comound No. | Inhibitory rate (%) | Comound No. | Inhibitory rate (%) | Comound No. | Inhibitory rate (%) |
|---|---|---|---|---|---|
| a-1 | 93.4 | a-29 | >100 | a-56 | >100 |
| a-2 | >100 | a-30 | >100 | a-57 | >100 |
| a-3 | >100 | a-31 | >100 | a-58 | >100 |
| a-4 | >100 | a-32 | >100 | a-59 | >100 |
| a-5 | >100 | a-33 | >100 | a-60 | 90.2 |

(continued)

| Comound No. | Inhibitory rate (%) | Comound No. | Inhibitory rate (%) | Comound No. | Inhibitory rate (%) |
|---|---|---|---|---|---|
| a-6 | 98.9 | a-34 | >100 | a-61 | 24.3 |
| a-7 | 92.4 | a-35 | >100 | a-62 | >100 |
| a-8 | 99.6 | a-36 | >100 | a-63 | 58.8 |
| a-9 | 80.6 | a-37 | >100 | a-64 | 94.2 |
| a-10 | >100 | a-38 | >100 | a-65 | >100 |
| a-11 | 28.9 | a-39 | >100 | a-66 | 72.6 |
| a-12 | >100 | a-40 | >100 | a-67 | 63.7 |
| a-13 | >100 | a-41 | >100 | a-68 | 85.9 |
| a-14 | >100 | a-42 | >100 | a-69 | 81.8 |
| a-15 | 87.8 | a-43 | 92.0 | a-70 | >100 |
| a-16 | >100 | a-44 | 95.2 | a-71 | 88.8 |
| a-17 | >100 | a-45 | 80.3 | a-72 | 90.7 |
| a-18 | 98.3 | a-46 | >100 | a-73 | 68.9 |
| a-19 | 97.0 | a-47 | >100 | a-74 | >100 |
| a-20 | 90.7 | a-48 | >100 | a-75 | 89.4 |
| a-21 | >100 | a-49 | >100 | a-76 | 95.1 |
| a-22 | >100 | a-50 | >100 | a-77 | >100 |
| a-23 | 96.7 | a-51 | >100 | a-78 | >100 |
| a-24 | >100 | a-52 | >100 | a-79 | >100 |
| a-25 | >100 | a-53 | >100 | a-80 | >100 |
| a-26 | >100 | a-54 | >100 | a-81 | 62.3 |
| a-27 | >100 | a-55 | >100 | a-82 | 75.3 |
| a-28 | >100 | | | | |

From the above, it was suggested that compound (I) has a CCR10 antagonistic effect, and is useful as a preventive and/or therapeutic agent for a disease involving CCR10.

Compounds (I) and (IA) and pharmaceutically acceptable salts thereof to be used in the present invention can be administered alone, however usually, they are preferably provided as various pharmaceutical preparations. In addition, such pharmaceutical preparations are used for animals and humans.

The pharmaceutical preparation relating to the present invention can contain, as an active ingredient, compounds (I) or (IA), or a pharmaceutically acceptable salt thereof alone or as a mixture with an active ingredient for any other treatment. Moreover, the pharmaceutical preparation can be produced by mixing the active ingredient with one or more kinds of pharmaceutically acceptable carriers (for example, diluent, solvent, excipient and the like) according to any method well known in the technical field of pharmaceutical science.

As the administration route, a route most effective for the treatment is preferably employed, which may be an oral or parenteral route such as intravenous route, external route or the like.

The dosage form include, for example, tablet, injection, ointment and the like.

Tablet can be produced by using an excipient such as lactose and the like, a disintegrant such as starch and the like, a lubricant such as magnesium stearate and the like, a binder such as hydroxypropylcellulose and the like, and the like, and is suitable for oral administration.

Injection and the like can be produced by using diluents or solvents such as a salt solution, a glucose solution or a mixture of salt solution and a glucose solution and the like, or a solvent and the like.

Ointment can be produced by using a base such as petrolatum and the like, and an additive such as stearyl alcohol and the like.

The dose and administration frequency of compounds (I) and (IA) and a pharmaceutically acceptable salt thereof to be used in the present invention varies depending on the mode of administration, age and body weight of patients, nature and severity of the symptom to be treated and the like, it is generally within the range of 0.01 to 1000 mg, preferably 0.05 to 100 mg, for oral administration to an adult, which is administered at once or in several times a day. In the case of intravenous administration, external administration and the like, 0.001 to 1000 mg, preferably 0.01 to 100 mg, is administered to an adult at once or in several times a day. However, these doses and administration frequencies vary depending on the above various conditions.

The present invention is explained more specifically in the following by Examples and Reference Examples, however the scope of the present invention is not limited to these Examples and Reference Examples.

Also, the proton nuclear magnetic resonance spectrum ($^1$H NMR) used in the Reference Examples were measured at 270 MHz or 300 MHz, and exchanging protons may not be clearly observed depending on the compound and measurement conditions. The indication of the multiplicity of the signals is conventional, where br represents an apparently broad signal.

Examples

[Example 1]

Formulation Example 1 (tablet)

A tablet having the following composition is prepared by a conventional method. Compound a-1 (40 g), lactose (286.8 g) and potato starch (60 g) are mixed, and 10% aqueous solution (120 g) of hydroxypropylcellulose is added thereto. This mixture is kneaded, granulated, dried, and sieved to give granules for tableting by a conventional method. Magnesium stearate (1.2 g) is added thereto, and the mixture is tableted by a tableting machine with a punch having a diameter of 8 mm (manufactured by Kikusui, RT-15) to give tablets (containing 20 mg of active ingredient per tablet).

Formulation

| | |
|---|---|
| compound a-1 | 20 mg |
| lactose | 143.4 mg |
| potato starch | 30 mg |
| hydroxypropylcellulose | 6 mg |
| magnesium stearate | 0.6 mg |
| | 200 mg |

[Example 2]

Formulation Example 2 (injection)

An injection having the following composition was prepared by a conventional method. Compound a-1 (1 g) and D-mannitol (5 g) are added to distilled water for injection, and hydrochloric acid and aqueous sodium hydroxide solution are further added to adjust pH to 6, and the total amount is made 1000 mL with distilled water for injection. The obtained mixture is aseptically filled in a glass vial by 2 mL to give an injection (containing 2 mg of active ingredient per vial). Formulation

| | |
|---|---|
| compound a-1 | 2 mg |
| D-mannitol | 10 mg |
| hydrochloric acid | appropriate amount |
| aqueous sodium hydroxide solution | appropriate amount |
| distilled water for injection | appropriate amount |
| | 2.00 mL |

Reference Example 1: N-[7-(4-Fluoro-2-methylphenylamino)-6-(4-phenylpiperidine-1-carbonyl)pyrazolo[1,5-a]pyrimidin-3-ylsulfonyl]cyclopropanecarboxamide (compound a-1)

(step 1)

Ethyl 7-(4-fluoro-2-methylphenylamino)pyrazolo[1,5-a]pyrimidine-6-carboxylate

Ethyl 7-hydroxypyrazolo[1,5-a]pyrimidine-6-carboxylate (Journal of Medicinal Chemistry, vol. 49, page 2526, 2006) (3.20 g, 15.5 mmol) was dissolved in phosphorus oxychloride (30 mL), N,N-diisopropylethylamine (5.4 mL, 30.9 mmol) was added, and the mixture was stirred with heating at 100°C for 3 hr. After confirmation of consumption the starting materials, phosphorus oxychloride was evaporated under reduced pressure, and the residue was dissolved in ethyl acetate. The solution was added dropwise to a saturated aqueous sodium hydrogen carbonate solution under ice-cooling. To the saturated aqueous sodium hydrogen carbonate solution was added ethyl acetate, and the mixture was extracted 3 times. The combined organic layer was dried over anhydrous magnesium sulfate, and concentrated under reduced pressure.

The obtained residue was dissolved in N,N-dimethylacetamide (30 mL), 4-fluoro-2-methylaniline (2.2 mL, 20.1 mmol) and N,N-diisopropylethylamine (5.4 mL, 30.9 mmol) were added, and the mixture was stirred with heating at 80°C for 2 hr. After cooling, to the reaction mixture was added dropwise 5% aqueous citric acid solution, chloroform was added, and the mixture was extracted 3 times. The combined organic layer was dried over anhydrous magnesium sulfate, and concentrated under reduced pressure. The obtained residue was purified by silica gel column chromatography (hexane/ethyl acetate=2/1) to give the title compound (3.14 g, 65%).
ESI-MS m/z: 315 $(M+H)^+$; $^1H$-NMR ($CDCl_3$, $\delta$): 1.42 (t, J = 7.2 Hz, 3H), 2.22 (s, 3H), 4.38 (q, J = 7.2 Hz, 2H), 6.46 (d, J = 2.3 Hz, 1H), 6.55-7.09 (m, 3H), 7.83 (d, J = 2.3 Hz, 1H), 8.83 (s, 1H), 10.69 (s, 1H).

(step 2)

6-Ethoxycarbonyl-7-(4-fluoro-2-methylphenylamino)pyrazolo[1,5-a]pyrimidine-3-sulfonic acid

Ethyl 7-(4-fluoro-2-methylphenylamino)pyrazolo[1,5-a]pyrimidine-6-carboxylate (0.500 g, 1.59 mmol) obtained in step 1 was dissolved in methylene chloride (6 mL), chlorosulfonic acid (0.21 mL, 3.19 mmol) was added, and the mixture was stirred at room temperature overnight. The reaction mixture was concentrated under reduced pressure, a 1/1 mixed solvent of diisopropyl ether and 2-propanol was added, and the mixture was stirred at room temperature for 1 hr. The resulting crystals were suction filtered and washed with 2-propanol to give the title compound (0.580 g, 92%).
ESI-MS m/z: 395 $(M+H)^+$; $^1H$-NMR ($CDCl_3$, $\delta$): 1.13 (t, J = 7.2 Hz, 3H), 2.29 (s, 3H), 3.94 (q, J = 7.2 Hz, 2H), 6.96-7.46 (m, 4H), 8.28 (s, 1H), 8.50 (s, 1H).

(step 3)

Ethyl 3-(N-tert-butylsulfamoyl)-7-(4-fluoro-2-methylphenylamino)pyrazolo[1,5-a]pyrimidine-6-carboxylate

6-Ethoxycarbonyl-7-(4-fluoro-2-methylphenylamino)pyrazolo[1,5-a]pyrimidine-3-sulfonic acid (0.400 g, 1.01 mmol) obtained in step 2 was dissolved in 1,2-dichloroethane (4 mL), thionyl chloride (0.29 mL, 4.05 mmol) and DMF (0.024 mL, 0.304 mmol) were added, and the mixture was heated under reflux for 2 hr. The reaction mixture was concentrated under reduced pressure, the obtained residue was dissolved in methylene chloride (4 mL), tert-butylamine (0.21 mL, 2.02 mmol) and triethylamine (0.35 mL, 2.53 mmol) were added dropwise and the mixture was stirred at room temperature for 2 hr. 1 mol/L hydrochloric acid was added dropwise to the reaction mixture, and the mixture was extracted 3 times with chloroform. The combined organic layer was dried over anhydrous magnesium sulfate, and concentrated under reduced pressure to give the title compound (0.29 g, 63%).
ESI-MS m/z: 450 $(M+H)^+$; $^1H$-NMR ($CDCl_3$, $\delta$): 1.22 (s, 9H), 1.44 (t, J = 7.2 Hz, 3H), 2.18 (s, 3H), 4.43 (q, J = 7.2 Hz, 2H), 6.82-7.12 (m, 4H), 8.06 (s, 1H), 9.01 (s, 1H), 10.90 (s, 1H).

(step 4)

N-tert-Butyl-7-(4-fluoro-2-methylphenylamino)-6-(4-phenylpiperidine-1-carbonyl)pyrazolo[1,5-a]pyrimidine-3-sulfonamide

Ethyl 3-(N-tert-butylsulfamoyl)-7-(4-fluoro-2-methylphenylamino)pyrazolo[1,5-a]pyrimidine-6-carboxylate (0.28 g, 0.624 mmol) obtained in step 3 was dissolved in ethanol (2 mL), 2 mol/L aqueous sodium hydroxide solution

(2 mL) was added, and the mixture was stirred with heating at 80°C for 4 hr. Under ice-cooling, 2 mol/L hydrochloric acid was added dropwise to adjust the reaction mixture to pH 1, and the mixture was extracted 3 times with chloroform. The combined organic layer was dried over anhydrous magnesium sulfate, and concentrated under reduced pressure.

The residue (0.265 g) obtained above was dissolved in DMF (3 mL), 1-ethyl-3-[3-(dimethylamino)propyl]carbodiimide hydrochloride (0.142 g, 0.748 mmol), 1-hydroxybenzotriazole monohydrate (0.113 g, 0.748 mmol) and 4-phenylpiperidine (0.120 g, 0.748 mmol) were added, and the mixture was stirred at room temperature overnight. To the reaction mixture was added dropwise saturated aqueous sodium hydrogen carbonate solution, and the mixture was extracted 3 times with chloroform. The combined organic layer was dried over anhydrous magnesium sulfate, and concentrated under reduced pressure. The obtained residue was purified by silica gel column chromatography (chloroform/methanol=9/1) to give the title compound (0.143 g, 41%).
ESI-MS m/z: 565 $(M+H)^+$; $^1H$-NMR ($CDCl_3$, $\delta$): 1.28 (s, 9H), 1.39-1.58 (m, 2H), 1.77-1.89 (m, 2H), 2.40 (s, 3H), 2.59-2.74 (m, 1H), 3.85-4.26 (m, 4H), 5.04 (s, 1H), 6.89-7.40 (m, 8H), 8.16 (s, 1H), 8.35 (s, 1H), 8. 39 (s, 1H).

(step 5)

7-(4-Fluoro-2-methylphenylamino)-6-(4-phenylpiperidine-1-carbonyl)pyrazolo[1,5-a]pyrimidine-3-sulfonamide

N-tert-Butyl-7-(4-fluoro-2-methylphenylamino)-6-(4-phenylpiperidine-1-carbonyl)pyrazolo[1,5-a]pyrimidine-3-sulfonamide (0.874 g, 1.548 mmol) obtained in step 4 was dissolved in trifluoroacetic acid (13 mL), anisole (0.4 mL) was added, and the mixture was stirred at room temperature overnight. The reaction mixture was concentrated under reduced pressure, the obtained residue was purified by silica gel column chromatography (chloroform /methanol=10/1) to give the title compound (0.442 g, 56%).
ESI-MS m/z: 509 $(M+H)^+$; $^1H$-NMR (DMSO-$d_6$, $\delta$): 1.57-1.71 (m, 4H), 2.27 (s, 3H), 2.64-2.73 (m, 1H), 3.78-3.82 (m, 2H), 4.01-4.05 (m, 2H), 7.05-7.33 (m, 10H), 8.28(s, 1H), 8.45 (s, 1H), 10.04 (s, 1H).

(step 6)

N-[7-(4-Fluoro-2-methylphenylamino)-6-(4-phenylpiperidine-1-carbonyl)pyrazolo[1,5-a]pyrimidin-3-ylsulfonyl]cyclopropanecarboxamide (compound a-1)

Cyclopropanecarboxylic acid (0.066 g, 0.768 mmol) was dissolved in DMF (0.8 mL), and 1,1'-carbonyldiimidazole (0.125 g, 0.768 mmol) was added. After stirring at room temperature for 30 min, 7-(4-fluoro-2-methylphenylamino)-6-(4-phenylpiperidine-1-carbonyl)pyrazolo[1,5-a]pyrimidine-3-sulfonamide (0.078 g, 0.154 mmol) obtained in step 5 and 1,8-diazabicyclo[5.4.0]undec-7-en (0.140 g, 0.922 mmol) were added, and the mixture was stirred at 80°C for 3 hr. 5% Aqueous citric acid solution was added to quench the reaction, and the precipitated crystals were collected by filtration. The obtained crystals were purified by silica gel column chromatography (chloroform/methanol=10/1) to give the title compound (0.026 g, 29%).
ESI-MS m/z: 577 $(M+H)^+$; $^1H$-NMR ($CDCl_3$, $\delta$): 0.82-0.90 (m, 2H), 1.00-1.06 (m, 2H), 1.44-1.55 (m, 2H), 1.56-1.61 (m, 1H), 1.80-1.85 (m, 2H), 2.39 (s, 3H), 2.61-2.71 (m, 1H), 3.83-4.15 (m, 4H), 6.96 (td, J = 8.2, 2.5 Hz, 1H), 7.08 (dd, J = 9.1, 2.8 Hz, 1H), 7.14-7.23 (m, 4H), 7.30-7.36 (m, 2H), 8.23 (s, 1H), 8.44 (s, 1H), 8.61 (s, 1H), 9.54 (s, 1H).

Reference Example 2: N-Ethyl-7-(4-fluoro-2-methylphenylamino)-6-(4-phenylpiperidine-1-carbonyl)pyrazolo[1,5-a]pyrimidine-3-sulfonamide (compound a-2)

(step 1)

Ethyl 3-(N-ethylsulfamoyl)-7-(4-fluoro-2-methylphenylamino)pyrazolo[1,5-a]pyrimidine-6-carboxylate

Using 6-ethoxycarbonyl-7-(4-fluoro-2-methylphenylamino)pyrazolo[1,5-a]pyrimidine-3-sulfonic acid (0.500 g, 1.268 mmol) obtained in Reference Example 1, step 2 and ethylamine (2.0 mol/L THF solution; 2.5 mL, 5.071 mmol) instead of tert-butylamine, and in the same manner as in Reference Example 1, step 3, the title compound (0.178 g, 33%) was obtained.

(step 2)

N-Ethyl-7-(4-fluoro-2-methylphenylamino)-6-(4-phenylpiperidine-1-carbonyl)pyrazolo[1,5-a]pyrimidine-3-sulfonamide (compound a-2)

Using ethyl 3-(N-ethylsulfamoyl)-7-(4-fluoro-2-methylphenylamino)pyrazolo[1,5-a]pyrimidine-6-carboxylate (0.178 g, 0.423 mmol) obtained in step 1 and in the same manner as in Reference Example 1, step 4, the title compound (0.057 g, 25%) was obtained.
ESI-MS m/z: 537 $(M+H)^+$; $^1$H-NMR ($CDCl_3$, δ): 1.17 (t, J = 7.3 Hz, 3H), 1.43-1.55 (m, 2H), 1.80-1.86 (m, 2H), 2.40 (s, 3H), 2.62-2.71 (m, 1H), 3.02-3.12 (m, 2H), 3.75-4.00 (m, 4H), 4.94 (t, J = 5.8 Hz, 1H), 6.96 (td, J = 8.3, 3.0 Hz, 1H), 7.08 (dd, J = 8.8, 2.8 Hz, 1H), 7.14-7.36 (m, 6H), 8.17 (s, 1H), 8.33 (s, 1H), 8.43(s, 1H).

Reference Example 3: N-Ethyl-7-(4-fluoro-2-methylphenylamino)-6-[4-(4-fluorophenyl)piperidine-1-carbonyl]pyrazolo[1,5-a]pyrimidine-3-sulfonamide (compound a-3)

Using ethyl 3-(N-ethylsulfamoyl)-7-(4-fluoro-2-methylphenylamino)pyrazolo[1,5-a]pyrimidine-6-carboxylate (0.483 g, 1.145 mmol) obtained in Reference Example 2, step 1 and 4-(4-fluorophenyl)piperidine hydrochloride (0.099 g, 0.458 mmol) instead of 4-phenylpiperidine, and in the same manner as in Reference Example 1, step 4, the title compound (0.020 g, 3%) was obtained.
ESI-MS m/z: 555 $(M+H)^+$; $^1$H-NMR ($CDCl_3$, δ) : 1.17 (t, J = 7.3 Hz, 3H), 1.42-1.52 (m, 2H), 1.78-1.84 (m, 2H), 2.40 (s, 3H), 2.61-2.70 (m, 1H), 3.02-3.12 (m, 2H), 3.93-4.19 (m, 4H), 5.04 (s, 1H), 6.87-7.20 (m, 7H), 8.20 (s, 1H), 8.32 (s, 1H), 8.43 (s, 1H).

Reference Example 4: 7-(4-Fluoro-2-methylphenylamino)-6-[4-(4-fluorophenyl)piperidine-1-carbonyl]-N-(2,2,2-trifluoroethyl)pyrazolo[1,5-a]pyrimidine-3-sulfonamide

(compound a-4)

(step 1)

Ethyl 7-(4-fluoro-2-methylphenylamino)-3-[N-(2,2,2-trifluoroethyl)sulfamoyl]pyrazolo[1,5-a]pyrimidine-6-carboxylate

Using 6-ethoxycarbonyl-7-(4-fluoro-2-methylphenylamino)pyrazolo[1,5-a]pyrimidine-3-sulfonic acid (0.970 g, 2.460 mmol) obtained in Reference Example 1, step 2 and 2,2,2-trifluoroethylamine (0.975 g, 9.838 mmol) instead of tert-butylamine, and in the same manner as in Reference Example 1, step 3, the title compound (1.024 g, 87%) was obtained.

(step 2)

7-(4-Fluoro-2-methylphenylamino)-6-[4-(4-fluorophenyl)piperidine-1-carbonyl]-N-(2,2,2-trifluoroethyl)pyrazolo[1,5-a]pyrimidine-3-sulfonamide (compound a-4)

Using ethyl 7-(4-fluoro-2-methylphenylamino)-3-[N-(2,2,2-trifluoroethyl)sulfamoyl]pyrazolo[1,5-a]pyrimidine-6-carboxylate (0.142 g, 0.298 mmol) obtained in step 1 and 4-(4-fluorophenyl)piperidine hydrochloride (0.072 g, 0.335 mmol) instead of 4-phenylpiperidine, and in the same manner as in Reference Example 1, step 4, the title compound (0.028 g, 21%) was obtained.
ESI-MS m/z: 609 $(M+H)^+$; $^1$H-NMR ($CDCl_3$, δ): 1.39-1.52 (m, 2H), 1.79-1.84 (m, 2H), 2.39 (s, 3H), 2.60-2.69 (m, 1H), 3.69-3.80 (m, 2H), 4.11-4.30 (m, 4H), 5.68 (s, 1H), 6.91-7.20 (m, 7H), 8.19 (s, 1H), 8.33 (s, 1H), 8.41 (s, 1H).

Reference Example 5: N-Cyclopropyl-7-(4-fluoro-2-methylphenylamino)-6-[4-(4-fluorophenyl)piperidine-1-carbonyl]pyrazolo[1,5-a]pyrimidine-3-sulfonamide (compound a-5)

(step 1)

Ethyl 3-(N-cyclopropylsulfamoyl)-7-(4-fluoro-2-methylphenylamino)-pyrazolo[1,5-a]pyrimidine-6-carboxylate

Using 6-ethoxycarbonyl-7-(4-fluoro-2-methylphenylamino)pyrazolo[1,5-a]pyrimidine-3-sulfonic acid (0.400 g, 1.145 mmol) obtained in Reference Example 1, step 2 and cyclopropylamine (0.261 g, 4.580 mmol) instead of tert-butylamine, and in the same manner as in Reference Example 1, step 3, the title compound (0.496 g, 100%) was obtained.

(step 2)

N-Cyclopropyl-7-(4-fluoro-2-methylphenylamino)-6-[4-(4-fluorophenyl)piperidine-1-carbonyl]pyrazolo[1,5-a]pyrimidine-3-sulfonamide (compound a-5)

Using ethyl 3-(N-cyclopropylsulfamoyl)-7-(4-fluoro-2-methylphenylamino)-pyrazolo[1,5-a]pyrimidine-6-carboxylate (0.496 g, 1.145 mmol) obtained in step 1 and 4-(4-fluorophenyl)piperidine hydrochloride (0.072 g, 0.335 mmol) instead of 4-phenylpiperidine, and in the same manner as in Reference Example 1, step 4, the title compound (0.0283 g, 3%) was obtained.
ESI-MS m/z: 567 $(M+H)^+$; $^1$H-NMR ($CDCl_3$, δ): 0.61-0.64 (m, 2H), 0.72-0.76 (m, 2H), 1.38-1.51 (m, 2H), 1.78-1.83 (m, 2H), 2.25-2.30 (m, 1H), 2.40 (s, 3H), 2.61-2.69 (m, 1H), 4.07-4.23 (m, 4H), 5.49 (s, 1H), 6.91-7.20 (m, 7H), 8.19 (s, 1H), 8.33 (s, 1H), 8.48 (s, 1H).

Reference Example 6: N-[7-(2-Chlorophenylamino)-6-(4-phenylpiperidine-1-carbonyl)pyrazolo[1,5-a]pyrimidin-3-ylsulfonyl]cyclopropanecarboxamide (compound a-6)

(step 1)

Ethyl 7-(2-chlorophenylamino)pyrazolo[1,5-a]pyrimidine-6-carboxylate

Using ethyl 7-hydroxypyrazolo[1,5-a]pyrimidine-6-carboxylate (3.00 g, 14.5mmol) and 2-chloroaniline (2.77 g, 21.7 mmol) instead of 4-fluoro-2-methylaniline, and in the same manner as in Reference Example 1, step 1, the title compound (1.44 g, 31%) was obtained.

(step 2)

7-(2-Chlorophenylamino)-6-(ethoxycarbonyl)pyrazolo[1,5-a]pyrimidine-3-sulfonic acid

Using ethyl 7-(2-chlorophenylamino)pyrazolo[1,5-a]pyrimidine-6-carboxylate (1.44 g, 4.55 mmol) obtained in step 1 and in the same manner as in Reference Example 1, step 2, the title compound (1.70 g, 94%) was obtained.

(step 3)

Ethyl 3-(N-tert-butylsulfamoyl)-7-(2-chlorophenylamino)pyrazolo[1,5-a]pyrimidine-6-carboxylate

Using 7-(2-chlorophenylamino)-6-(ethoxycarbonyl)pyrazolo[1,5-a]pyrimidine-3-sulfonic acid (1.70 g, 4.30 mmol) obtained in step 2 and in the same manner as in Reference Example 1, step 3, the title compound (1.94 g, 100%) was obtained.

(step 4)

N-tert-Butyl-7-(2-chlorophenylamino)-6-(4-phenylpiperidine-1-carbonyl)pyrazolo[1,5-a]pyrimidine-3-sulfonamide

Using ethyl 3-(N-tert-butylsulfamoyl)-7-(2-chlorophenylamino)pyrazolo[1,5-a]pyrimidine-6-carboxylate (1.94 g, 4.30 mmol) obtained in step 3 and in the same manner as in Reference Example 1, step 4, the title compound (0.804 g, 33%) was obtained.

(step 5)

7-(2-Chlorophenylamino)-6-(4-phenylpiperidine-1-carbonyl)pyrazolo[1,5-a]pyrimidine-3-sulfonamide

Using N-tert-butyl-7-(2-chlorophenylamino)-6-(4-phenylpiperidine-1-carbonyl)pyrazolo[1,5-a]pyrimidine-3-sulfonamide (0.804 g, 1.417 mmol) obtained in step 4 and in the same manner as in Reference Example 1, step 5, the title compound (0.499 g, 69%) was obtained.

(step 6)

N-[7-(2-Chlorophenylamino)-6-(4-phenylpiperidine-1-carbonyl)pyrazolo[1,5-a]pyrimidin-3-ylsulfonyl]cyclopropanecarboxamide (compound a-6)

Using 7-(2-chlorophenylamino)-6-(4-phenylpiperidine-1-carbonyl)pyrazolo[1,5-a]pyrimidine-3-sulfonamide (0.050 g, 0.098 mmol) obtained in step 5 and in the same manner as in Reference Example 1, step 6, the title compound (0.034 g, 29%) was obtained.
ESI-MS m/z: 579 $(M+H)^+$; $^1H$-NMR ($CDCl_3$, $\delta$) : 0.84-0.90 (m, 2H), 1.01-1.06 (m, 2H), 1.36-1.50 (m, 2H), 1.56-1.59 (m, 1H), 1.76-1.81 (m, 2H), 2.56-2.64 (m, 1H), 3.61-3.74 (m, 2H), 4.12-4.32 (m, 2H), 7.11-7.14 (m, 2H), 7.20-7.42 (m, 6H), 7.54-7.59 (m, 1H), 8.53 (s, 1H), 8.62 (s, 1H), 8.64 (s, 1H), 9.47 (s, 1H).

Reference Example 7: 7-(2-Chlorophenylamino)-N-(ethylcarbamoyl)-6-(4-phenylpiperidine-1-carbonyl)pyrazolo[1,5-a]pyrimidine-3-sulfonamide (compound a-7)

7-(2-Chlorophenylamino)-6-(4-phenylpiperidine-1-carbonyl)pyrazolo[1,5-a]pyrimidine-3-sulfonamide (0.050 g, 0.098 mmol) obtained in Reference Example 6, step 5 was dissolved in acetone (0.5 mL), potassium carbonate (0.041 g, 0.293 mmol) and ethyl isocyanate (0.017 g, 0.245 mmol) were added, and the mixture was stirred at 50 °C for 2 hr. Saturated aqueous ammonium chloride solution was added to quench the reaction, and the mixture was extracted 3 times with ethyl acetate. The organic layers were combined, washed with saturated brine, dried over anhydrous sodium sulfate, and concentrated under reduced pressure. The obtained residue was purified by silica gel column chromatography (chloroform/methanol=99/1) to give the title compound (0.027 g, 48%).
ESI-MS m/z: 582 $(M+H)^+$; $^1H$-NMR ($CDCl_3$, $\delta$) : 1.16 (t, J = 7.3 Hz, 3H), 1.35-1.47 (m, 2H), 1.77-1.81 (m, 2H), 2.57-2.65 (m, 1H), 3.22-3.31 (m, 2H), 3.72-3.85 (m, 2H), 4.15-4.30 (m, 2H), 6.71 (s, 1H), 7.11-7.14 (m, 2H), 7.20-7.44 (m, 6H), 7.55-7.61 (m, 1H), 7.95 (s, 1H), 8.45 (s, 1H), 8.52 (s, 2H).

Reference Example 8: Methyl 7-(2-chlorophenylamino)-6-(4-phenylpiperidine-1-carbonyl)pyrazolo[1,5-a]pyrimidin-3-ylsulfonylcarbamate (compound a-8)

7-(2-Chlorophenylamino)-6-(4-phenylpiperidine-1-carbonyl)pyrazolo[1,5-a]pyrimidine-3-sulfonamide (0.070 g, 0.137 mmol) obtained in Reference Example 6, step 5 was dissolved in dichloromethane (0.7 mL), and the solution was cooled to 0 °C. Pyridine (0.325 g, 4.110 mmol) and methyl chloroformate (0.350 g, 3.699 mmol) were added, the mixture was stirred at room temperature for 48 hr, and 1 mol/L hydrochloric acid was added to quench the reaction. The organic layer was separated, and the aqueous layer was extracted twice with chloroform. The organic layers were combined, washed with saturated brine, dried over anhydrous sodium sulfate, and concentrated under reduced pressure. The obtained residue was purified by silica gel column chromatography (chloroform/methanol=99/1) to give the title compound (0.031 g, 40%).
ESI-MS m/z: 569 $(M+H)^+$; $^1H$-NMR ($CDCl_3$, $\delta$) : 1.35-1.51 (m, 2H), 1.76-1.81 (m, 2H), 2.56-2.65 (m, 1H), 3.70 (s, 3H), 4.06-4.40 (m, 4H), 7.11-7.14 (m, 2H), 7.20-7.43 (m, 6H), 7.57 (dd, J = 6.8, 2.4 Hz, 1H), 8.49 (s, 1H), 8.53 (s, 1H), 8.59 (s, 1H), 8.63 (s, 1H).

Reference Example 9: N-{7-(2-Chlorophenylamino)-6-[4-(4-fluorophenyl)piperidine-1-carbonyl]pyrazolo[1,5-a]pyrimidin-3-ylsulfonyl}cyclopropanecarboxamide (compound a-9)

(step 1)

N-tert-Butyl-7-(2-chlorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chlorophenylamino)pyrazolo[1,5-a]pyrimidine-6-carboxylate (4.25 g, 8.82 mmol) obtained in Reference Example 6, step 3 and 4-(4-fluorophenyl)piperidine (1.87 g, 10.46 mmol) instead of 4-phenylpiperidine, and in the same manner as in Reference Example 1, step 4, the title compound (2.42 g, 59%) was obtained.

(step 2)

7-(2-Chlorophenylamino)-6-[4-(4-fluorophenyl)piperidine-1-carbonyl]pyrazolo[1,5-a]pyrimidine-3-sulfonamide

Using N-tert-butyl-7-(2-chlorophenylamino)-6-[4-(4-fluorophenyl)piperidine-1-carbonyl]pyrazolo[1,5-a]pyrimidine-3-sulfonamide (2.24 g, 4.14 mmol) obtained in step 1 and in the same manner as in Reference Example 1, step 5, the title compound (1.49 g, 68%) was obtained.

(step 3)

N-{7-(2-Chlorophenylamino)-6-[4-(4-fluorophenyl)piperidine-1-carbonyl]pyrazolo[1,5-a]pyrimidin-3-ylsulfonyl}cyclopropanecarboxamide (compound a-9)

Using 7-(2-chlorophenylamino)-6-[4-(4-fluorophenyl)piperidine-1-carbonyl]pyrazolo[1,5-a]pyrimidine-3-sulfonamide (0.080 g, 0.151 mmol) obtained in step 2 and in the same manner as in Reference Example 1, step 6, the title compound (0.080 g, 89%) was obtained.
ESI-MS m/z: 597 $(M+H)^+$; $^1$H-NMR (DMSO-$d_6$, δ): 0.64-0.82 (m, 4H), 1.40-1.78 (m, 4H), 1.98-2.23 (m, 1H), 2.62-2.75 (m, 1H), 2.93-3.08 (m, 1H), 3.37-3.43 (m, 1H), 3.76-4.02 (m, 2H), 7.09-7.55 (m, 8H), 8.36 (s, 1H), 8.56 (s, 1H), 10.40 (s, 1H).

Reference Example 10: 7-(2-Chlorophenylamino)-N-(ethylcarbamoyl)-6-[4-(4-fluorophenyl)piperidine-1-carbonyl]pyrazolo[1,5-a]pyrimidine-3-sulfonamide (compound a-10)

Using 7-(2-chlorophenylamino)-6-[4-(4-fluorophenyl)piperidine-1-carbonyl]pyrazolo[1,5-a]pyrimidine-3-sulfonamide (0.080 g, 0.151 mmol) obtained in Reference Example 9, step 2 and in the same manner as in Reference Example 7, the title compound (0.106 g, 94%) was obtained.
ESI-MS m/z: 600 $(M+H)^+$; $^1$H-NMR (DMSO-$d_6$, δ): 0.94 (t, J = 7.1 Hz, 3H), 1.21-1.69 (m, 4H), 2.07-2.23 (m, 1H), 2.64-2.74 (m, 1H), 2.93-3.03 (m, 2H), 3.01-3.07 (m, 1H), 3.75-4.04 (m, 2H), 5.70 (s, 1H), 6.39 (t, J = 5.6 Hz, 1H), 7.09-7.57 (m, 8H), 8.34 (s, 1H), 8.56 (s, 1H), 10.42 (s, 1H).

Reference Example 11: 7-(2-Chlorophenylamino)-6-[4-(4-fluorophenyl)piperidine-1-carbonyl]-N-(2-methoxyethyl)pyrazolo[1,5-a]pyrimidine-3-sulfonamide (compound a-11)

(step 1)

Ethyl 7-(2-chlorophenylamino)-3-[N-(2-methoxyethyl)sulfamoyl]pyrazolo[1,5-a]pyrimidine-6-carboxylate

Using 7-(2-chlorophenylamino)-6-(ethoxycarbonyl)pyrazolo[1,5-a]pyrimidine-3-sulfonic acid (0.400 g, 1.008 mmol) obtained in Reference Example 6, step 2 and 2-methoxyethylamine (0.303 g, 4.032 mmol) instead of tert-butylamine, and in the same manner as in Reference Example 1, step 3, the title compound (0.459 g, 100%) was obtained.

(step 2)

7-(2-Chlorophenylamino)-6-[4-(4-fluorophenyl)piperidine-1-carbonyl]-N-(2-methoxyethyl)pyrazolo[1,5-a]pyrimidine-3-sulfonamide (compound a-11)

Using ethyl 7-(2-chlorophenylamino)-3-[N-(2-methoxyethyl)sulfamoyl]pyrazolo[1,5-a]pyrimidine-6-carboxylate (0.459 g, 1.008 mmol) obtained in step 1 and 4-(4-fluorophenyl)piperidine (0.158 g, 0.881 mmol) instead of 4-phenylpiperidine, and in the same manner as in Reference Example 1, step 4, the title compound (0.086 g, 15%) was obtained.
ESI-MS m/z: 587 $(M+H)^+$; $^1$H-NMR ($CDCl_3$, 5): 1.31-1.45 (m, 2H), 1.74-1.79 (m, 2H), 2.55-2.64 (m, 1H), 3.20-3.26 (m, 2H), 3.30 (s, 3H), 3.49 (t, J = 5.1 Hz, 2H), 3.89-4.26 (m, 4H), 5.37 (t, J = 5.9 Hz, 1H), 6.96-7.10 (m, 4H), 7.28-7.42 (m, 3H), 7.54-7.57 (m, 1H), 8.44 (s, 1H), 8.49 (s, 1H), 8.49 (s, 1H).

Reference Example 12: N-{7-(2-Chloro-5-methylphenylamino)-6-[4-(4-fluorophenyl)piperidine-1-carbonyl]pyrazolo[1,5-a]pyrimidin-3-ylsulfonyl}cyclopropanecarboxamide (compound a-12)

(step 1)

ethyl 7-(2-Chloro-5-methylphenylamino)pyrazolo[1,5-a]pyrimidine-6-carboxylate

Using ethyl 7-hydroxypyrazolo[1,5-a]pyrimidine-6-carboxylate (5.00 g, 24.1 mmol) and 2-chloro-5-methylaniline (8.54 g, 60.3 mmol) instead of 4-fluoro-2-methylaniline, and in the same manner as in Reference Example 1, step 1, the title compound (2.94 g, 37%) was obtained.

(step 2)

7-(2-Chloro-5-methylphenylamino)-6-(ethoxycarbonyl)pyrazolo[1,5-a]pyrimidine-3-sulfonic acid

Using ethyl 7-(2-chloro-5-methylphenylamino)pyrazolo[1,5-a]pyrimidine-6-carboxylate (2.94 g, 8.89 mmol) obtained in step 1 and in the same manner as in Reference Example 1, step 2, the title compound (3.60 g, 99%) was obtained.

(step 3)

Ethyl 3-(N-tert-butylsulfamoyl)-7-(2-chloro-5-methylphenylamino)pyrazolo[1,5-a]pyrimidine-6-carboxylate

Using 7-(2-chloro-5-methylphenylamino)-6-(ethoxycarbonyl)pyrazolo[1,5-a]pyrimidine-3-sulfonic acid (3.00 g, 7.30 mmol) obtained in step 2 and in the same manner as in Reference Example 1, step 3, the title compound (3.30 g, 97%) was obtained.

(step 4)

N-tert-Butyl-7-(2-chloro-5-methylphenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chloro-5-methylphenylamino)pyrazolo[1,5-a]pyrimidine-6-carboxylate (2.31 g, 4.96 mmol) obtained in step 3 and 4-(4-fluorophenyl)piperidine (0.92 g, 5.14 mmol) instead of 4-phenylpiperidine, and in the same manner as in Reference Example 1, step 4, the title compound (0.85 g, 41%) was obtained.

(step 5)

7-(2-Chloro-5-methylphenylamino)-6-[4-(4-fluorophenyl)piperidine-1-carbonyl]pyrazolo[1,5-a]pyrimidine-3-sulfonamide

Using N-tert-butyl-7-(2-chloro-5-methylphenylamino)-6-[4-(4-fluorophenyl)piperidine-1-carbonyl]pyrazolo[1,5-a]pyrimidine-3-sulfonamide (0.85 g, 1.42 mmol) obtained in step 4 and in the same manner as in Reference Example 1, step 5, the title compound (0.42 g, 55%) was obtained.

(step 6)

N-{7-(2-Chloro-5-methylphenylamino)-6-[4-(4-fluorophenyl)piperidine-1-carbonyl]pyrazolo[1,5-a]pyrimidin-3-ylsulfonyl}cyclopropanecarboxamide(compound a-12)

Using 7-(2-chloro-5-methylphenylamino)-6-[4-(4-fluorophenyl)piperidine-1-carbonyl]pyrazolo[1,5-a]pyrimidine-3-sulfonamide (0.08 g, 0.147 mmol) obtained in step 5 and in the same manner as in Reference Example 1, step 6, the title compound (0.072 g, 80%) was obtained.
ESI-MS m/z: 611 $(M+H)^+$; $^1$H-NMR ($CDCl_3$, δ) : 0.82-0.89 (m, 2H), 1.00-1.06 (m, 2H), 1.31-1.48 (m, 2H), 1.56-1.59 (m, 1H), 1.71-1.76 (m, 2H), 2.35 (s, 3H), 2.52-2.56 (m, 1H), 3.61-3.74 (m, 2H), 4.12-4.32 (m, 2H), 6.94-7.13 (m, 5H), 7.22 (br s, 1H), 7.42 (d, J = 8.3 Hz, 1H), 8.50 (br s, 1H), 8.65 (s, 1H), 8.76 (s, 1H), 9.77 (br s, 1H).

Reference Example 13: N-{7-(2-Chloro-5-methylphenylamino)-6-[4-(4-fluorophenyl)piperidine-1-carbonyl]pyrazolo[1,5-a]pyrimidin-3-ylsulfonyl}ethanecarboxamide (compound a-13)

Using 7-(2-chloro-5-methylphenylamino)-6-[4-(4-fluorophenyl)piperidine-1-carbonyl]pyrazolo[1,5-a]pyrimidine-3-sulfonamide (0.08 g, 0.147 mmol) obtained in Reference Example 12, step 5 and propionic acid (0.055 mL, 0.737 mmol) instead of cyclopropanecarboxylic acid, and in the same manner as in Reference Example 1, step 6, the title compound (0.081 g, 92%) was obtained.
ESI-MS m/z: 599 $(M+H)^+$; $^1$H-NMR ($CDCl_3$, $\delta$): 1.08 (d, J = 7.3 Hz, 3H), 1.35-1.48 (m, 2H), 1.71-1.76 (m, 2H), 2.36 (s, 3H), 2.37 (q, J = 7.3 Hz, 2H), 2.52-2.61 (m, 1H), 3.61-3.74 (m, 2H), 4.12-4.32 (m, 2H), 6.94-7.14 (m, 5H), 7.23 (br s, 1H), 7.42 (d, J = 8.3 Hz, 1H), 8.52 (br s, 1H), 8.68 (s, 1H), 8.75 (s, 1H), 9.80 (brs, 1H).

Reference Example 14: 7-(2-Chloro-5-methylphenylamino)-N-(ethylcarbamoyl)-6-[4-(4-fluorophenyl)piperidine-1-carbonyl]pyrazolo[1,5-a]pyrimidine-3-sulfonamide (compound a-14)

Using 7-(2-chloro-5-methylphenylamino)-6-[4-(4-fluorophenyl)piperidine-1-carbonyl]pyrazolo[1,5-a]pyrimidine-3-sulfonamide (0.08 g, 0.147 mmol) obtained in Reference Example 12, step 5 and in the same manner as in Reference Example 7, the title compound (0.056 g, 62%) was obtained.
ESI-MS m/z: 614 $(M+H)^+$; $^1$H-NMR ($CDCl_3$, $\delta$) : 1.16 (d, J = 7.3 Hz, 3H), 1.35-1.39 (m, 2H), 1.72-1.77 (m, 2H), 2.37 (s, 3H), 2.54-2.61 (m, 1H), 3.21-3.31 (m, 2H), 3.61-3.74 (m, 2H), 4.12-4.32 (m, 2H), 6.70 (br s, 1H), 6.95-7.14 (m, 5H), 7.24-7.26 (m, 1H), 7.42 (d, J = 8.9 Hz, 1H), 7.97 (br s, 1H), 8.45 (s, 1H), 8.49 (br s, 1H), 8.56 (s, 1H).

Reference Example 15: N-Ethyl-7-(2-chloro-5-methylphenylamino)-6-[4-(4-fluorophenyl)piperidine-1-carbonyl]pyrazolo[1,5-a]pyrimidine-3-sulfonamide (compound a-15)

(step 1)

Ethyl 7-(2-chloro-5-methylphenylamino)-3-(N-ethylsulfamoyl)pyrazolo[1,5-a]pyrimidine-6-carboxylate

Using 7-(2-chloro-5-methylphenylamino)-6-(ethoxycarbonyl)pyrazolo[1,5-a]pyrimidine-3-sulfonic acid (0.72 g, 1.75 mmol) obtained in Reference Example 12, step 2 and ethylamine (2.0 mol/L THF solution; 3.5 mL, 7.01 mmol) instead of tert-butylamine, and in the same manner as in Reference Example 1 step 3, the title compound (0.67 g, 87%) was obtained.

(step 2)

N-Ethyl-7-(2-chloro-5-methylphenylamino)-6-[4-(4-fluorophenyl)piperidine-1-carbonyl]pyrazolo[1,5-a]pyrimidine-3-sulfonamide (compound a-15)

Using ethyl 7-(2-chloro-5-methylphenylamino)-3-(N-ethylsulfamoyl)pyrazolo[1,5-a]pyrimidine-6-carboxylate (0.084 g, 0.205 mmol) obtained in step 1 and 4-(4-fluorophenyl)piperidine (0.030 g, 0.183 mmol) instead of 4-phenylpiperidine, and in the same manner as in Reference Example 1, step 4, the title compound (0.019 g, 16%) was obtained.
ESI-MS m/z: 571 $(M + H)^+$; $^1$H-NMR ($CDCl_3$, $\delta$) : 1.17 (t, J = 7.3 Hz, 3H), 1.20-1.43 (m, 2H), 1.62-1.80 (m, 2H), 2.37 (s, 3H), 2.52-2.62 (m, 1H), 3.03-3.10 (m, 2H), 3.20-4.40 (m, 4H), 4.97 (br t, J = 6.0 Hz, 1H), 6.90-7.27 (m, 6H), 7.37 (d, J = 8.3 Hz, 1H), 8.45 (s, 1H), 8.46 (s, 1H), 8.50 (s, 1H).

Reference Example 16: N-Ethyl-7-(4-chloro-2-methylphenylamino)-6-[4-(4-fluorophenyl)piperidine-1-carbonyl]pyrazolo[1,5-a]pyrimidine-3-sulfonamide (compound a-16)

(step 1)

Ethyl 7-(4-chloro-2-methylphenylamino)pyrazolo[1,5-a]pyrimidine-6-carboxylate
Using ethyl 7-hydroxypyrazolo[1,5-a]pyrimidine-6-carboxylate (5.00 g, 24.1 mmol) and 4-chloro-2-methylaniline (5.13 g, 36.2 mmol) instead of 4-fluoro-2-methylaniline, and in the same manner as in Reference Example 1, step 1, the title compound (3.80 g, 48%) was obtained.

(step 2)

7-(4-Chloro-2-methylphenylamino)-6-(ethoxycarbonyl)pyrazolo[1,5-a]pyrimidine-3-sulfonic acid

Using ethyl 7-(4-chloro-2-methylphenylamino)pyrazolo[1,5-a]pyrimidine-6-carboxylate (3.80 g, 11.5 mmol) obtained in step 1 and in the same manner as in Reference Example 1, step 2, the title compound (4.58 g, 97%) was obtained.

(step 3)

Ethyl 7-(4-chloro-2-methylphenylamino)-3-(N-ethylsulfamoyl)pyrazolo[1,5-a]pyrimidine-6-carboxylate

Using 7-(4-chloro-2-methylphenylamino)-6-(ethoxycarbonyl)pyrazolo[1,5-a]pyrimidine-3-sulfonic acid (1.00 g, 2.43 mmol) obtained in step 2 and ethylamine (2.0 mol/L THF solution; 4.9 mL, 9.74 mmol) instead of tert-butylamine, and in the same manner as in Reference Example 1, step 3, the title compound (1.01 g, 95%) was obtained.

(step 4)

N-Ethyl-7-(4-chloro-2-methylphenylamino)-6-[4-(4-fluorophenyl)piperidine-1-carbonyl]pyrazolo[1,5-a]pyrimidine-3-sulfonamide (compound a-16)

Using ethyl 7-(4-chloro-2-methylphenylamino)-3-(N-ethylsulfamoyl)pyrazolo[1,5-a]pyrimidine-6-carboxylate (0.33 g, 0.77 mmol) obtained in step 3 and 4-(4-fluorophenyl)piperidine (0.26 g, 1.44 mmol) instead of 4-phenylpiperidine, and in the same manner as in Reference Example 1, step 4, the title compound (0.029 g, 6%) was obtained.
ESI-MS m/z: 569 $(M - H)^-$; $^1$H-NMR ($CDCl_3$, $\delta$) : 1.16 (t, J = 7.3 Hz, 3H), 1.30-1.50 (m, 2H), 1.72-1.87 (m, 2H), 2.39 (s, 3H), 2.58-2.80 (m, 1H), 3.02-3.12 (m, 2H), 3.25-4.03 (m, 2H), 4.05-4.28 (m, 2H), 4.90 (t, J = 5.9 Hz, 1H), 6.95-7.30 (m, 6H), 7.37 (s, 1H), 8.19 (s, 1H), 8.34 (s, 1H), 8.43 (s, 1H).

<u>Reference Example 17</u>: N-[7-(4-Fluoro-2-methylphenylamino)-6-(4-phenylpiperidine-1-carbonyl)pyrazolo[1,5-a]pyrimidin-3-ylsulfonyl]acetamide (compound a-17)

7-(4-Fluoro-2-methylphenylamino)-6-(4-phenylpiperidine-1-carbonyl)pyrazolo[1,5-a]pyrimidine-3-sulfonamide (0.098 g, 0.175 mmol) obtained in Reference Example 1, step 5 was dissolved in pyridine (2 mL), 4-(dimethylamino)pyridine (0.023 g, 0.189 mmol) and acetyl chloride (0.25 mL, 3.50 mmol) were added, and the mixture was stirred with heating at 50 °C for 2 hr. 1 mol/L Hydrochloric acid was added dropwise to the reaction mixture, and the mixture was extracted 3 times with chloroform. The combined organic layer was dried over anhydrous magnesium sulfate, and concentrated under reduced pressure. The obtained residue was purified by silica gel column chromatography (chloroform/methanol=9/1) to give the title compound (0.013 g, 14%).
ESI-MS m/z: 551 $(M+H)^+$; $^1$H-NMR ($CDCl_3$, $\delta$) : 1.44-1.62 (m, 2H), 1.72-1.91 (m, 2H), 2.10 (s, 3H), 2.40 (s, 3H), 2.56-2.76 (m, 1H), 3.63-4.43 (m, 4H), 6.91-7.04 (m, 1H), 7.04-7.37 (m, 7H), 8.28 (s, 1H), 8.48 (s, 1H), 8.63 (s, 1H), 10.21 (s, 1H).

<u>Reference Example 18</u>: N-{7-(2-Chloro-4-methylphenylamino)-6-[4-(4-fluorophenyl)piperidine-1-carbonyl]pyrazolo[1,5-a]pyrimidin-3-ylsulfonyl}propionamide (compound a-18)

(step 1)

Ethyl 7-(2-chloro-4-methylphenylamino)pyrazolo[1,5-a]pyrimidine-6-carboxylate

Using ethyl 7-hydroxypyrazolo[1,5-a]pyrimidine-6-carboxylate (15.0 g, 72.4 mmol) and 2-chloro-4-methylaniline (20.5 g, 144.7 mmol) instead of 4-fluoro-2-methylaniline, and in the same manner as in Reference Example 1, step 1, the title compound (8.37 g, 35%) was obtained.

(step 2)

6-Ethoxycarbonyl-7-(2-chloro-4-methylphenylamino)pyrazolo[1,5-a]pyrimidine-3-sulfonic acid

Using ethyl 7-(2-chloro-4-methylphenylamino)pyrazolo[1,5-a]pyrimidine-6-carboxylate (8.37 g, 25.3 mmol) obtained in step 1 and in the same manner as in Reference Example 1, step 2, the title compound (10.7 g, 100%) was obtained.

(step 3)

Ethyl 3-(N-tert-butylsulfamoyl)-7-(2-chloro-4-methylphenylamino)pyrazolo[1,5-a]pyrimidine-6-carboxylate

Using 6-ethoxycarbonyl-7-(2-chloro-4-methylphenylamino)pyrazolo[1,5-a]pyrimidine-3-sulfonic acid (10.66 g, 25.3 mmol) obtained in step 2 and in the same manner as in Reference Example 1, step 3, the title compound (11.17 g, 95%) was obtained.

(step 4)

N-tert-Butyl-7-(2-chloro-4-methylphenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chloro-4-methylphenylamino)pyrazolo[1,5-a]pyrimidine-6-carboxylate (11.17 g, 24.0 mmol) obtained in step 3 and 4-(4-fluorophenyl)piperidine (5.93 g, 33.09 mmol) instead of 4-phenylpiperidine, and in the same manner as in Reference Example 1, step 4, the title compound (8.33 g, 63%) was obtained.

(step 5)

7-(2-Chloro-4-methylphenylamino)-6-[4-(4-fluorophenyl)piperidine-1-carbonyl]pyrazolo[1,5-a]pyrimidine-3-sulfonamide

Using N-tert-butyl-7-(2-chloro-4-methylphenylamino)-6-[4-(4-fluorophenyl)piperidine-1-carbonyl]pyrazolo[1,5-a]pyrimidine-3-sulfonamide (3.80 g, 6.34 mmol) obtained in step 4 and in the same manner as in Reference Example 1, step 5, the title compound (2.22 g, 64%) was obtained.

(step 6)

N-{7-(2-Chloro-4-methylphenylamino)-6-[4-(4-fluorophenyl)piperidine-1-carbonyl]pyrazolo[1,5-a]pyrimidin-3-ylsulfonyl}propionamide (compound a-18)

Using 7-(2-chloro-4-methylphenylamino)-6-[4-(4-fluorophenyl)piperidine-1-carbonyl]pyrazolo[1,5-a]pyrimidine-3-sulfonamide (0.050 g, 0.092 mmol) obtained in step 5 as a starting material and propionic acid (0.034 mL, 0.460 mmol) instead of cyclopropanecarboxylic acid, and in the same manner as in Reference Example 1, step 6, the title compound (0.052 g, 93%) was obtained.
ESI-MS m/z: 599 $(M + H)^+$; $^1H$-NMR ($CDCl_3$, $\delta$): 1.09 (t, J = 7.4 Hz, 3H), 1.31-1.43 (m, 2H), 1.74-1.79 (m, 2H), 2.35 (q, J = 7.5 Hz, 2H), 2.39 (s, 3H), 2.56-2.65 (m, 1H), 3.41-3.52 (m, 4H), 6.96-7.29 (m, 6H), 7.36 (s, 1H), 8.44 (s, 1H), 8.53 (s, 1H), 8.65 (s, 1H).

Reference Example 19: N-{7-(2,4-Dichlorophenylamino)-6-[4-(4-fluorophenyl)piperidine-1-carbonyl]pyrazolo[1,5-a]pyrimidin-3-ylsulfonyl}propionamide (compound a-19)

(step 1)

Ethyl 7-(2,4-dichlorophenylamino)pyrazolo[1,5-a]pyrimidine-6-carboxylate

Using ethyl 7-hydroxypyrazolo[1,5-a]pyrimidine-6-carboxylate (7.50 g, 36.2 mmol) and 2,4-dichloroaniline (5.30 g, 32.6 mmol) instead of 4-fluoro-2-methylaniline, and in the same manner as in Reference Example 1, step 1, the title compound (9.09 g, 72%) was obtained.

(step 2)

6-Ethoxycarbonyl-7-(2,4-dichlorophenylamino)pyrazolo[1,5-a]pyrimidine-3-sulfonic acid

Using ethyl 7-(2,4-dichlorophenylamino)pyrazolo[1,5-a]pyrimidine-6-carboxylate (9.09 g, 25.9 mmol) obtained in step 1 and in the same manner as in Reference Example 1, step 2, the title compound (8.69 g, 78%) was obtained.

(step 3)

Ethyl 3-(N-tert-butylsulfamoyl)-7-(2,4-dichlorophenylamino)pyrazolo[1,5-a]pyrimidine-6-carboxylate

Using 6-ethoxycarbonyl-7-(2,4-dichlorophenylamino)pyrazolo[1,5-a]pyrimidine-3-sulfonic acid (8.69 g, 20.2 mmol) obtained in step 2 and in the same manner as in Reference Example 1, step 3, the title compound (7.74 g, 79%) was obtained.

(step 4)

N-tert-Butyl-7-(2,4-dichlorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2,4-dichlorophenylamino)pyrazolo[1,5-a]pyrimidine-6-carboxylate (2.98 g, 6.13 mmol) obtained in step 3 and 4-(4-fluorophenyl)piperidine (1.5 g, 8.18 mmol) instead of 4-phenylpiperidine, and in the same manner as in Reference Example 1, step 4, the title compound (1.57 g, 41%) was obtained.

(step 5)

7-(2,4-Dichlorophenylamino)-6-[4-(4-fluorophenyl)piperidine-1-carbonyl]pyrazolo[1,5-a]pyrimidine-3-sulfonamide

Using N-tert-butyl-7-(2,4-dichlorophenylamino)-6-[4-(4-fluorophenyl)piperidine-1-carbonyl]pyrazolo[1,5-a]pyrimidine-3-sulfonamide (1.57 g, 2.53 mmol) obtained in step 4 and in the same manner as in Reference Example 1, step 5, the title compound (1.30 g, 91%) was obtained.

(step 6)

N-{7-(2,4-Dichlorophenylamino)-6-[4-(4-fluorophenyl)piperidine-1-carbonyl]pyrazolo[1,5-a]pyrimidin-3-ylsulfonyl}propionamide (compound a-19)

Using 7-(2,4-dichlorophenylamino)-6-[4-(4-fluorophenyl)piperidine-1-carbonyl]pyrazolo[1,5-a]pyrimidine-3-sulfonamide (0.050 g, 0.089 mmol) obtained in step 5 and propionic acid (0.033 mL, 0.444 mmol) instead of cyclopropanecarboxylic acid, and in the same manner as in Reference Example 1, step 6, the title compound (0.034 g, 62%) was obtained.
ESI-MS m/z: 619 $(M + H)^+$; $^1H$-NMR ($CDCl_3$, $\delta$): 1.08 (t, J = 7.5 Hz, 3H), 1.37-1.48 (m, 2H), 1.79-1.83 (m, 2H), 2.36 (q, J = 7.5 Hz, 2H), 2.61-2.69 (m, 1H), 4.09-4.35 (m, 4H), 6.97-7.13 (m, 4H), 7.32-7.35 (m, 2H), 7.57 (s, 1H), 8.48 (s, 1H), 8.60 (s, 1H), 8.67 (s, 1H), 9.47 (s, 1H).

Reference Example 20: 7-(2,4-Dichlorophenylamino)-N-(ethylcarbamoyl)-6-[4-(4-fluorophenyl)piperidine-1-carbonyl]pyrazolo[1,5-a]pyrimidine-3-sulfonamide (compound a-20)

Using 7-(2,4-dichlorophenylamino)-6-[4-(4-fluorophenyl)piperidine-1-carbonyl]pyrazolo[1,5-a]pyrimidine-3-sulfonamide (0.050 g, 0.089 mmol) obtained in Reference Example 19, step 5 and in the same manner as in Reference Example 7, the title compound (0.038 g, 67%) was obtained.
ESI-MS m/z: 634 $(M + H)^+$; $^1H$-NMR ($CDCl_3$, $\delta$) : 1.15 (t, J = 7.3 Hz, 3H), 1.35-1.48 (m, 2H), 1.80-1.84 (m, 2H), 2.62-2.70 (m, 1H), 3.21-3.30 (m, 2H), 4.00-4.11 (m, 4H), 6.66-6.69 (m, 1H), 6.98-7.13 (m, 4H), 7.34-7.36 (m, 2H), 7.55 (d, J = 0.7 Hz, 1H), 8.16 (s, 1H), 8.44 (s, 1H), 8.49 (s, 1H), 8.49 (s, 1H).

Reference Example 21: 7-(2-Chloro-4-fluorophenylamino)-N-(cyclopropylcarbamoyl)-6-[4-(4-fluorophenyl)piperidine-1-carbonyl]pyrazolo[1,5-a]pyrimidine-3-sulfonamide (compound a-21)

(step 1)

Ethyl 7-(2-chloro-4-fluorophenylamino)pyrazolo[1,5-a]pyrimidine-6-carboxylate

Using ethyl 7-hydroxypyrazolo[1,5-a]pyrimidine-6-carboxylate (5.0 g, 24.1 mmol) and 2-chloro-4-fluoroaniline (2.81 g, 19.3 mmol) instead of 4-fluoro-2-methylaniline, and in the same manner as in Reference Example 1, step 1, the title compound (5.56 g, 69%) was obtained.

(step 2)

6-Ethoxycarbonyl-7-(2-chloro-4-fluorophenylamino)pyrazolo[1,5-a]pyrimidine-3-sulfonic acid

Using ethyl 7-(2-chloro-4-fluorophenylamino)pyrazolo[1,5-a]pyrimidine-6-carboxylate (5.55 g, 16.6 mmol) obtained in step 1 and in the same manner as in Reference Example 1, step 2, the title compound (6.09 g, 88%) was obtained.

(step 3)

Ethyl 3-(N-tert-butylsulfamoyl)-7-(2-chloro-4-fluorophenylamino)pyrazolo[1,5-a]pyrimidine-6-carboxylate

Using 6-ethoxycarbonyl-7-(2-chloro-4-fluorophenylamino)pyrazolo[1,5-a]pyrimidine-3-sulfonic acid (6.09 g, 14.7 mmol) obtained in step 2 and in the same manner as in Reference Example 1, step 3, the title compound (4.98 g, 72%) was obtained.

(step 4)

N-tert-Butyl-7-(2-chloro-4-fluorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chloro-4-fluorophenylamino)pyrazolo[1,5-a]pyrimidine-6-carboxylate (4.98 g, 10.60 mmol) obtained in step 3 and 4-(4-fluorophenyl)piperidine (1.43 g, 7.97 mmol) instead of 4-phenylpiperidine, and in the same manner as in Reference Example 1, step 4, the title compound (2.95 g, 93%) was obtained.

(step 5)

7-(2-Chloro-4-fluorophenylamino)-6-[4-(4-fluorophenyl)piperidine-1-carbonyl]pyrazolo[1,5-a]pyrimidine-3-sulfonamide

Using N-tert-butyl-7-(2-chloro-4-fluorophenylamino)-6-[4-(4-fluorophenyl)piperidine-1-carbonyl]pyrazolo[1,5-a]pyrimidine-3-sulfonamide (2.95 g, 4.89 mmol) obtained in step 4 and in the same manner as in Reference Example 1, step 5, the title compound (2.24 g, 84%) was obtained.

(step 6)

7-(2-Chloro-4-fluorophenylamino)-N-(cyclopropylcarbamoyl)-6-[4-(4-fluorophenyl)piperidine-1-carbonyl]pyrazolo[1,5-a]pyrimidine-3-sulfonamide (compound a-21)

Using 7-(2-chloro-4-fluorophenylamino)-6-[4-(4-fluorophenyl)piperidine-1-carbonyl]pyrazolo[1,5-a]pyrimidine-3-sulfonamide (0.10 g, 0.18 mmol) obtained in step 5 and cyclopropyl isocyanate (0.046 g, 0.549 mmol) instead of ethyl isocyanate, and in the same manner as in Reference Example 7, the title compound (0.055 g, 47%) was obtained.
ESI-MS m/z: 630 $(M + H)^+$; $^1H$-NMR (DMSO-$d_6$, 5): 0.28-0.36 (m, 2H), 0.52-0.65 (m, 2H), 1.20-1.90 (m, 4H), 2.20-2.28 (m, 1H), 2.40-2.44 (m, 1H), 2.72-2.78 (m, 1H), 3.00-3.05 (m, 1H), 3.72-4.12 (m, 2H), 6.53-6.55 (m, 1H), 7.05-7.62 (m, 7H), 8.37 (s, 1H), 8.58 (s, 1H), 10.35-10.62 (m, 2H).

Reference Example 22: N-{7-(2,5-Dichlorophenylamino)-6-[4-(4-fluorophenyl)piperidine-1-carbonyl]pyrazolo[1,5-a]pyrimidin-3-ylsulfonyl}propionamide (compound a-22)

(step 1)

Ethyl 7-(2,5-dichlorophenylamino)pyrazolo[1,5-a]pyrimidine-6-carboxylate

Using ethyl 7-hydroxypyrazolo[1,5-a]pyrimidine-6-carboxylate (7.50 g, 36.2 mmol) and 2,5-dichloroaniline (5.30 g, 32.6 mmol) instead of 4-fluoro-2-methylaniline, and in the same manner as in Reference Example 1, step 1, the title compound (8.64 g, 68%) was obtained.

(step 2)

6-Ethoxycarbonyl-7-(2,5-dichlorophenylamino)pyrazolo[1,5-a]pyrimidine-3-sulfonic acid

Using ethyl 7-(2,5-dichlorophenylamino)pyrazolo[1,5-a]pyrimidine-6-carboxylate (8.64 g, 24.6 mmol) obtained in step 1 and in the same manner as in Reference Example 1, step 2, the title compound (9.51 g, 90%) was obtained.

(step 3)

Ethyl 3-(N-tert-butylsulfamoyl)-7-(2,5-dichlorophenylamino)pyrazolo[1,5-a]pyrimidine-6-carboxylate

Using 6-ethoxycarbonyl-7-(2,5-dichlorophenylamino)pyrazolo[1,5-a]pyrimidine-3-sulfonic acid (9.51 g, 22.1 mmol) obtained in step 2 and in the same manner as in Reference Example 1, step 3, the title compound (8.52 g, 79%) was obtained.

(step 4)

N-tert-Butyl-7-(2,5-dichlorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2,5-dichlorophenylamino)pyrazolo[1,5-a]pyrimidine-6-carboxylate (2.95 g, 6.06 mmol) obtained in step 3 and 4-(4-fluorophenyl)piperidine (1.5 g, 8.18 mmol) instead of 4-phenylpiperidine, and in the same manner as in Reference Example 1, step 4, the title compound (2.35 g, 70%) was obtained.

(step 5)

7-(2,5-Dichlorophenylamino)-6-[4-(4-fluorophenyl)piperidine-1-carbonyl]pyrazolo[1,5-a]pyrimidine-3-sulfonamide

Using N-tert-butyl-7-(2,5-dichlorophenylamino)-6-[4-(4-fluorophenyl)piperidine-1-carbonyl]pyrazolo[1,5-a]pyrimidine-3-sulfonamide (2.35 g, 3.79 mmol) obtained in step 4 and in the same manner as in Reference Example 1, step 5, the title compound (1.56 g, 73%) was obtained.

(step 6)

N-{7-(2,5-Dichlorophenylamino)-6-[4-(4-fluorophenyl)piperidine-1-carbonyl]pyrazolo[1,5-a]pyrimidin-3-ylsulfonyl}propionamide (compound a-22)

Using 7-(2,5-dichlorophenylamino)-6-[4-(4-fluorophenyl)piperidine-1-carbonyl]pyrazolo[1,5-a]pyrimidine-3-sulfonamide (0.050 g, 0.089 mmol) obtained in step 5 and propionic acid (0.033 mL, 0.444 mmol) instead of cyclopropanecarboxylic acid, and in the same manner as in Reference Example 1, step 6, the title compound (0.050 g, 92%) was obtained.
ESI-MS m/z: 619 $(M + H)^+$; $^1H$-NMR ($CDCl_3$, $\delta$): 1.09 (t, J = 7.3 Hz, 3H), 1.46-1.56 (m, 2H), 1.81-1.87 (m, 2H), 2.36 (q, J = 7.5 Hz, 2H), 2.62-2.70 (m, 1H), 4.23-4.53 (m, 4H), 6.96-7.02 (m, 2H), 7.09-7.14 (m, 2H), 7.26-7.33 (m, 2H), 7.49 (d, J = 8.4 Hz, 1H), 8.64 (s, 1H), 8.66 (s, 1H).

Reference Example 23: 7-(2,5-Dichlorophenylamino)-N-(ethylcarbamoyl)-6-[4-(4-fluorophenyl)piperidine-1-carbonyl]pyrazolo[1,5-a]pyrimidine-3-sulfonamide (compound a-23)

Using 7-(2,5-dichlorophenylamino)-6-[4-(4-fluorophenyl)piperidine-1-carbonyl]pyrazolo[1,5-a]pyrimidine-3-sulfonamide (0.050 g, 0.089 mmol) obtained in Reference Example 22, step 5 and in the same manner as in Reference Example 7, the title compound (0.034 g, 60%) was obtained.
ESI-MS m/z: 634 $(M + H)^+$; $^1H$ NMR ($CDCl_3$, $\delta$): 1.15 (t, J = 7.3 Hz, 3H), 1.46-1.57 (m, 2H), 1.83-1.88 (m, 2H), 2.63-2.72 (m, 1H), 3.20-3.30 (m, 2H), 3.95-4.30 (m, 4H), 6.60-6.63 (m, 1H), 6.97-7.03 (m, 2H), 7.10-7.15 (m, 2H), 7.26-7.33 (m, 2H), 7.48 (d, J = 8.4 Hz, 1H), 8.45 (s, 1H), 8.57 (s, 1H), 8.58 (br s, 1H).

Reference Example 24: N-{7-(2,5-Dichlorophenylamino)-6-[4-(4-fluorophenyl)piperidine-1-carbonyl]pyrazolo[1,5-a]pyrimidin-3-ylsulfonyl}acetamide (compound a-24)

Using 7-(2,5-dichlorophenylamino)-6-[4-(4-fluorophenyl)piperidine-1-carbonyl]pyrazolo[1,5-a]pyrimidine-3-sulfonamide (0.07 g, 0.12 mmol) obtained in Reference Example 22, step 5 and acetic acid (0.036 mL, 0.62 mmol) instead of cyclopropanecarboxylic acid, and in the same manner as in Reference Example 1, step 6, the title compound (0.048 g, 64%) was obtained.
ESI-MS m/z: 605 $(M + H)^+$; $^1$H-NMR (DMSO-$d_6$, $\delta$): 1.25-1.83 (m, 4H), 1.93 (s, 3H), 2.22-2.28 (m, 1H), 2.70-2.78 (m, 1H), 3.05-3.10 (m, 1H), 3.84-4.18 (m, 2H), 7.05-7.72 (m, 7H), 8.42 (s, 1H), 8.63 (s, 1H), 10.49 (br s, 1H), 12.17 (br s, 1H).

Reference Example 25: N-{7-(2,5-Dichlorophenylamino)-6-[4-(4-fluorophenyl)piperidine-1-carbonyl]pyrazolo[1,5-a]pyrimidin-3-ylsulfonyl}-3-methyloxetane-3-carboxamide (compound a-25)

Using 7-(2,5-dichlorophenylamino)-6-[4-(4-fluorophenyl)piperidine-1-carbonyl]pyrazolo[1,5-a]pyrimidine-3-sulfonamide (0.07 g, 0.12 mmol) obtained in Reference Example 22, step 5 and 3-methyloxetane-3-carboxylic acid (0.072 g, 0.62 mmol) instead of cyclopropanecarboxylic acid, and in the same manner as in Reference Example 1, step 6, the title compound (0.063 g, 76%) was obtained.
ESI-MS m/z: 661 $(M + H)^+$; $^1$H-NMR (DMSO-$d_6$, $\delta$): 1.31-1.80 (m, 4H), 1.46 (s, 3H), 2.22-2.29 (m, 1H), 2.66-2.78 (m, 1H), 3.02-3.11 (m, 1H), 3.87-3.95 (m, 1H), 4.03-4.10 (m, 1H), 4.22 (d, J = 6.2 Hz, 2H), 4.63 (d, J = 6.2 Hz, 2H), 7.07-7.37 (m, 4H), 7.44 (d, J = 8.2 Hz, 1H), 7.59 (d, J = 8.4 Hz, 1H), 7.67 (s, 1H), 8.43 (s, 1H), 8.69 (s, 1H), 10.53 (s, 1H), 12.30 (br s, 1H).

Reference Example 26: N-{7-(2,5-Dichlorophenylamino)-6-[4-(4-fluorophenyl)piperidine-1-carbonyl]pyrazolo[1,5-a]pyrimidin-3-ylsulfonyl}formamide (compound a-26)

Using 7-(2,5-dichlorophenylamino)-6-[4-(4-fluorophenyl)piperidine-1-carbonyl]pyrazolo[1,5-a]pyrimidine-3-sulfonamide (0.10 g, 0.18 mmol) obtained in Reference Example 22, step 5 and formic acid (0.033 mL, 0.89 mmol) instead of cyclopropanecarboxylic acid, and in the same manner as in Reference Example 1, step 6, the title compound (0.037 g, 34%) was obtained.
ESI-MS m/z: 591 $(M + H)^+$; $^1$H-NMR (DMSO-$d_6$, $\delta$): 1.20-1.80 (m, 4H), 2.23-2.29 (m, 1H), 2.50-2.58 (m, 1H), 2.70-2.74 (m, 1H), 3.05-3.12 (m, 1H), 3.85-4.20 (m, 2H), 7.10-7.75 (m, 7H), 8.47 (s, 1H), 8.70 (s, 1H), 8.82 (br s, 1H), 10.55 (br s, 1H).

Reference Example 27: 7-(2,5-Dichlorophenylamino)-6-[4-(4-fluorophenyl)piperidine-1-carbonyl]-N-methoxypyrazolo[1,5-a]pyrimidine-3-sulfonamide (compound a-27)

(step 1)

7-(2,5-Dichlorophenylamino)-6-[4-(4-fluorophenyl)piperidine-1-carbonyl]pyrazolo[1,5-a]pyrimidine-3-sulfonic acid

Using 6-ethoxycarbonyl-7-(2,5-dichlorophenylamino)pyrazolo[1,5-a]pyrimidine-3-sulfonic acid (0.5 g, 1.16 mmol) obtained in Reference Example 22, step 2 and 4-(4-fluorophenyl)piperidine (1.42 g, 7.94 mmol) instead of 4-phenylpiperidine, and in the same manner as in Reference Example 1, step 4, the title compound (0.57 g, 63%) was obtained.

(step 2)

7-(2,5-Dichlorophenylamino)-6-[4-(4-fluorophenyl)piperidine-1-carbonyl]-N-methoxypyrazolo[1,5-a]pyrimidine-3-sulfonamide (compound a-27)

Using 7-(2,5-dichlorophenylamino)-6-[4-(4-fluorophenyl)piperidine-1-carbonyl]pyrazolo[1,5-a]pyrimidine-3-sulfonic acid (0.15 g, 0.27 mmol) obtained in step 1 and O-methylhydroxylamine hydrochloride (0.089 g, 1.06 mmol) instead of tert-butylamine, and in the same manner as in Reference Example 1, step 3, the title compound (0.069 g, 44%) was obtained.
ESI-MS m/z: 593 $(M + H)^+$; $^1$H-NMR (DMSO-$d_6$, $\delta$): 1.20-1.81 (m, 4H), 2.23-2.29 (m, 1H), 2.68-2.74 (m, 1H), 3.07-3.12 (m, 1H), 3.67 (s, 3H), 3.81-4.20 (m, 2H), 7.10-7.75 (m, 7H), 8.44 (s, 1H), 8.57 (s, 1H), 10.26 (s, 1H), 10.46 (s, 1H).

Reference Example 28: 7-(2,5-Dichlorophenylamino)-N-ethoxy-6-[4-(4-fluorophenyl)piperidine-1-carbonyl]-pyrazolo[1,5-a]pyrimidine-3-sulfonamide (compound a-28)

Using 7-(2,5-Dichlorophenylamino)-6-[4-(4-fluorophenyl)piperidine-1-carbonyl]pyrazolo[1,5-a]pyrimidine-3-sulfonic acid (0.15 g, 0.27 mmol) obtained in Reference Example 27, step 1 and O-ethylhydroxylamine hydrochloride (0.104 g, 1.06 mmol) instead of tert-butylamine, and in the same manner as in Reference Example 1, step 3, the title compound (0.036 g, 22%) was obtained.
ESI-MS m/z: 607 $(M + H)^+$; $^1$H-NMR (DMSO-$d_6$, $\delta$): 1.01 (t, J = 7.3 Hz, 3H), 1.20-1.81 (m, 4H), 2.23-2.29 (m, 1H), 2.68-2.74 (m, 1H), 3.07-3.12 (m, 1H), 3.92 (q, J = 7.3 Hz, 2H), 3.81-4.20 (m, 2H), 7.10-7.75 (m, 7H), 8.43 (s, 1H), 8.56 (s, 1H), 10.10 (s, 1H), 10.46 (s, 1H).

Reference Example 29: N-{7-(2-Chloro-5-trifluoromethylphenylamino)-6-[4-(4-fluorophenyl)piperidine-1-carbonyl]pyrazolo[1,5-a]pyrimidin-3-ylsulfonyl}propionamide (compound a-29)

(step 1)

Ethyl 7-(2-chloro-5-trifluoromethylphenylamino)pyrazolo[1,5-a]pyrimidine-6-carboxylate

Using ethyl 7-hydroxypyrazolo[1,5-a]pyrimidine-6-carboxylate (5.0 g, 24.1 mmol) and 2-chloro-5-trifluoromethylaniline (4.25 g, 21.7 mmol) instead of 4-fluoro-2-methylaniline, and in the same manner as in Reference Example 1, step 1, the title compound (6.49 g, 70%) was obtained.

(step 2)

6-Ethoxycarbonyl-7-(2-chloro-5-trifluoromethylphenylamino)pyrazolo[1,5-a]pyrimidine-3-sulfonic acid

Using ethyl 7-(2-chloro-5-trifluoromethylphenylamino)pyrazolo[1,5-a]pyrimidine-6-carboxylate (6.49 g, 16.9 mmol) obtained in step 1 and in the same manner as in Reference Example 1, step 2, the title compound (7.26 g, 93%) was obtained.

(step 3)

Ethyl 3-(N-tert-butylsulfamoyl)-7-(2-chloro-5-trifluoromethylphenylamino)pyrazolo[1,5-a]pyrimidine-6-carboxylate

Using 6-ethoxycarbonyl-7-(2-chloro-5-trifluoromethylphenylamino)pyrazolo[1,5-a]pyrimidine-3-sulfonic acid (7.26 g, 15.6 mmol) obtained in step 2 and in the same manner as in Reference Example 1, step 3, the title compound (5.44 g, 69%) was obtained.

(step 4)

N-tert-Butyl-7-(2-chloro-5-trifluoromethylphenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chloro-5-trifluoromethylphenylamino)pyrazolo[1,5-a]pyrimidine-6-carboxylate (2.87 g, 5.52 mmol) obtained in step 3 and 4-(4-fluorophenyl)piperidine (1.37 g, 7.62 mmol) instead of 4-phenylpiperidine, and in the same manner as in Reference Example 1, step 4, the title compound (2.63 g, 79%) was obtained.

(step 5)

7-(2-Chloro-5-trifluoromethylphenylamino)-6-[4-(4-fluorophenyl)piperidine-1-carbonyl]pyrazolo[1,5-a]pyrimidine-3-sulfonamide

Using N-tert-butyl-7-(2-chloro-5-trifluoromethylphenylamino)-6-[4-(4-fluorophenyl)piperidine-1-carbonyl]pyrazolo[1,5-a]pyrimidine-3-sulfonamide (2.63 g, 4.03 mmol) obtained in step 4 and in the same manner as in Reference Example 1, step 5, the title compound (1.30 g, 54%) was obtained.

(step 6)

N-{7-(2-Chloro-5-trifluoromethylphenylamino)-6-[4-(4-fluorophenyl)piperidine-1-carbonyl]pyrazolo[1,5-a]pyrimidin-3-ylsulfonyl}propionamide (compound a-29)

Using 7-(2-chloro-5-trifluoromethylphenylamino)-6-[4-(4-fluorophenyl)piperidine-1-carbonyl]pyrazolo[1,5-a]pyrimidine-3-sulfonamide (0.050 g, 0.084 mmol) obtained in step 5 and propionic acid (0.031 mL, 0.419 mmol) instead of cyclopropanecarboxylic acid, and in the same manner as in Reference Example 1, step 6, the title compound (0.048 g, 87%) was obtained.
ESI-MS m/z: 653 $(M + H)^+$; $^1H$ NMR ($CDCl_3$, $\delta$) :1-09 (t, J = 7.3 Hz, 3H), 1.42-1.54 (m, 2H), 1.78-1.83 (m, 2H), 2.37 (q, J = 7.5 Hz, 2H), 2.59-2.67 (m, 1H), 3.40-3.55 (m, 4H), 6.95-7.01 (m, 2H), 7.07-7.12 (m, 2H), 7.55-7.60 (m, 2H), 7.70 (d, J = 8.1 Hz, 1H), 8.67 (s, 1H), 8.85 (s, 1H).

Reference Example 30: 7-(2-Chloro-5-fluorophenylamino)-N-(ethylcarbamoyl)-6-[4-(4-fluorophenyl)piperidine-1-carbonyl]pyrazolo[1,5-a]pyrimidine-3-sulfonamide (compound a-30)

(step 1)

Ethyl 7-(2-chloro-5-fluorophenylamino)pyrazolo[1,5-a]pyrimidine-6-carboxylate

Using ethyl 7-hydroxypyrazolo[1,5-a]pyrimidine-6-carboxylate (3.5 g, 16.9 mmol) and 2-chloro-5-fluoroaniline (2.21 g, 15.2 mmol) instead of 4-fluoro-2-methylaniline, and in the same manner as in Reference Example 1, step 1, the title compound (4.27 g, 75%) was obtained.

(step 2)

6-Ethoxycarbonyl-7-(2-chloro-5-fluorophenylamino)pyrazolo[1,5-a]pyrimidine-3-sulfonic acid

Using ethyl 7-(2-chloro-5-fluorophenylamino)pyrazolo[1,5-a]pyrimidine-6-carboxylate (4.27 g, 12.8 mmol) obtained in step 1 and in the same manner as in Reference Example 1, step 2, the title compound (4.75 g, 90%) was obtained.

(step 3)

Ethyl 3-(N-tert-butylsulfamoyl)-7-(2-chloro-5-fluorophenylamino)pyrazolo[1,5-a]pyrimidine-6-carboxylate

Using 6-ethoxycarbonyl-7-(2-chloro-5-fluorophenylamino)pyrazolo[1,5-a]pyrimidine-3-sulfonic acid (4.75 g, 11.5 mmol) obtained in step 2 and in the same manner as in Reference Example 1, step 3, the title compound (4.16 g, 77%) was obtained.

(step 4)

N-tert-Butyl-7-(2-chloro-5-fluorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chloro-5-fluorophenylamino)pyrazolo[1,5-a]pyrimidine-6-carboxylate (4.16 g, 8.85 mmol) obtained in step 3 and 4-(4-fluorophenyl)piperidine (2.04 g, 11.4 mmol) instead of 4-phenylpiperidine, and in the same manner as in Reference Example 1 step 4, the title compound (3.54 g, 77%) was obtained.

(step 5)

7-(2-Chloro-5-fluorophenylamino)-6-[4-(4-fluorophenyl)piperidine-1-carbonyl]pyrazolo[1,5-a]pyrimidine-3-sulfonamide

Using N-tert-butyl-7-(2-chloro-5-fluorophenylamino)-6-[4-(4-fluorophenyl)piperidine-1-carbonyl]pyrazolo[1,5-a]pyrimidine-3-sulfonamide (3.54 g, 5.87 mmol) obtained in step 4 and in the same manner as in Reference Example 1, step 5, the title compound (2.71 g, 84%) was obtained.

(step 6)

7-(2-Chloro-5-fluorophenylamino)-N-(ethylcarbamoyl)-6-[4-(4-fluorophenyl)piperidine-1-carbonyl]pyrazolo[1,5-a]pyrimidine-3-sulfonamide (compound a-30)

Using 7-(2-chloro-5-fluorophenylamino)-6-[4-(4-fluorophenyl)piperidine-1-carbonyl]pyrazolo[1,5-a]pyrimidine-3-sulfonamide (0.050 g, 0.091 mmol) obtained in step 5 and in the same manner as in Reference Example 7, the title compound (0.053 g, 93%) was obtained.
ESI-MS m/z: 617 $(M + H)^+$; $^1H$ NMR ($CDCl_3$, $\delta$) : 1.15 (t, J = 7.3 Hz, 3H), 1.42-1.55 (m, 2H), 1.81-1.87 (m, 2H), 2.63-2.72 (m, 1H), 3.21-3.30 (m, 2H), 3.97-4.24 (m, 4H), 6.62-6.65 (m, 1H), 6.97-7.14 (m, 6H), 7.49-7.53 (m, 1H), 8.45 (s, 1H), 8.54 (s, 1H), 8.59 (s, 1H).

<u>Reference Example 31:</u> N-{7-(2-Fluoro-5-methylphenylamino)-6-[4-(4-fluorophenyl)piperidine-1-carbonyl]pyrazolo[1,5-a]pyrimidin-3-ylsulfonyl}propionamide (compound a-22)

(step 1)

Ethyl 7-(2-fluoro-5-methylphenylamino)pyrazolo[1,5-a]pyrimidine-6-carboxylate

Using ethyl 7-hydroxypyrazolo[1,5-a]pyrimidine-6-carboxylate (5.0 g, 24.1 mmol) and 2-fluoro-5-methylaniline (2.43 g, 19.3 mmol) instead of 4-fluoro-2-methylaniline, and in the same manner as in Reference Example 1, step 1, the title compound (3.80 g, 50%) was obtained.

(step 2)

6-Ethoxycarbonyl-7-(2-fluoro-5-methylphenylamino)pyrazolo[1,5-a]pyrimidine-3-sulfonic acid

Using ethyl 7-(2-fluoro-5-methylphenylamino)pyrazolo[1,5-a]pyrimidine-6-carboxylate (3.80 g, 12.1 mmol) obtained in step 1 and in the same manner as in Reference Example 1, step 2, the title compound (3.53 g, 74%) was obtained.

(step 3)

Ethyl 3-(N-tert-butylsulfamoyl)-7-(2-fluoro-5-methylphenylamino)pyrazolo[1,5-a]pyrimidine-6-carboxylate

Using 6-ethoxycarbonyl-7-(2-fluoro-5-methylphenylamino)pyrazolo[1,5-a]pyrimidine-3-sulfonic acid (3.53 g, 8.95 mmol) obtained in step 2 and in the same manner as in Reference Example 1, step 3, the title compound (2.59 g, 64%) was obtained.

(step 4)

N-tert-Butyl-7-(2-fluoro-5-methylphenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-fluoro-5-methylphenylamino)pyrazolo[1,5-a]pyrimidine-6-carboxylate (1.29 g, 2.89 mmol) obtained in step 3 and 4-(4-fluorophenyl)piperidine (0.73 g, 4.06 mmol) instead of 4-phenylpiperidine, and in the same manner as in Reference Example 1, step 4, the title compound (1.40 g, 95%) was obtained.

(step 5)

7-(2-Fluoro-5-methylphenylamino)-6-[4-(4-fluorophenyl)piperidine-1-carbonyl]pyrazolo[1,5-a]pyrimidine-3-sulfonamide

Using N-tert-butyl-7-(2-fluoro-5-methylphenylamino)-6-[4-(4-fluorophenyl)piperidine-1-carbonyl]pyrazolo[1,5-a]pyrimidine-3-sulfonamide (1.40 g, 2.40 mmol) obtained in step 4 and in the same manner as in Reference Example 1, step 5, the title compound (1.06 g, 84%) was obtained.

(step 6)

N-{7-(2-Fluoro-5-methylphenylamino)-6-[4-(4-fluorophenyl)piperidine-1-carbonyl]pyrazolo[1,5-a]pyrimidin-3-ylsulfonyl}propionamide (compound a-22)

Using 7-(2-fluoro-5-methylphenylamino)-6-[4-(4-fluorophenyl)piperidine-1-carbonyl]pyrazolo[1,5-a]pyrimidine-3-sulfonamide (0.10 g, 0.19 mmol) obtained in step 5 and propionic acid (0.071 mL, 0.95 mmol) instead of cyclopropanecarboxylic acid, and in the same manner as in Reference Example 1, step 6, the title compound (0.032 g, 40%) was obtained.
ESI-MS m/z: 583 $(M + H)^+$; $^1$H-NMR (DMSO-$d_6$, $\delta$): 0.89 (t, J = 7.4 Hz, 3H), 1.00-1.78 (m, 4H), 2.05-2.11 (m, 1H), 2.22 (q, J = 7.4 Hz, 2H), 2.28 (s, 3H), 2.67-2.72 (m, 1H), 2.92-2.98 (m, 1H), 3.70-4.13 (m, 2H), 7.03-7.35 (m, 7H), 8.38 (s, 1H), 8.62 (s, 1H), 10. 39 (s, 1H), 12.11 (br s, 1H).

Reference Example 32: 7-(2-Fluoro-5-methylphenylamino)-N-(cyclopropylcarbamoyl)-6-[4-(4-fluorophenyl)piperidine-1-carbonyl]pyrazolo[1,5-a]pyrimidine-3-sulfonamide (compound a-32)

Using 7-(2-fluoro-5-methylphenylamino)-6-[4-(4-fluorophenyl)piperidine-1-carbonyl]pyrazolo[1,5-a]pyrimidine-3-sulfonamide (0.10 g, 0.19 mmol) obtained in Reference Example 31, step 5 and cyclopropylisocyanate (0.048 g, 0.57 mmol) instead of ethylisocyanate, and in the same manner as in Reference Example 7, the title compound (0.053 g, 62%) was obtained.
ESI-MS m/z: 610 $(M + H)^+$; $^1$H-NMR (DMSO-$d_6$, $\delta$): 0.31-0.37 (m, 2H), 0.52-0.59 (m, 2H), 1.00-1.78 (m, 4H), 2.04-2.09 (m, 1H), 2.29 (s, 3H), 2.38-2.44 (m, 1H), 2.68-2.72 (m, 1H), 2.92-2.97 (m, 1H), 3.66-4.10 (m, 2H), 6.52-6.57 (m, 1H), 7.04-7.35 (m, 7H), 8.38 (s, 1H), 8.57 (s, 1H), 10.30-10.60 (m, 2H).

Reference Example 33: N-{7-(5-Chloro-2-fluorophenylamino)-6-[4-(4-fluorophenyl)piperidine-1-carbonyl]pyrazolo[1,5-a]pyrimidin-3-ylsulfonyl}acetamide (compound a-33)

(step 1)

Ethyl 7-(5-chloro-2-fluorophenylamino)pyrazolo[1,5-a]pyrimidine-6-carboxylate

Using ethyl 7-hydroxypyrazolo[1,5-a]pyrimidine-6-carboxylate (2.0 g, 9.65 mmol) and 5-chloro-2-fluoroaniline (1.26 g, 8.69 mmol) instead of 4-fluoro-2-methylaniline, and in the same manner as in Reference Example 1, step 1, the title compound (2.07 g, 64%) was obtained.

(step 2)

6-Ethoxycarbonyl-7-(5-chloro-2-fluorophenylamino)pyrazolo[1,5-a]pyrimidine-3-sulfonic acid

Using ethyl 7-(5-chloro-2-fluorophenylamino)pyrazolo[1,5-a]pyrimidine-6-carboxylate (2.07 g, 6.18 mmol) obtained in step 1 and in the same manner as in Reference Example 1, step 2, the title compound (2.19 g, 85%) was obtained.

(step 3)

Ethyl 3-(N-tert-butylsulfamoyl)-7-(5-chloro-2-fluorophenylamino)pyrazolo[1,5-a]pyrimidine-6-carboxylate

Using 6-ethoxycarbonyl-7-(5-chloro-2-fluorophenylamino)pyrazolo[1,5-a]pyrimidine-3-sulfonic acid (2.19 g, 5.28 mmol) obtained in step 2 and in the same manner as in Reference Example 1, step 3, the title compound (1.82 g, 73%) was obtained.

(step 4)

N-tert-Butyl-7-(5-chloro-2-fluorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(5-chloro-2-fluorophenylamino)pyrazolo[1,5-a]pyrimidine-6-carboxylate (1.82 g, 3.87 mmol) obtained in step 3 and 4-(4-fluorophenyl)piperidine (0.88 g, 4.89 mmol) instead of 4-phenylpiperidine, and in the same manner as in Reference Example 1, step 4, the title compound (0.92 g, 47%) was obtained.

(step 5)

7-(5-Chloro-2-fluorophenylamino)-6-[4-(4-fluorophenyl)piperidine-1-carbonyl]pyrazolo[1,5-a]pyrimidine-3-sulfonamide

Using N-tert-butyl-7-(5-chloro-2-fluorophenylamino)-6-[4-(4-fluorophenyl)piperidine-1-carbonyl]pyrazolo[1,5-a]pyrimidine-3-sulfonamide (0.92 g, 1.53 mmol) obtained in step 4 and in the same manner as in Reference Example 1, step 5, the title compound (0.72 g, 86%) was obtained.

(step 6)

N-{7-(5-Chloro-2-fluorophenylamino)-6-[4-(4-fluorophenyl)piperidine-1-carbonyl]pyrazolo[1,5-a]pyrimidin-3-ylsulfonyl}acetamide (compound a-33)

Using 7-(5-chloro-2-fluorophenylamino)-6-[4-(4-fluorophenyl)piperidine-1-carbonyl]pyrazolo[1,5-a]pyrimidine-3-sulfonamide (0.050 g, 0.091 mmol) obtained in step 5 and acetic acid (0.026 mL, 0.457 mmol) instead of cyclopropanecarboxylic acid, and in the same manner as in Reference Example 1, step 6, the title compound (0.047 g, 87%) was obtained.
ESI-MS m/z: 589 $(M + H)^+$; $^1H$ NMR ($CDCl_3$, δ): 1.47-1.55 (m, 2H), 1.85-1.89 (m, 2H), 2.11 (s, 3H), 2.65-2.74 (m, 1H), 3.31-3.60 (m, 4H), 6.98-7.03 (m, 2H), 7.11-7.24 (m, 3H), 7.30-7.36 (m, 2H), 8.48 (s, 1H), 8.59 (s, 1H), 8.64 (s, 1H).

<u>Reference Example 34</u>: 7-(5-Chloro-2-fluorophenylamino)-N-(ethylcarbamoyl)-6-[4-(4-fluorophenyl)piperidine-1-carbonyl]pyrazolo[1,5-a]pyrimidine-3-sulfonamide (compound a-34)

Using 7-(5-chloro-2-fluorophenylamino)-6-[4-(4-fluorophenyl)piperidine-1-carbonyl]pyrazolo[1,5-a]pyrimidine-3-sulfonamide (0.050 g, 0.091 mmol) obtained in Reference Example 33, step 5 and in the same manner as in Reference Example 7, the title compound (0.039 g, 68%) was obtained.
ESI-MS m/z: 618 $(M + H)^+$; $^1H$ NMR ($CDCl_3$, δ) : 1.14 (t, J = 7.2 Hz, 3H), 1.48-1.60 (m, 2H), 1.86-1.90 (m, 2H), 2.66-2.75 (m, 1H), 3.20-3.29 (m, 2H), 3.98-4.30 (m, 4H), 6.63-6.66 (m, 1H), 6.98-7.04 (m, 2H), 7.11-7.20 (m, 3H), 7.29-7.34 (m, 2H), 8.44 (s, 1H), 8.51 (s, 1H), 8.53 (s, 1H).

<u>Reference Example 35</u>: N-{7-(2,5-Difluorophenylamino)-6-[4-(4-fluorophenyl)piperidine-1-carbonyl]pyrazolo[1,5-a]pyrimidin-3-ylsulfonyl}acetamide (compound a-35)

(step 1)

Ethyl 7-(2,5-difluorophenylamino)pyrazolo[1,5-a]pyrimidine-6-carboxylate

Using ethyl 7-hydroxypyrazolo[1,5-a]pyrimidine-6-carboxylate (2.0 g, 9.65 mmol) and 2,5-difluoroaniline (1.12 g, 8.69 mmol) instead of 4-fluoro-2-methylaniline, and in the same manner as in Reference Example 1, step 1, the title compound (1.72 g, 56%) was obtained.

(step 2)

6-Ethoxycarbonyl-7-(2,5-difluorophenylamino)pyrazolo[1,5-a]pyrimidine-3-sulfonic acid

Using ethyl 7-(2,5-difluorophenylamino)pyrazolo[1,5-a]pyrimidine-6-carboxylate (1.72 g, 5.40 mmol) obtained in step 1 and in the same manner as in Reference Example 1, step 2, the title compound (1.86 g, 86%) was obtained.

(step 3)

Ethyl 3-(N-tert-butylsulfamoyl)-7-(2,5-difluorophenylamino)pyrazolo[1,5-a]pyrimidine-6-carboxylate

Using 6-ethoxycarbonyl-7-(2,5-difluorophenylamino)pyrazolo[1,5-a]pyrimidine-3-sulfonic acid (1.86 g, 4.67 mmol) obtained in step 2 and in the same manner as in Reference Example 1, step 3, the title compound (1.38 g, 65%) was obtained.

(step 4)

N-tert-Butyl-7-(2,5-difluorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2,5-difluorophenylamino)pyrazolo[1,5-a]pyrimidine-6-carboxylate (1.38 g, 3.04 mmol) obtained in step 3 and 4-(4-fluorophenyl)piperidine (0.70 g, 3.88 mmol) instead of 4-phenylpiperidine, and in the same manner as in Reference Example 1, step 4, the title compound (0.64 g, 43%) was obtained.

(step 5)

7-(2,5-Difluorophenylamino)-6-[4-(4-fluorophenyl)piperidine-1-carbonyl]pyrazolo[1,5-a]pyrimidine-3-sulfonamide

Using N-tert-butyl-7-(2,5-difluorophenylamino)-6-[4-(4-fluorophenyl)piperidine-1-carbonyl]pyrazolo[1,5-a]pyrimidine-3-sulfonamide (0.64 g, 1.10 mmol) obtained in step 4 and in the same manner as in Reference Example 1, step 5, the title compound (0.51 g, 88%) was obtained.

(step 6)

N-{7-(2,5-Difluorophenylamino)-6-[4-(4-fluorophenyl)piperidine-1-carbonyl]pyrazolo[1,5-a]pyrimidin-3-ylsulfonyl}acetamide (compound a-35)

Using 7-(2,5-difluorophenylamino)-6-[4-(4-fluorophenyl)piperidine-1-carbonyl]pyrazolo[1,5-a]pyrimidine-3-sulfonamide (0.050 g, 0.094 mmol) obtained in step 5 and acetic acid (0.027 mL, 0.471 mmol) instead of cyclopropanecarboxylic acid, and in the same manner as in Reference Example 1, step 6, the title compound (0.046 g, 84%) was obtained.
ESI-MS m/z: 573 $(M + H)^1$; $^1$H NMR ($CDCl_3$, 5): 1.48-1.58 (m, 2H), 1.83-1.89 (m, 2H), 2.10 (s, 3H), 2.65-2.73 (m, 1H), 3.38-3.56 (m, 4H), 6.91-7.24 (m, 7H), 8.49 (s, 1H), 8.56 (s, 1H), 8.64 (s, 1H).

<u>Reference Example 36</u>: N-{7-(2,5-Difluorophenylamino)-6-[4-(4-fluorophenyl)piperidine-1-carbonyl]pyrazolo[1,5-a]pyrimidin-3-ylsulfonyl}propionamide (compound a-36)

Using 7-(2,5-difluorophenylamino)-6-[4-(4-fluorophenyl)piperidine-1-carbonyl]pyrazolo[1,5-a]pyrimidine-3-sulfonamide (0.050 g, 0.094 mmol) obtained in Reference Example 35, step 5 and propionic acid (0.035 mL, 0.471 mmol) instead of cyclopropanecarboxylic acid, and in the same manner as in Reference Example 1, step 6, the title compound (0.048 g, 86%) was obtained.
ESI-MS m/z: 587 $(M + H)^+$; $^1$H NMR ($CDCl_3$, $\delta$): 1.08 (t, J = 7.5 Hz, 3H), 1.47-1.58 (m, 2H), 1.83-1.87 (m, 2H), 2.35 (q, J = 7.5 Hz, 2H), 2.64-2.73 (m, 1H), 3.41-3.58 (m, 4H), 6.91-7.24 (m, 7H), 8.55 (s, 1H), 8.65 (s, 1H).

<u>Reference Example 37</u>: 7-(2-Chloro-5-methylphenylamino)-N-(2,2-difluorocyclopropylcarbamoyl)-6-[4-(4-fluorophenyl)piperidine-1-carbonyl]pyrazolo[1,5-a]pyrimidine-3-sulfonamide (compound a-37)

2,2-Difluorocyclopropanecarboxylic acid (0.067 g, 0.552 mmol) was dissolved in 1,4-dioxane (2 mL), diphenylphosphoryl azide (0.182 g, 0.66 mmol) and triethylamine (0.092 mL, 0.66 mmol) were added, and the mixture was heated under reflux for 2 hr. The reaction solution was allowed to cool, 7-(2-chloro-5-methylphenylamino)-6-[4-(4-fluorophenyl)piperidine-1-carbonyl]pyrazolo[1,5-a]pyrimidine-3-sulfonamide (0.10 g, 0.18 mmol) obtained in Reference Example 12, step 5 and potassium carbonate were added, and the mixture was stirred at 50 °C for 3 hr. 5% Aqueous citric acid solution was added to quench the reaction, and the precipitated crystals were collected by filtration. The obtained crystals were purified by silica gel column chromatography (chloroform/methanol=10/1) to give the title compound (0.014 g, 11%).
ESI-MS m/z: 662 $(M + H)^+$; $^1$H-NMR (DMSO-$d_6$, $\delta$): 0.96-1.90 (m, 5H), 2.07-2.12 (m, 1H), 2.31 (s, 3H), 2.68-2.72 (m, 1H), 2.94-2.98 (m, 1H), 3.13-3.15 (m, 1H), 3.76-3.80 (m, 1H), 3.98-4.01 (m, 1H), 6.84 (br s, 1H), 7.03-7.52 (m, 8H), 8.37 (s, 1H), 8.59 (s, 1H), 10.34 (s, 1H), 10.87 (br s, 1H).

Reference Example 38: N-{7-(2,3-Dichlorophenylamino)-6-[4-(4-fluorophenyl)piperidine-1-carbonyl]pyrazolo[1,5-a]pyrimidin-3-ylsulfonyl}acetamide (compound a-38)

(step 1)

Ethyl 7-(2,3-dichlorophenylamino)pyrazolo[1,5-a]pyrimidine-6-carboxylate

Using ethyl 7-hydroxypyrazolo[1,5-a]pyrimidine-6-carboxylate (5.0 g, 24.1 mmol) and 2,3-dichloroaniline (3.13 g, 19.3 mmol) instead of 4-fluoro-2-methylaniline, and in the same manner as in Reference Example 1, step 1, the title compound (5.36 g, 63%) was obtained.

(step 2)

6-Ethoxycarbonyl-7-(2,3-dichlorophenylamino)pyrazolo[1,5-a]pyrimidine-3-sulfonic acid

Using ethyl 7-(2,3-dichlorophenylamino)pyrazolo[1,5-a]pyrimidine-6-carboxylate (5.36 g, 15.3 mmol) obtained in step 1 and in the same manner as in Reference Example 1, step 2, the title compound (6.00 g, 91%) was obtained.

(step 3)

Ethyl 3-(N-tert-butylsulfamoyl)-7-(2,3-dichlorophenylamino)pyrazolo[1,5-a]pyrimidine-6-carboxylate

Using 6-ethoxycarbonyl-7-(2,3-dichlorophenylamino)pyrazolo[1,5-a]pyrimidine-3-sulfonic acid (6.00 g, 13.9 mmol) obtained in step 2 and in the same manner as in Reference Example 1, step 3, the title compound (4.56 g, 67%) was obtained.

(step 4)

N-tert-Butyl-7-(2,3-dichlorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2,3-dichlorophenylamino)pyrazolo[1,5-a]pyrimidine-6-carboxylate (4.56 g, 9.38 mmol) obtained in step 3 and 4-(4-fluorophenyl)piperidine (2.31 g, 12.9 mmol) instead of 4-phenylpiperidine, and in the same manner as in Reference Example 1, step 4, the title compound (4.52 g, 85%) was obtained.

(step 5)

7-(2,3-Dichlorophenylamino)-6-[4-(4-fluorophenyl)piperidine-1-carbonyl]pyrazolo[1,5-a]pyrimidine-3-sulfonamide

Using N-tert-butyl-7-(2,3-dichlorophenylamino)-6-[4-(4-fluorophenyl)piperidine-1-carbonyl]pyrazolo[1,5-a]pyrimidine-3-sulfonamide (4.52 g, 7.30 mmol) obtained in step 4 and in the same manner as in Reference Example 1, step 5, the title compound (2.78 g, 68%) was obtained.

(step 6)

N-{7-(2,3-Dichlorophenylamino)-6-[4-(4-fluorophenyl)piperidine-1-carbonyl]pyrazolo[l,5-a]pyrimidin-3-ylsulfonyl}acetamide (compound a-38)

Using 7-(2,3-dichlorophenylamino)-6-[4-(4-fluorophenyl)piperidine-1-carbonyl]pyrazolo[1,5-a]pyrimidine-3-sulfonamide (0.050 g, 0.089 mmol) obtained in step 5 and acetic acid (0.025 mL, 0.444 mmol) instead of cyclopropanecarboxylic acid, and in the same manner as in Reference Example 1, step 6, the title compound (0.033 g, 61%) was obtained.
ESI-MS m/z: 605 $(M + H)^+$; $^1H$ NMR ($CDCl_3$, δ): 1.27-1.42 (m, 2H), 1.76-1.81 (m, 2H), 2.12 (s, 3H), 2.56-2.65 (m, 1H), 3.61-3.93 (m, 4H), 6.91-7.11 (m, 4H), 7.28-7.36 (m, 2H), 7.50 (dd, J = 7.3, 2.2 Hz, 1H), 8.61 (s, 1H), 8.62 (s, 1H), 8.66 (s, 1H), 9.50 (s, 1H).

Reference Example 39: 7-(3,4-Dichlorophenylamino)-N-(ethylcarbamoyl)-6-[4-(4-fluorophenyl)piperidine-1-carbonyl]pyrazolo[1,5-a]pyrimidine-3-sulfonamide (compound a-39)

(step 1)

Ethyl 7-(3,4-dichlorophenylamino)pyrazolo[1,5-a]pyrimidine-6-carboxylate

Using ethyl 7-hydroxypyrazolo[1,5-a]pyrimidine-6-carboxylate (3.0 g, 14.5 mmol) and 3,4-dichloroaniline (1.88 g, 11.6 mmol) instead of 4-fluoro-2-methylaniline, and in the same manner as in Reference Example 1, step 1, the title compound (2.90 g, 57%) was obtained.

(step 2)

6-Ethoxycarbonyl-7-(3,4-dichlorophenylamino)pyrazolo[1,5-a]pyrimidine-3-sulfonic acid

Using ethyl 7-(3,4-dichlorophenylamino)pyrazolo[1,5-a]pyrimidine-6-carboxylate (2.90 g, 8.26 mmol) obtained in step 1 and in the same manner as in Reference Example 1, step 2, the title compound (3.36 g, 94%) was obtained.

(step 3)

Ethyl 3-(N-tert-butylsulfamoyl)-7-(3,4-dichlorophenylamino)pyrazolo[1,5-a]pyrimidine-6-carboxylate

Using 6-ethoxycarbonyl-7-(3,4-dichlorophenylamino)pyrazolo[1,5-a]pyrimidine-3-sulfonic acid (3.36 g, 7.79 mmol) obtained in step 2 and in the same manner as in Reference Example 1, step 3, the title compound (1.40 g, 37%) was obtained.

(step 4)

N-tert-Butyl-7-(3,4-dichlorophenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(3,4-dichlorophenylamino)pyrazolo[1,5-a]pyrimidine-6-carboxylate (1.40 g, 2.88 mmol) obtained in step 3 and 4-(4-fluorophenyl)piperidine (0.48 g, 2.65 mmol) instead of 4-phenylpiperidine, and in the same manner as in Reference Example 1, step 4, the title compound (0.35 g, 32%) was obtained.

(step 5)

7-(3,4-Dichlorophenylamino)-6-[4-(4-fluorophenyl)piperidine-1-carbonyl]pyrazolo[1,5-a]pyrimidine-3-sulfonamide

Using N-tert-butyl-7-(3,4-dichlorophenylamino)-6-[4-(4-fluorophenyl)piperidine-1-carbonyl]pyrazolo[1,5-a]pyrimidine-3-sulfonamide (0.35 g, 0.569 mmol) obtained in step 4 and in the same manner as in Reference Example 1, step 5, the title compound (0.25 g, 79%) was obtained.

(step 6)

7-(3,4-Dichlorophenylamino)-N-(ethylcarbamoyl)-6-[4-(4-fluorophenyl)piperidine-1-carbonyl]pyrazolo[1,5-a]pyrimidine-3-sulfonamide (compound a-39)

Using 7-(3,4-dichlorophenylamino)-6-[4-(4-fluorophenyl)piperidine-1-carbonyl]pyrazolo[1,5-a]pyrimidine-3-sulfonamide (0.050 g, 0.089 mmol) obtained in step 5 and in the same manner as in Reference Example 7, the title compound (0.054 g, 60%) was obtained.
ESI-MS m/z: 631 $(M + H)^+$; $^1H$ NMR ($CDCl_3$, $\delta$): 0.85-0.88 (m, 2H), 0.94-0.98 (m, 2H), 1.38-1.55 (m, 2H), 1.56-1.63 (m, 1H), 1.81-1.89 (m, 2H), 2.64-2.73 (m, 1H), 4.22-4.38 (m, 4H), 6.98-7.04 (m, 2H), 7.12-7.21 (m, 3H), 7.41 (d, J = 2.6 Hz, 1H), 7.54 (d, J = 8.4 Hz, 1H), 8.41 (s, 1H), 8.59 (s, 1H).

Reference Example 40: N-{7-(2-Chloro-4-fluoro-5-methylphenylamino)-6-[4-(4-fluorophenyl)piperidine-1-carbonyl]pyrazolo[1,5-a]pyrimidin-3-ylsulfonyl}propionamide (compound a-40)

(step 1)

Ethyl 7-(2-chloro-4-fluoro-5-methylphenylamino)pyrazolo[1,5-a]pyrimidine-6-carboxylate

Using ethyl 7-hydroxypyrazolo[1,5-a]pyrimidine-6-carboxylate (4.35 g, 21.0 mmol) and 2-chloro-4-fluoro-5-methylaniline (3.02 g, 18.9 mmol) instead of 4-fluoro-2-methylaniline, and in the same manner as in Reference Example 1 step 1, the title compound (5.54 g, 76%) was obtained.

(step 2)

6-Ethoxycarbonyl-7-(2-chloro-4-fluoro-5-methylphenylamino)pyrazolo[1,5-a]pyrimidine-3-sulfonic acid

Using ethyl 7-(2-chloro-4-fluoro-5-methylphenylamino)pyrazolo[1,5-a]pyrimidine-6-carboxylate (5.54 g, 15.9 mmol) obtained in step 1 and in the same manner as in Reference Example 1, step 2, the title compound (5.92 g, 87%) was obtained.

(step 3)

Ethyl 3-(N-tert-butylsulfamoyl)-7-(2-chloro-4-fluoro-5-methylphenylamino)pyrazolo[1,5-a]pyrimidine-6-carboxylate

Using 6-ethoxycarbonyl-7-(2-chloro-4-fluoro-5-methylphenylamino)pyrazolo[1,5-a]pyrimidine-3-sulfonic acid (5.92 g, 13.8 mmol) obtained in step 2 and in the same manner as in Reference Example 1, step 3, the title compound (5.14 g, 77%) was obtained.

(step 4)

N-tert-Butyl-7-(2-chloro-4-fluoro-5-methylphenylamino)-6-[4-(4-fluorophenyl)piperidine-1-carbonyl]pyrazolo[1,5-a]pyrimidine-3-sulfonamide

Using ethyl 3-(N-tert-butylsulfamoyl)-7-(2-chloro-4-fluoro-5-methylphenylamino)pyrazolo[1,5-a]pyrimidine-6-carboxylate (2.94 g, 6.07 mmol) obtained in step 3 and 4-(4-fluorophenyl)piperidine (1.18 g, 6.58 mmol) instead of 4-phenylpiperidine, and in the same manner as in Reference Example 1, step 4, the title compound (0.90 g, 33%) was obtained.

(step 5)

7-(2-Chloro-4-fluoro-5-methylphenylamino)-6-[4-(4-fluorophenyl)piperidine-1-carbonyl]pyrazolo[1,5-a]pyrimidine-3-sulfonamide

Using N-tert-butyl-7-(2-chloro-4-fluoro-5-methylphenylamino)-6-[4-(4-fluorophenyl)piperidine-1-carbonyl]pyrazolo[1,5-a]pyrimidine-3-sulfonamide (0.90 g, 1.45 mmol) obtained in step 4 and in the same manner as in Reference Example 1, step 5, the title compound (0.59 g, 73%) was obtained.

(step 6)

N-{7-(2-Chloro-4-fluoro-5-methylphenylamino)-6-[4-(4-fluorophenyl)piperidine-1-carbonyl]pyrazolo[1,5-a]pyrimidin-3-ylsulfonyl}propionamide (compound a-40)

Using 7-(2-chloro-4-fluoro-5-methylphenylamino)-6-[4-(4-fluorophenyl)piperidine-1-carbonyl]pyrazolo[1,5-a]pyrimidine-3-sulfonamide (0.050 g, 0.089 mmol) obtained in step 5 and propionic acid (0.033 mL, 0.446 mmol) instead of cyclopropanecarboxylic acid, and in the same manner as in Reference Example 1, step 6, the title compound (0.052 g, 95%) was obtained.
ESI-MS m/z: 617 $(M + H)^+$; $^1H$ NMR ($CDCl_3$, $\delta$) : 1.09 (t, J = 7.5 Hz, 3H), 1.34-1.47 (m, 2H), 1.76-1.82 (m, 2H), 2.29 (d, J = 1.5 Hz, 3H), 2.35 (q, J = 7.5 Hz, 2H), 2.57-2.67 (m, 1H), 3.46-3.83 (m, 4H), 6.96-7.02 (m, 2H), 7.07-7.11 (m, 2H), 7.22-7.29 (m, 2H), 8.57 (s, 1H), 8.65 (s, 1H).

Reference Example 41: N-{7-(2-Chloro-4-fluoro-5-methylphenylamino)-6-[4-(4-fluorophenyl)piperidine-1-carbonyl]pyrazolo[1,5-a]pyrimidin-3-ylsulfonyl}cyclopropanecarboxamide (compound a-41)

Using 7-(2-chloro-4-fluoro-5-methylphenylamino)-6-[4-(4-fluorophenyl)piperidine-1-carbonyl]pyrazolo[1,5-a]pyrimidine-3-sulfonamide (0.050 g, 0.089 mmol) obtained in Reference Example 40, step 5 and in the same manner as in Reference Example 1, step 6, the title compound (0.054 g, 96%) was obtained.
ESI-MS m/z: 629 $(M + H)^+$; $^1H$ NMR ($CDCl_3$, $\delta$) : 0.83-0.90 (m, 2H), 1.00-1.05 (m, 2H), 1.37-1.48 (m, 2H), 1.56-1.63 (m, 1H), 1.77-1.82 (m, 2H), 2.29 (d, J = 1.5 Hz, 3H), 2.58-2.67 (m, 1H), 3.43-3.64 (m, 4H), 6.96-7.02 (m, 2H), 7.07-7.11 (m, 2H), 7.21-7.29 (m, 2H), 8.59 (s, 1H), 8.62 (s, 1H).

Reference Example 42: 7-(2-Chloro-4-fluoro-5-methylphenylamino)-N-(ethylcarbamoyl)-6-[4-(4-fluorophenyl)piperidine-1-carbonyl]pyrazolo[1,5-a]pyrimidine-3-sulfonamide (compound a-42)

Using 7-(2-chloro-4-fluoro-5-methylphenylamino)-6-[4-(4-fluorophenyl)piperidine-1-carbonyl]pyrazolo[1,5-a]pyrimidine-3-sulfonamide (0.050 g, 0.089 mmol) obtained in Reference Example 40, step 5 and in the same manner as in Reference Example 7, the title compound (0.050 g, 88%) was obtained.
ESI-MS m/z: 632 $(M + H)^+$; $^1H$ NMR ($CDCl_3$, $\delta$) : 1.16 (t, J = 7.1 Hz, 3H), 1.35-1.47 (m, 2H), 1.77-1.83 (m, 2H), 2.30 (d, J = 1.5 Hz, 3H), 2.58-2.67 (m, 1H), 3.20-3.31 (m, 2H), 4.07-4.24 (m, 4H), 6.70 (s, 1H), 6.97-7.03 (m, 2H), 7.07-7.12 (m, 2H), 7.20-7.32 (m, 2H), 8.00 (s, 1H), 8.39 (s, 1H), 8.43 (s, 1H), 8.48 (s, 1H).

Reference Example 43: 7-(4-Fluoro-2-methylphenylamino)-N-(ethylcarbamoyl)-6-[3H-spiro(isobenzofuran-1,4'-piperidine)-1'-carbonyl]pyrazolo[1,5-a]pyrimidine-3-sulfonamide (compound a-43)

(step 1)

N-tert-Butyl-7-(4-fluoro-2-methylphenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(4-fluoro-2-methylphenylamino)pyrazolo[1,5-a]pyrimidine-6-carboxylate (1.70 g, 3.78 mmol) obtained in Reference Example 1, step 3 and 3H-spiroisobenzofuran-1,4'-piperidine hydrochloride (0.83 g, 3.67 mmol) instead of 4-phenylpiperidine, and in the same manner as in Reference Example 1, step 4, the title compound (0.44 g, 30%) was obtained.

(step 2)

7-(4-Fluoro-2-methylphenylamino)-6-[3H-spiro(isobenzofuran-1,4'-piperidine)-1'-carbonyl]pyrazolo[1,5-a]pyrimidine-3-sulfonamide

Using N-tert-butyl-7-(4-fluoro-2-methylphenylamino)-6-[3H-spiro(isobenzofuran-1,4'-piperidine)-1'-carbonyl]pyrazolo[1,5-a]pyrimidine-3-sulfonamide (0.44 g, 0.742 mmol) obtained in step 1 and in the same manner as in Reference Example 1, step 5, the title compound (0.25 g, 63%) was obtained.

(step 3)

N-(Ethylcarbamoyl)-7-(4-fluoro-2-methylphenylamino)-6-[3H-spiro(isobenzofuran-1,4'-piperidine)-1'-carbonyl]pyrazolo[1,5-a]pyrimidine-3-sulfonamide (compound a-43)

Using 7-(4-fluoro-2-methylphenylamino)-6-[3H-spiro(isobenzofuran-1,4'-piperidine)-1'-carbonyl]pyrazolo[1,5-a]pyrimidine-3-sulfonamide (0.05 g, 0.093 mmol) obtained in step 2 and in the same manner as in Reference Example 7, the title compound (0.034 g, 60%) was obtained.
ESI-MS m/z: 608 $(M + H)^+$; $^1H$-NMR ($CDCl_3$, $\delta$): 1.15 (t, J = 7.6 Hz, 3H), 1.40-1.75 (m, 4H), 2.39 (s, 3H), 3.21-3.21 (m, 2H), 3.40-4.30 (m, 4H), 5.07 (s, 2H), 6.66 (br s, 1H), 6.97-7.12 (m, 3H), 7.18-7.32 (m, 5H), 8.23 (br s, 1H), 8.41 (s, 1H), 8.43 (s, 1H).

Reference Example 44: 7-(2-Chloro-5-methylphenylamino)-N-(ethylcarbamoyl)-6-[3H-spiro(isobenzofuran-1,4'-piperidine)-1'-carbonyl]pyrazolo[1,5-a]pyrimidine-3-sulfonamide (compound a-44)

(step 1)

N-tert-Butyl-7-(2-chloro-5-methylphenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(2-chloro-5-methylphenylamino)pyrazolo[1,5-a]pyrimidine-6-carboxylate (1.00 g, 2.14 mmol) obtained in Reference Example 12, step 3 and 3H-spiroisobenzofuran-1,4'-piperidine hydrochloride (0.50 g, 2.23 mmol) instead of 4-phenylpiperidine, and in the same manner as in Reference Example 1, step 4, the title compound (0.41 g, 31%) was obtained.

(step 2)

7-(2-Chloro-5-methylphenylamino)-6-[3H-spiro(isobenzofuran-1,4'-piperidine)-1'-carbonyl]pyrazolo[1,5-a]pyrimidine-3-sulfonamide

Using N-tert-butyl-7-(2-chloro-5-methylphenylamino)-6-[3H-spiro(isobenzofuran-1,4'-piperidine)-1'-carbonyl]pyrazolo[1,5-a]pyrimidine-3-sulfonamide (0.41 g, 0.673 mmol) obtained in step 1 and in the same manner as in Reference Example 1, step 5, the title compound (0.20 g, 54%) was obtained.

(step 3)

7-(2-Chloro-5-methylphenylamino)-N-(ethylcarbamoyl)-6-[3H-spiro(isobenzofuran-1,4'-piperidine)-1'-carbonyl]pyrazolo[1,5-a]pyrimidine-3-sulfonamide (compound a-44)

Using 7-(2-chloro-5-methylphenylamino)-6-[3H-spiro(isobenzofuran-1,4'-piperidine)-1'-carbonyl]pyrazolo[1,5-a]pyrimidine-3-sulfonamide (0.085 g, 0.162 mmol) obtained in step 2 and in the same manner as in Reference Example 7, the title compound (0.077 g, 48%) was obtained.
ESI-MS m/z: 624 $(M + H)^+$; $^1H$-NMR ($CDCl_3$, $\delta$) : 1.15 (t, J = 7.2 Hz, 3H), 1.40-1.80 (m, 4H), 2.38 (s, 3H), 2.40-4.30 (m, 4H), 3.26 (m, 2H), 5.03 (s, 2H), 6.66 (br-s, 1H), 6.99-7.35 (m, 6H), 7.46 (d, J = 8.1 Hz, 1H), 8.45 (s, 1H), 8.50 (br s, 1H), 8.60 (s, 1H).

Reference Example 45: N-Ethyl-7-(2-chloro-5-methylphenylamino)-6-[3H-spiro(isobenzofuran-1,4'-piperidine)-1'-carbonyl]pyrazolo[1,5-a]pyrimidine-3-sulfonamide (compound a-45)

Using ethyl 7-(2-chloro-5-methylphenylamino)-3-(N-ethylsulfamoyl)pyrazolo[1,5-a]pyrimidine-6-carboxylate (0.156 g, 0.359 mmol) obtained in Reference Example 15, step 1 and 3H-spiroisobenzofuran-1,4'-piperidine hydrochloride (0.083 g, 0.366 mmol) instead of 4-phenylpiperidine, and in the same manner as in Reference Example 1, step 4, the title compound (0.035 g, 17%) was obtained.
ESI-MS m/z: 581 $(M + H)^+$; $^1H$-NMR (DMSO-$d_6$, $\delta$): 1.02 (t, J = 7.3 Hz, 3H), 1.45-2.00 (m, 4H), 2.33 (s, 3H), 2.93-2.99 (m, 2H), 3.00-3.10 (m, 1H), 3.10-3.40 (m, 1H), 3.58-4.00 (m, 2H), 4.99 (s, 2H), 7.12-7.38 (m, 5H), 7.38-7.52 (m, 2H), 8.41 (s, 1H), 8.48 (s, 1H), 10.21 (br s, 1H).

Reference Example 46: N-{7-(4-Chloro-2-methylphenylamino)-6-[3H-spiro(isobenzofuran-1,4'-piperidine)-1'-carbonyl]pyrazolo[1,5-a]pyrimidin-3-ylsulfonyl}cyclopropanecarboxyamide (compound a-46)

(step 1)

Ethyl 3-(N-tert-butylsulfamoyl)-7-(4-chloro-2-methylphenylamino)-pyrazolo[1,5-a]pyrimidine-6-carboxylate

Using 7-(4-chloro-2-methylphenylamino)-6-(ethoxycarbonyl)pyrazolo[1,5-a]pyrimidine-3-sulfonic acid (3.59 g, 8.74 mmol) obtained in Reference Example 16, step 2 and in the same manner as in Reference Example 1, step 3, the title compound (3.96 g, 99%) was obtained.

(step 2)

N-tert-Butyl-7-(4-chloro-2-methylphenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(4-chloro-2-methylphenylamino)pyrazolo[1,5-a]pyrimidine-6-carboxylate (1.21 g, 2.59 mmol) obtained in step 1 and 3H-spiroisobenzofuran-1,4'-piperidine (0.696 g, 3.08 mmol) instead of 4-phenylpiperidine, and in the same manner as in Reference Example 1, step 4, the title compound (0.974 g, 62%) was obtained.

(step 3)

7-(4-Chloro-2-methylphenylamino)-6-[3H-spiro(isobenzofuran-1,4'-piperidine)-1'-carbonyl]pyrazolo[1,5-a]pyrimidine-3-sulfonamide

Using N-tert-butyl-7-(4-chloro-2-methylphenylamino)-6-[3H-spiro(isobenzofuran-1,4'-piperidine)-1'-carbonyl]pyrazolo[1,5-a]pyrimidine-3-sulfonamide (0.85 g, 1.42 mmol) obtained in step 2 and in the same manner as in Reference Example 1, step 5, the title compound (0.42 g, 55%) was obtained.

(step 4)

N-{7-(4-Chloro-2-methylphenylamino)-6-[3H-spiro(isobenzofuran-1,4'-piperidine)-1'-carbonyl]pyrazolo[1,5-a]pyrimidin-3-ylsulfonyl}cyclopropanecarboxyamide (compound a-46)

Using 7-(4-chloro-2-methylphenylamino)-6-[3H-spiro(isobenzofuran-1,4'-piperidine)-1'-carbonyl]pyrazolo[1,5-a]pyrimidine-3-sulfonamide (0.080 g, 0.145 mmol) obtained in step 3 and in the same manner as in Reference Example 1, step 6, the title compound (0.080 g, 89%) was obtained.
ESI-MS m/z: 619 $(M - H)^-$; $^1$H-NMR ($CDCl_3$, δ): 0.83-0.87 (m, 2H), 1.01-1.04 (m, 2H), 1.39-1.95 (m, 5H), 2.39 (s, 3H), 3.15-4.30 (m, 4H), 5.07 (s, 2H), 7.05-7.40 (m, 7H), 8.26 (br s, 1H), 8.58 (s, 1H), 8.62 (s, 1H).

Reference Example 47: 7-(4-Chloro-2-methylphenylamino)-N-(ethylcarbamoyl)-6-[3H-spiro(isobenzofuran-1,4'-piperidine)-1'-carbonyl]pyrazolo[1,5-a]pyrimidine-3-sulfonamide (compound a-47)

Using 7-(4-chloro-2-methylphenylamino)-6-[3H-spiro(isobenzofuran-1,4'-piperidine)-1'-carbonyl]pyrazolo[1,5-a]pyrimidine-3-sulfonamide (0.080 g, 0.145 mmol) obtained in Reference Example 46, step 3 and in the same manner as in Reference Example 7, the title compound (0.090 g, 99%) was obtained.
ESI-MS m/z: 622 $(M - H)^-$; $^1$H-NMR ($CDCl_3$, δ): 1.14 (t, J = 7.2 Hz, 3H), 1.90-2.10 (m, 4H), 2.39 (s, 3H), 3.20-3.26 (m, 2H), 3.40-4.30 (m, 4H), 5.07 (s, 2H), 6.65 (br s, 1H), 7.07-7.43 (m, 7H), 8.28 (br s, 1H), 8.42 (s, 1H), 8.43 (s, 1H).

Reference Example 48: N-{7-(2-Fluoro-4-methylphenylamino)-6-[3H-spiro(isobenzofuran-1,4'-piperidine)-1'-carbonyl]pyrazolo[1,5-a]pyrimidin-3-ylsulfonyl}propionamide (compound a-48)

(step 1)

Ethyl 7-(2-fluoro-4-methylphenylamino)pyrazolo[1,5-a]pyrimidine-6-carboxylate

Using ethyl 7-hydroxypyrazolo[1,5-a]pyrimidine-6-carboxylate (5.0 g, 24.1 mmol) and 4-methyl-2-fluoroaniline (6.04 g, 48.3 mmol) instead of 4-fluoro-2-methylaniline, and in the same manner as in Reference Example 1, step 1, the title compound (4.05 g, 53%) was obtained.

(step 2)

6-Ethoxycarbonyl-7-(2-fluoro-4-methylphenylamino)pyrazolo[1,5-a]pyrimidine-3-sulfonic acid

Using ethyl 7-(2-fluoro-4-methylphenylamino)pyrazolo[1,5-a]pyrimidine-6-carboxylate (4.05 g, 12.9mmol) obtained in step 1 and in the same manner as in Reference Example 1, step 2, the title compound (5.31 g, 100%) was obtained.

(step 3)

Ethyl 3-(N-tert-butylsulfamoyl)-7-(2-fluoro-4-methylphenylamino)pyrazolo[1,5-a]pyrimidine-6-carboxylate

Using 6-ethoxycarbonyl-7-(2-fluoro-4-methylphenylamino)pyrazolo[1,5-a]pyrimidine-3-sulfonic acid (5.31 g, 12.9 mmol) obtained in step 2 and in the same manner as in Reference Example 1, step 3, the title compound (5.88 g, 99%) was obtained.

(step 4)

N-tert-Butyl-7-(2-fluoro-4-methylphenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(2-fluoro-4-methylphenylamino)pyrazolo[1,5-a]pyrimidine-6-carboxylate (2.94 g, 6.4 mmol) obtained in step 3 and 3H-spiroisobenzofuran-1,4'-piperidine hydrochloride (1.61 g, 7.12 mmol) instead of 4-phenylpiperidine, and in the same manner as in Reference Example 1, step 4, the title compound (1.03 g, 25%) was obtained.

(step 5)

7-(2-Fluoro-4-methylphenylamino)-6-[3H-spiro(isobenzofuran-1,4'-piperidine)-1'-carbonyl]pyrazolo[1,5-a]pyrimidine-3-sulfonamide

Using N-tert-butyl-7-(2-fluoro-4-methylphenylamino)-6-[3H-spiro(isobenzofuran-1,4'-piperidine)-1'-carbonyl]pyrazolo[1,5-a]pyrimidine-3-sulfonamide (1.03 g, 1.74 mmol) obtained in step 4 and in the same manner as in Reference Example 1, step 5, the title compound (0.81 g, 87%) was obtained.

(step 6)

N-{7-(2-Fluoro-4-methylphenylamino)-6-[3H-spiro(isobenzofuran-1,4'-piperidine)-1'-carbonyl]pyrazolo[1,5-a]pyrimidin-3-ylsulfonyl}propionamide (compound a-48)

Using 7-(2-fluoro-4-methylphenylamino)-6-[3H-spiro(isobenzofuran-1,4'-piperidine)-1'-carbonyl]pyrazolo[1,5-a]pyrimidine-3-sulfonamide (0.10 g, 0.19 mmol) obtained in step 5 and propionic acid (0.069 mL, 0.93 mmol) instead of cyclopropanecarboxylic acid, and in the same manner as in Reference Example 1, step 6, the title compound (0.057 g, 52%) was obtained.
ESI-MS m/z: 593 $(M + H)^+$; $^1H$-NMR (DMSO-$d_6$, $\delta$): 0.88 (t, J = 7.4 Hz, 3H), 1.40-2.00 (m, 4H), 2.23 (q, J = 7.4 Hz, 2H), 2.36 (s, 3H), 2.40 (m, 1H), 3.10-3.96 (m, 2H), 5.01 (s, 2H), 6.37 (br t, J = 5.3 Hz, 1H), 7.05-7.40 (m, 7H), 8.42 (s, 1H), 8.62 (s, 1H), 10. 37 (s, 1H), 12.11 (s, 1H).

Reference Example 49: 7-(2-Fluoro-4-methylphenylamino)-N-(ethylcarbamoyl)-6-[3H-spiro(isobenzofuran-1,4'-piperidine)-1'-carbonyl]pyrazolo[1,5-a]pyrimidine-3-sulfonamide (compound a-49)

Using 7-(2-fluoro-4-methylphenylamino)-6-[3H-spiro(isobenzofuran-1,4'-piperidine)-1'-carbonyl]pyrazolo[1,5-a]pyrimidine-3-sulfonamide (0.08 g, 0.15 mmol) obtained in Reference Example 48, step 5 and in the same manner as in Reference Example 7, the title compound (0.037 g, 40%) was obtained.
ESI-MS m/z: 608 $(M + H)^+$; $^1H$-NMR (DMSO-$d_6$, $\delta$): 0.94 (t, J = 7.3 Hz, 3H), 1.40-2.00 (m, 4H), 2.36 (s, 3H), 2.92-3.02 (m, 2H), 3.09-3.40 (m, 2H), 3.60-3.95 (m, 2H), 5.00 (s, 2H), 6.37 (br t, J = 5.3 Hz, 1H), 7.05 (d, J = 7.6 Hz, 1H), 7.16 (d, J = 10.6 Hz, 1H), 7.22-7.38 (m, 5H), 8.41 (s, 1H), 8.57 (s, 1H), 10.34 (s, 1H), 10.58 (s, 1H).

Reference Example 50: N-{7-(2-Chloro-4-fluorophenylamino)-6-[3H-spiro(isobenzofuran-1,4'-piperidine)-1'-carbonyl]pyrazolo[1,5-a]pyrimidin-3-ylsulfonyl}propionamide (compound a-50)

(step 1)

N-tert-Butyl-7-(2-chloro-4-fluorophenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(2-chloro-4-fluorophenylamino)pyrazolo[1,5-a]pyrimidine-6-carboxylate (2.50 g, 5.30 mmol) obtained in Reference Example 21, step 3 and 3H-spiroisobenzofuran-1,4'-piperidine hydrochloride (1.84 g, 8.16 mmol) instead of 4-phenylpiperidine, and in the same manner as in Reference Example 1, step 4, the title compound (2.45 g, 79%) was obtained.

(step 2)

7-(2-Chloro-4-fluorophenylamino)-6-[3H-spiro(isobenzofuran-1,4'-piperidine)-1'-carbonyl]pyrazolo[1,5-a]pyrimidine-3-sulfonamide

Using N-tert-butyl-7-(2-chloro-4-fluorophenylamino)-6-[3H-spiro(isobenzofuran-1,4'-piperidine)-1'-carbonyl]pyrazolo[1,5-a]pyrimidine-3-sulfonamide (2.45 g, 4.00 mmol) obtained in step 1 and in the same manner as in Reference Example 1, step 5, the title compound (1.72 g, 77%) was obtained.

(step 3)

N-{7-(2-Chloro-4-fluorophenylamino)-6-[3H-spiro(isobenzofuran-1,4'-piperidine)-1'-carbonyl]pyrazolo[1,5-a]pyrimidin-3-ylsulfonyl}propionamide (compound a-50)

Using 7-(2-chloro-4-fluorophenylamino)-6-[3H-spiro(isobenzofuran-1,4'-piperidine)-1'-carbonyl]pyrazolo[1,5-a]pyrimidine-3-sulfonamide (0.10 g, 0.18 mmol) obtained in step 2 and propionic acid (0.067 mL, 0.90 mmol) instead of cyclopropanecarboxylic acid, and in the same manner as in Reference Example 1, step 6, the title compound (0.085 g, 77%) was obtained.
ESI-MS m/z: 613 $(M + H)^+$; $^1$H-NMR (DMSO-$d_6$, $\delta$): 0.89 (t, J = 7.4 Hz, 3H), 1.45-2.05 (m, 4H), 2.22 (q, J = 7.4 Hz, 2H), 2.43-2.47 (m, 1H), 3.26-3.30 (m, 1H), 3.58-4.00 (m, 2H), 5.01 (s, 2H), 7.23-7.72 (m, 7H), 8.44 (s, 1H), 8.62 (s, 1H), 10.42 (br s, 1H).

Reference Example 51: N-{7-(2-Fluoro-5-methylphenylamino)-6-[3H-spiro(isobenzofuran-1,4'-piperidine)-1'-carbonyl]pyrazolo[1,5-a]pyrimidin-3-ylsulfonyl}propionamide (compound a-51)

(step 1)

N-tert-Butyl-7-(2-fluoro-5-methylphenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(2-fluoro-5-methylphenylamino)pyrazolo[1,5-a]pyrimidine-6-carboxylate (1.29 g, 5.76 mmol) obtained in Reference Example 31, step 3 and 3H-spiroisobenzofuran-1,4'-piperidine hydrochloride (0.92 g, 4.06 mmol) instead of 4-phenylpiperidine, and in the same manner as in Reference Example 1, step 4, the title compound (1.40 g, 81%) was obtained.

(step 2)

7-(2-Fluoro-5-methylphenylamino)-6-[3H-spiro(isobenzofuran-1,4'-piperidine)-1'-carbonyl]pyrazolo[1,5-a]pyrimidine-3-sulfonamide

Using N-tert-butyl-7-(2-fluoro-5-methylphenylamino)-6-[3H-spiro(isobenzofuran-1,4'-piperidine)-1'-carbonyl]pyrazolo[1,5-a]pyrimidine-3-sulfonamide (1.40 g, 2.36 mmol) obtained in step 1 and in the same manner as in Reference Example 1, step 5, the title compound (0.84 g, 67%) was obtained.

(step 3)

N-{7-(2-Fluoro-5-methylphenylamino)-6-[3H-spiro(isobenzofuran-1,4'-piperidine)-1'-carbonyl]pyrazolo[1,5-a]pyrimidin-3-ylsulfonyl}propionamide (compound a-51)

Using 7-(2-fluoro-5-methylphenylamino)-6-[3H-spiro(isobenzofuran-1,4'-piperidine)-1'-carbonyl]pyrazolo[1,5-a]pyrimidine-3-sulfonamide (0.10 g, 0.19 mmol) obtained in step 2 and propionic acid (0.069 mL, 0.93 mmol) instead of cyclopropanecarboxylic acid, and in the same manner as in Reference Example 1, step 6, the title compound (0.041 g, 59%) was obtained.
ESI-MS m/z: 583 $(M + H)^+$; $^1$H-NMR (DMSO-$d_6$, $\delta$): 0.89 (t, J = 7.6 Hz, 3H), 1.42-2.00 (m, 4H), 2.22 (q, J = 6.6 Hz, 2H), 2.24-2.30 (m, 1H), 2.32 (s, 3H), 3.10-4.00 (m, 3H), 5.00 (s, 2H), 7.14-7.35 (m, 7H), 8.44 (s, 1H), 8.63 (s, 1H), 10.42 (s, 1H), 12.12 (s, 1H).

Reference Example 52: N-{7-(2-Fluoro-5-methylphenylamino)-6-[3H-spiro(isobenzofuran-1,4'-piperidine)-1'-carbonyl]pyrazolo[1,5-a]pyrimidin-3-ylsulfonyl}cyclopropanecarboxyamide (compound a-52)

Using 7-(2-fluoro-5-methylphenylamino)-6-[3H-spiro(isobenzofuran-1,4'-piperidine)-1'-carbonyl]pyrazolo[1,5-a]pyrimidine-3-sulfonamide (0.10 g, 0.19 mmol) obtained in Reference Example 51, step 2 and in the same manner as in Reference Example 1, step 6, the title compound (0.040 g, 57%) was obtained.
ESI-MS m/z: 605 $(M + H)^+$; $^1$H-NMR (DMSO-$d_6$, $\delta$): 0.60-0.86 (m, 4H), 1.45-1.98 (m, 5H), 2.31 (s, 3H), 2.32-2.36 (m, 1H), 3.10-3.96 (m, 3H), 5.00 (s, 2H), 7.12-7.40 (m, 7H), 8.44 (s, 1H), 8.58 (s, 1H), 10. 41 (br s, 1H), 12.42 (br s, 1H).

Reference Example 53: N-{7-(2,5-Dichlorophenylamino)-6-[3H-spiro(isobenzofuran-1,4'-piperidine)-1'-carbonyl]pyrazolo[1,5-a]pyrimidin-3-ylsulfonyl}propionamide (compound a-53)

(step 1)

N-tert-Butyl-7-(2,5-dichlorophenylamino)-6-[3H-spiro(isobenzofuran-1,4'-piperidine)-1'-carbonyl]pyrazolo[1,5-a]pyrimidine-3-sulfonamide

Using ethyl 3-(N-tert-butylsulfamoyl)-7-(2,5-dichlorophenylamino)pyrazolo[1,5-a]pyrimidine-6-carboxylate (2.36 g, 4.89 mmol) obtained in Reference Example 22, step 3 and 3H-spiroisobenzofuran-1,4'-piperidine hydrochloride (1.48 g, 6.55 mmol) instead of 4-phenylpiperidine, and in the same manner as in Reference Example 1, step 4, the title compound (1.95 g, 71%) was obtained.

(step 2)

7-(2,5-Dichlorophenylamino)-6-[3H-spiro(isobenzofuran-1,4'-piperidine)-1'-carbonyl]pyrazolo[1,5-a]pyrimidine-3-sulfonamide

Using N-tert-butyl-7-(2,5-dichlorophenylamino)-6-[3H-spiro(isobenzofuran-1,4'-piperidine)-1'-carbonyl]pyrazolo[1,5-a]pyrimidine-3-sulfonamide (1.95 g, 3.10 mmol) obtained in step 1 and in the same manner as in Reference Example 1, step 5, the title compound (1.68 g, 95%) was obtained.

(step 3)

N-{7-(2,5-Dichlorophenylamino)-6-[3H-spiro(isobenzofuran-1,4'-piperidine)-1'-carbonyl]pyrazolo[1,5-a]pyrimidin-3-ylsulfonyl}propionamide (compound a-53)

Using 7-(2,5-dichlorophenylamino)-6-[3H-spiro(isobenzofuran-1,4'-piperidine)-1'-carbonyl]pyrazolo[1,5-a]pyrimidine-3-sulfonamide (0.05 g, 0.087 mmol) obtained in step 2 and propionic acid (0.032 mL, 0.436 mmol) instead of cyclopropanecarboxylic acid, and in the same manner as in Reference Example 1, step 6, the title compound (0.027 g, 49%) was obtained.
ESI-MS m/z: 629 $(M + H)^+$; $^1$H NMR ($CDCl_3$, $\delta$): 1.09 (t, J = 7.3 Hz, 3H), 1.68-1.82 (m, 4H), 2.38 (q, J = 7.5 Hz, 2H), 3.32-3.68 (m, 4H), 5.06 (s, 2H), 7.08-7.11 (m, 1H), 7.19-7.37 (m, 5H), 7.53 (d, J = 8.4 Hz, 1H), 8.52 (s, 1H), 8.68 (s, 1H), 8.85 (s, 1H), 9.55 (s, 1H).

Reference Example 54: N-{7-(2,5-Dichlorophenylamino)-6-[3H-spiro(isobenzofuran-1,4'-piperidine)-1'-carbonyl]pyrazolo[1,5-a]pyrimidin-3-ylsulfonyl}cyclopropanecarboxamide (compound a-54)

Using 7-(2,5-dichlorophenylamino)-6-[3H-spiro(isobenzofuran-1,4'-piperidine)-1'-carbonyl]pyrazolo[1,5-a]pyrimidine-3-sulfonamide (0.05 g, 0.087 mmol) obtained in Reference Example 53, step 2 and in the same manner as in Reference Example 1, step 6, the title compound (0.022 g, 39%) was obtained.
ESI-MS m/z: 641 $(M + H)^+$; $^1$H NMR ($CDCl_3$, $\delta$): 0.84-0.90 (m, 2H), 1.00-1.05 (m, 2H), 1.65-1.70 (m, 1H), 1.73-1.81 (m, 4H), 3.39-3.63 (m, 4H), 5.06 (s, 2H), 7.08-7.11 (m, 1H), 7.20-7.36 (m, 5H), 7.52 (d, J = 8.4 Hz, 1H), 8.50 (s, 1H), 8.65 (s, 1H), 8.88 (s, 1H), 9.59 (s, 1H).

Reference Example 55: N-{7-(2-Chloro-5-methylphenylamino)-6-[3H-spiro(isobenzofuran-1,4'-piperidine)-1'-carbonyl]pyrazolo[1,5-a]pyrimidin-3-ylsulfonyl}acetamide (compound a-55)

Using 7-(2-chloro-5-methylphenylamino)-6-[3H-spiro(isobenzofuran-1,4'-piperidine)-1'-carbonyl]pyrazolo[1,5-a]pyrimidine-3-sulfonamide (0.07 g, 0.13 mmol) obtained in Reference Example 44, step 2 and acetic acid (0.036 mL, 0.63mmol) instead of cyclopropanecarboxylic acid, and in the same manner as in Reference Example 1, step 6, the title compound (0.027 g, 39%) was obtained.
ESI-MS m/z: 595 $(M + H)^+$; $^1$H-NMR (DMSO-$d_6$, $\delta$): 1.60-1.90 (m, 3H), 1.92 (s, 3H), 2.33 (s, 3H), 2.28-2.32 (m, 1H), 3.15-3.50 (m, 2H), 3.65-3.73 (m, 1H), 3.88-3.92 (m, 1H), 4.99 (s, 2H), 7.20-7.40 (m, 7H), 7.46 (d, J = 8.4 Hz, 1H), 8.43 (s, 1H), 8.61 (s, 1H), 10.36 (br s, 1H).

Reference Example 56: N-{7-(2-Chloro-5-methylphenylamino)-6-[3H-spiro(isobenzofuran-1,4'-piperidine)-1'-carbonyl]pyrazolo[1,5-a]pyrimidin-3-ylsulfonyl}propionamide (compound a-56)

Using 7-(2-chloro-5-methylphenylamino)-6-[3H-spiro(isobenzofuran-1,4'-piperidine)-1'-carbonyl]pyrazolo[1,5-a]pyrimidine-3-sulfonamide (0.07 g, 0.13 mmol) obtained in Reference Example 44, step 2 and propionic acid (0.047 mL, 0.63mmol) instead of cyclopropanecarboxylic acid, and in the same manner as in Reference Example 1, step 6, the title compound (0.052 g, 67%) was obtained.
ESI-MS m/z: 609 $(M + H)^+$; $^1$H-NMR (DMSO-$d_6$, $\delta$): 0.89 (t, J = 7.3 Hz, 3H),1.42-2.00 (m, 4H), 2.23 (q, J = 7.4 Hz, 2H), 2.33 (s, 3H), 3.15-3.40 (m, 2H), 3.62-3.67 (m, 1H), 3.88-3.92 (m, 1H), 4.99 (s, 2H), 7.18-7.36 (m, 6H), 7.47 (d, J = 8.4 Hz, 1H), 8.44 (s, 1H), 8.63 (s, 1H), 10.37 (s, 1H), 12.13 (s, 1H).

Reference Example 57: N-{7-(2-Chloro-5-methylphenylamino)-6-[3H-spiro(isobenzofuran-1,4'-piperidine)-1'-carbonyl]pyrazolo[1,5-a]pyrimidin-3-ylsulfonyl}cyclobutanecarboxamide (compound a-57)

Using 7-(2-chloro-5-methylphenylamino)-6-[3H-spiro(isobenzofuran-1,4'-piperidine)-1'-carbonyl]pyrazolo[1,5-a]pyrimidine-3-sulfonamide (0.07 g, 0.13 mmol) obtained in Reference Example 44, step 2 and cyclobutanecarboxylic acid (0.061 mL, 0.63mmol) instead of cyclopropanecarboxylic acid, and in the same manner as in Reference Example 1, step 6, the title compound (0.049 g, 61%) was obtained.
ESI-MS m/z: 634 $(M + H)^+$; $^1$H-NMR (DMSO-$d_6$, $\delta$): 1.46-2.15 (m, 11H), 2.33 (s, 3H), 3.05-3.35 (m, 2H), 3.62-3.67 (m, 1H), 3.88-3.90 (m, 1H), 4.99 (s, 2H), 7.19-7.36 (m, 6H), 7.47 (d, J = 8.2 Hz, 1H), 8.42 (s, 1H), 8.63 (s, 1H), 10.37 (s, 1H), 12.01 (s, 1H).

Reference Example 58: N-{7-(2-Chloro-5-methylphenylamino)-6-[3H-spiro(isobenzofuran-1,4'-piperidine)-1'-carbonyl]pyrazolo[1,5-a]pyrimidin-3-ylsulfonyl}-3-methyloxetane-3-carboxamide (compound a-58)

Using 7-(2-chloro-5-methylphenylamino)-6-[3H-spiro(isobenzofuran-1,4'-piperidine)-1'-carbonyl]pyrazolo[1,5-a]pyrimidine-3-sulfonamide (0.07 g, 0.13 mmol) obtained in Reference Example 44, step 2 and 3-methyloxetane-3-carboxylic acid (0.073 g, 0.63mmol) instead of cyclopropanecarboxylic acid, and in the same manner as in Reference Example 1, step 6, the title compound (0.050 g, 61%) was obtained.
ESI-MS m/z: 651 $(M + H)^+$; $^1$H-NMR (DMSO-$d_6$, $\delta$): 1.38-1.98 (m, 4H), 1.46 (s, 3H), 2.33 (s, 3H), 3.20-3.24 (m, 1H), 3.50-3.73 (m, 2H), 3.86-3.90 (m, 1H), 4.20-4.24 (m, 2H), 4.62-4.66 (m, 2H), 4.99 (s, 2H), 7.18-7.39 (m, 6H), 7.47 (d, J = 8.2 Hz, 1H), 8.45 (s, 1H), 8.68 (s, 1H), 10.40 (s, 1H), 12.30 (br s, 1H).

Reference Example 59: 7-(2-Fluoro-4-methylphenylamino)-N-(2,2-difluorocyclopropylcarbamoyl)-6-[3H-spiro(isobenzofuran-1,4'-piperidine)-1'-carbonyl]pyrazolo[1,5-a]pyrimidine-3-sulfonamide (compound a-59)

Using 7-(2-fluoro-4-methylphenylamino)-6-[3H-spiro(isobenzofuran-1,4'-piperidine)-1'-carbonyl]pyrazolo[1,5-a]pyrimidine-3-sulfonamide (0.1 g, 0.18 mmol) obtained in Reference Example 48, step 5 and in the same manner as in Reference Example 37, the title compound (0.012 g, 10%) was obtained.
ESI-MS m/z: 672 $(M + H)^+$; $^1$H-NMR (DMSO-$d_6$, δ): 0.75-1.30 (m, 1H), 1.34-1.47 (m, 1H), 1.50-1.85 (m, 6H), 2.38 (s, 3H), 3.00-4.25 (m, 3H), 5.03 (s, 2H), 6.90-7.35 (m, 8H), 7.47 (d, J = 8.2 Hz, 1H), 8.50 (s, 1H), 8.52 (br s, 1H), 8.76 (br s, 1H).

Reference Example 60: N-{7-(2-Chloro-5-methylphenylamino)-6-[6-(4-fluorophenyl)-2,6-diazaspiro[3,3]heptane-2-carbonyl]pyrazolo[1,5-a]pyrimidin-3-ylsulfonyl}propionamide (compound a-60)

(step 1)

N-tert-Butyl-7-(2-chloro-5-methylphenylamino)-6-[6-(4-fluorophenyl)-2,6-diazaspiro[3,3]heptane-2-carbonyl]pyrazolo[1,5-a]pyrimidine-3-sulfonamide

Using ethyl 3-(N-tert-butylsulfamoyl)-7-(2-chloro-5-methylphenylamino)pyrazolo[1,5-a]pyrimidine-6-carboxylate (0.184 g, 0.395 mmol) obtained in Reference Example 12, step 3 and 2-(4-fluorophenyl)-2,6-diazaspiro[3,3]heptane (0.065 g, 0.336 mmol) obtained in Reference Example 83 instead of 4-phenylpiperidine, and in the same manner as in Reference Example 1, step 4, the title compound (0.104 g, 50%) was obtained.

(step 2)

7-(2-Chloro-5-methylphenylamino)-6-[6-(4-fluorophenyl)-2,6-diazaspiro[3,3]heptane-2-carbonyl]pyrazolo[1,5-a]pyrimidine-3-sulfonamide

Using N-tert-butyl-7-(2-chloro-5-methylphenylamino)-6-[6-(4-fluorophenyl)-2,6-diazaspiro[3,3]heptane-2-carbonyl]piperidine-1-carbonyl]pyrazolo[1,5-a]pyrimidine-3-sulfonamide (0.104 g, 0.169 mmol) obtained in step 1 and in the same manner as in Reference Example 1, step 5, the title compound (0.058 g, 62%) was obtained.

(step 3)

N-{7-(2-Chloro-5-methylphenylamino)-6-[6-(4-fluorophenyl)-2,6-diazaspiro[3,3]heptane-2-carbonyl]pyrazolo[1,5-a]pyrimidin-3-ylsulfonyl}propionamide (compound a-60)

Using 7-(2-chloro-5-methylphenylamino)-6-[6-(4-fluorophenyl)-2,6-diazaspiro[3,3]heptane-2-carbonyl]pyrazolo[1,5-a]pyrimidine-3-sulfonamide (0.045 g, 0.081 mmol) obtained in step 2 and propionic acid (0.030 mL, 0.405 mmol) instead of cyclopropanecarboxylic acid, and in the same manner as in Reference Example 1, step 6, the title compound (0.027 g, 54%) was obtained.
ESI-MS m/z: 612 $(M + H)^+$; $^1$H NMR ($CDCl_3$, δ): 1.07 (t, J = 7.5 Hz, 3H), 2.30 (q, J = 7.6 Hz, 2H), 2.36 (s, 3H), 3.85 (s, 7H), 4.10-4.22 (m, 1H), 6.36-6.40 (m, 2H), 6.89-6.96 (m, 2H), 7.11-7.13 (m, 2H), 7.15 (s, 1H), 7.43 (d, J = 8.1 Hz, 1H), 8.52 (s, 1H), 8.58 (s, 1H).

Reference Example 61: 7-(2-Chloro-5-methylphenylamino)-N-(ethylcarbamoyl)-6-[6-(4-fluorophenyl)-2,6-diazaspiro[3,3]heptane-2-carbonyl]pyrazolo[1,5-a]pyrimidine-3-sulfonamide (compound a-61)

Using 7-(2-chloro-5-methylphenylamino)-6-[6-(4-fluorophenyl)-2,6-diazaspiro[3,3]heptane-2-carbonyl]pyrazolo[1,5-a]pyrimidine-3-sulfonamide (0.010 g, 0.018 mmol) obtained in Reference Example 60, step 2 and in the same manner as in Reference Example 7, the title compound (0.0073 g, 65%) was obtained.
ESI-MS m/z: 627 $(M + H)^+$; $^1$H NMR ($CDCl_3$, δ): 1.14 (t, J = 7.1 Hz, 3H), 2.36 (s, 3H), 2.52-2.69 (m, 2H), 3.21-3.29 (m, 2H), 3.86-4.37 (m, 6H), 6.41-6.45 (m, 2H), 6.70 (s, 1H), 6.91-6.97 (m, 2H), 7.11-7.14 (m, 2H), 7.39 (d, J = 8.8 Hz, 1H), 8.32 (s, 1H), 8.59 (s, 1H), 9.89 (s, 1H).

Reference Example 62: N,N-Diethyl-7-(4-fluoro-2-methylphenylamino)-6-(4-phenylpiperidine-1-carbonyl)pyrazolo[1,5-a]pyrimidine-3-sulfonamide (compound a-62)

According to Reference Example 2, the title compound was synthesized.
ESIMS m/z: 565 $(M + H)^+$

Reference Example 63: N-[7-(4-Fluoro-2-methylphenylamino)-6-(4-phenylpiperidine-1-carbonyl)pyrazolo[1,5-a]pyrimidin-3-ylsulfonyl]-2-methylaminoacetamide(compound a-63)

According to Reference Example 1, the title compound was synthesized.
ESIMS m/z: 580 $(M + H)^+$

Reference Example 64: N-[7-(4-Fluoro-2-methylphenylamino)-6-(4-phenylpiperidine-1-carbonyl)pyrazolo[1,5-a]pyrimidin-3-ylsulfonyl]-2-methoxyacetamide(compound a-64)

According to Reference Example 1, the title compound was synthesized.
ESIMS m/z: 581 (M + H)

Reference Example 65: N-[7-(4-Fluoro-2-methylphenylamino)-6-(4-phenylpiperidine-1-carbonyl)pyrazolo[1,5-a]pyrimidin-3-ylsulfonyl]-2-hydroxyacetamide(compound a-65)

According to Reference Example 1, the title compound was synthesized.
ESIMS m/z: 567 (M + H)

Reference Example 66: N-Ethylcarbamoyl-7-(4-fluoro-2-methylphenylamino)-6-(4-phenylpiperidine-1-carbonyl)pyrazolo[1,5-a]pyrimidine-3-sulfonamide(compound a-66)

(step 1)

7-(4-Fluoro-2-methylphenylamino)-N-methyl-6-(4-phenylpiperidine-1-carbonyl)pyrazolo[1,5-a]pyrimidine-3-sulfonamide

According to Reference Example 1, the title compound was synthesized.
ESIMS m/z: 523 (M + H)

(step 2)

N-Ethylcarbamoyl-7-(4-fluoro-2-methylphenylamino)-6-(4-phenylpiperidine-1-carbonyl)pyrazolo[1,5-a]pyrimidine-3-sulfonamide (compound a-66)

Using 7-(4-fluoro-2-methylphenylamino)-N-methyl-6-(4-phenylpiperidine-1-carbonyl)pyrazolo[1,5-a]pyrimidine-3-sulfonamide obtained in step 1, and according to Reference Example 7, the title compound was synthesized.
ESIMS m/z: 594 (M + H)

Reference Example 67: 7-(4-Fluoro-2-methylphenylamino)-N-phenylcarbamoyl-6-(4-phenylpiperidine-1-carbonyl)pyrazolo[1,5-a]pyrimidine-3-sulfonamide(compound a-67)

Using 7-(4-fluoro-2-methylphenylamino)-6-(4-phenylpiperidine-1-carbonyl)pyrazolo[1,5-a]pyrimidine-3-sulfonamide obtained in Reference Example 1, step 5, and according to Reference Example 7, the title compound was synthesized.
ESIMS m/z: 628 (M + H)

Reference Example 68: N-[7-(4-Fluoro-2-methylphenylamino)-6-(4-phenylpiperidine-1-carbonyl)pyrazolo[1,5-a]pyrimidin-3-ylsulfonyl]morpholine-4-carboxamide(compound a-68)

Using 7-(4-fluoro-2-methylphenylamino)-6-(4-phenylpiperidine-1-carbonyl)pyrazolo[1,5-a]pyrimidine-3-sulfonamide obtained in Reference Example 1, step 5, and according to Reference Example 8, the title compound was synthesized.
ESIMS m/z: 622 (M + H)

Reference Example 69: N-Ethylcarbamothioyl-7-(4-fluoro-2-methylphenylamino)-6-(4-phenylpiperidine-1-carbonyl)pyrazolo[1,5-a]pyrimidine-3-sulfonamide(compound a-69)

Using 7-(4-fluoro-2-methylphenylamino)-6-(4-phenylpiperidine-1-carbonyl)pyrazolo[1,5-a]pyrimidine-3-sulfonamide obtained in Reference Example 1, step 5, and according to Reference Example 7, the title compound was synthesized.
ESIMS m/z: 596 (M + H)

Reference Example 70: 2,2,2-Trifluoro-N-[7-(4-fluoro-2-methylphenylamino)-6-(4-phenylpiperidine-1-carbonyl)pyrazolo[1,5-a]pyrimidin-3-ylsulfonyl]acetamide(compound a-70)

According to Reference Example 1, the title compound was synthesized.
ESIMS m/z: 605 (M + H)

Reference Example 71: 7-(4-Fluoro-2-methylphenylamino)-N-methylsulfonyl-6-(4-phenylpiperidine-1-carbonyl)pyrazolo[1,5-a]pyrimidine-3-sulfonamide(compound a-71)

Using 7-(4-fluoro-2-methylphenylamino)-6-(4-phenylpiperidine-1-carbonyl)pyrazolo[1,5-a]pyrimidine-3-sulfonamide obtained in Reference Example 1, step 5, and according to Reference Example 8, the title compound was synthesized.
ESIMS m/z: 587 (M + H)

Reference Example 72: Methyl 7-(4-fluoro-2-methylphenylamino)-6-(4-phenylpiperidine-1-carbonyl)pyrazolo[1,5-a]pyrimidin-3-ylsulfonylcarbamate (compound a-72)

Using 7-(4-fluoro-2-methylphenylamino)-6-(4-phenylpiperidine-1-carbonyl)pyrazolo[1,5-a]pyrimidine-3-sulfonamide obtained in Reference Example 1, step 5, and according to Reference Example 8, the title compound was synthesized.
ESIMS m/z: 567 (M + H)

Reference Example 73: N-[7-(4-Fluoro-2-methylphenylamino)-6-(4-phenylpiperidine-1-carbonyl)pyrazolo[1,5-a]pyrimidin-3-ylsulfonyl]-N,N-dimethylformimidamide (compound a-73)

Using 7-(4-fluoro-2-methylphenylamino)-6-(4-phenylpiperidine-1-carbonyl)pyrazolo[1,5-a]pyrimidine-3-sulfonamide obtained in Reference Example 1, step 5, and according to Reference Example 8, the title compound was synthesized.
ESIMS m/z: 564(M + H)

Reference Example 74: Methyl 7-(2,5-dimethylphenylamino)-6-(4-phenylpiperidine-1-carbonyl)pyrazolo[1,5-a]pyrimidin-3-ylsulfonylcarbamate (compound a-74)

(step 1)

7-(2,5-Dimethylphenylamino)-6-(4-phenylpiperidine-1-carbonyl)pyrazolo[1,5-a]pyrimidine-3-sulfonamide

According to Reference Example 1, step 1 to step 5, the title compound was synthesized.
ESIMS m/z: 505 (M + H)

(step 2)

Methyl 7-(2,5-dimethylphenylamino)-6-(4-phenylpiperidine-1-carbonyl)pyrazolo[1,5-a]pyrimidin-3-ylsulfonylcarbamate (compound a-74)

Using 7-(2,5-dimethylphenylamino)-6-(4-phenylpiperidine-1-carbonyl)pyrazolo[1,5-a]pyrimidine-3-sulfonamide obtained in step 1, and according to Reference Example 8, the title compound was synthesized.
ESIMS m/z: 563 (M + H)

Reference Example 75: N-[7-Benzylamino-6-(4-phenylpiperidine-1-carbonyl)pyrazolo[1,5-a]pyrimidin-3-ylsulfonyl]cyclopropanecarboxamide (compound a-75)

(step 1)

7-Benzylamino-6-(4-phenylpiperidine-1-carbonyl)pyrazolo[1,5-a]pyrimidine-3-sulfonamide

According to Reference Example 1, step 1 to step 5, the title compound was synthesized.
ESIMS m/z: 491 (M + H)

(step 2)

N-[7-Benzylamino-6-(4-phenylpiperidine-1-carbonyl)pyrazolo[1,5-a]pyrimidin-3-ylsulfonyl]cyclopropanecarboxamide (compound a-75)

Using 7-benzylamino-6-(4-phenylpiperidine-1-carbonyl)pyrazolo[1,5-a]pyrimidine-3-sulfonamide obtained in step 1, and according to Reference Example 1, the title compound was synthesized.
ESIMS m/z: 559 (M + H)

Reference Example 76: 7-Benzylamino-N-ethylcarbamoyl-6-(4-phenylpiperidine-1-carbonyl)pyrazolo[1,5-a]pyrimidine-3-sulfonamide (compound a-76)

Using 7-benzylamino-6-(4-phenylpiperidine-1-carbonyl)pyrazolo[1,5-a]pyrimidine-3-sulfonamide obtained in Reference Example 75, step 1, and according to Reference Example 8, the title compound was synthesized.
ESIMS m/z: 563 (M + H)

Reference Example 77: N-Carbamoyl-7-(2-chloro-5-methylphenylamino)-6-[4-(4-fluorophenyl)piperidine-1-carbonyl]pyrazolo[1,5-a]pyrimidine-3-sulfonamide (compound a-77)

7-(2-Chloro-5-methylphenylamino)-6-[4-(4-fluorophenyl)piperidine-1-carbonyl]pyrazolo[1,5-a]pyrimidine-3-sulfonamide (0.1 g, 0.184 mmol) obtained in Reference Example 12, step 5 was dissolved in dioxane (1 mL), potassium cyanate (0.149 g, 1.842 mmol) was added and the mixture was stirred at 80°C for 2 days. The reaction mixture was allowed to cool, water (3 mL) was added, and the precipitated solid was collected by filtration and dried under reduced pressure to give the title compound (13 mg, 12%).
ESIMS m/z: 586 (M + H)

Reference Example 78: 7-(2,5-Dichlorophenylamino)-6-[4-(4-fluorophenyl)piperidine-1-carbonyl]-N-(1,3,4-thiadiazol-2-yl)pyrazolo[1,5-a]pyrimidine-3-sulfonamide (compound a-78)

According to Reference Example 27, the title compound was synthesized.
ESIMS m/z: 647 (M + H)

Reference Example 79: 7-(2,5-Dichlorophenylamino)-6-[4-(4-fluorophenyl)piperidine-1-carbonyl]-N-(pyridazin-3-yl)pyrazolo[1,5-a]pyrimidine-3-sulfonamide (compound a-79)

According to Reference Example 27, the title compound was synthesized.
ESIMS m/z: 641 (M + H)

Reference Example 80: 7-(2,5-Dichlorophenylamino)-6-[4-(4-fluorophenyl)piperidine-1-carbonyl]-N-(pyrimidin-2-yl)pyrazolo[1,5-a]pyrimidine-3-sulfonamide (compound a-80)

According to Reference Example 27, the title compound was synthesized.
ESIMS m/z: 641 (M + H)

Reference Example 81: 7-(2-Chloro-5-methylphenylamino)-6-[3H-spiro(isobenzofuran-1,4'-piperidine)-1'-carbonyl]-N-(tetrahydro-2H-pyran-4-ylcarbamoyl)pyrazolo[1,5-a]pyrimidine-3-sulfonamide (compound a-81)

According to Reference Example 59, the title compound was synthesized.

ESIMS m/z: 680 (M + H)

Reference Example 82: 7-(2-Chloro-5-methylphenylamino)-6-[3H-spiro(isobenzofuran-1,4'-piperidine)-1'-carbonyl]-N-(2,2,2-trifluoroethylcarbamoyl)pyrazolo[1,5-a]pyrimidine-3-sulfonamide (compound a-82)

According to Reference Example 59, the title compound was synthesized.
ESIMS m/z: 678 (M + H)

Reference Example 83: 2-(4-Fluorophenyl)-2,6-diazaspiro[3,3]heptane

(step 1)

tert-Butyl 6-(4-fluorophenyl)-2,6-diazaspiro[3,3]heptane-2-carboxylate

6-(tert-Butoxycarbonyl)-6-aza-2-azospiro[3,3]heptane oxalate (Organic Letters, vol. 10, page 3525, 2008; 0.050 g, 0.103 mmol) was dissolved in toluene (2.5 mL), 1-bromo-4-fluorobenzene (0.036 g, 0.206 mmol), tris(dibenzylideneacetone)dipalladium(0) (0.009 g, 0.010 mmol), (±)-BINAP (0.019 g, 0.031 mmol), potassium tert-butoxide (0.069 g, 0.617 mmol) and triethylamine (0.005 g, 0.051 mmol) were added, and the mixture was stirred at 110°C for 24 hr. The reaction mixture was allowed to cool to room temperature and filtered through Celite. The filtrate was concentrated under reduced pressure, and the obtained residue was purified by silica gel column chromatography (hexane/ethyl acetate=3/1) to give the title compound (0.029 g, 48%).

(step 2)

2-(4-Fluorophenyl)-2,6-diazaspiro[3,3]heptane

tert-Butyl 6-(4-fluorophenyl)-2,6-diazaspiro[3,3]heptane-2-carboxylate (0.029 g, 0.098 mmol) obtained in step 1 was dissolved in dichloromethane (0.3 mL), and trifluoroacetic acid (0.11 mL, 1.467 mmol) was added. The mixture was stirred at room temperature for 5 hr, and saturated aqueous sodium hydrogen carbonate solution was added to quench the reaction. The organic layer was separated, and the aqueous layer was extracted with chloroform. The organic layers were combined, washed with saturated brine, dried over anhydrous sodium sulfate, and concentrated under reduced pressure to give the title compound (0.013 g, 68%).
ESI-MS m/z: 193 $(M + H)^+$; $^1H$-NMR ($CDCl_3$, $\delta$): 2.51 (s, 1H), 3.81-3.98 (m, 8H), 6.34-6.44 (m, 2H), 6.87-6.95 (m, 2H).

INDUSTRIAL APPLICABILITY

According to the present invention, a chemokine receptor activity modulator containing a pyrazolopyrimidine derivative or a pharmaceutically acceptable salt thereof as an active ingredient and the like are provided.
This application is based on a patent application No. 2011-192130 filed in Japan (filing date: September 2, 2011), the contents of which are incorporated in full herein.

[Sequence Listing Free Text]

SEQ ID NO: 1-explanation of artificial sequence: synthetic DNA
SEQ ID NO: 2-explanation of artificial sequence: synthetic DNA
SEQ ID NO: 3-explanation of artificial sequence: synthetic DNA
SEQ ID NO: 4-explanation of artificial sequence: synthetic DNA

## Claims

**1.** A chemokine receptor activity modulator containing a pyrazolopyrimidine derivative represented by the formula (I)

[Chemical Formula 1]

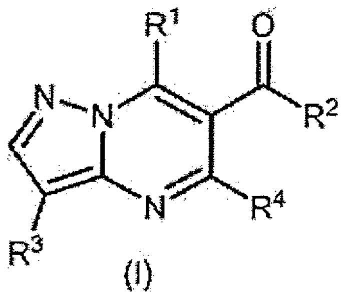

[wherein, $R^1$ represents $-NR^{1a}R^{1b}$ (wherein, $R^{1a}$ and $R^{1b}$ are the same or different and each represents a hydrogen atom, lower alkyl optionally having substituent(s), cycloalkyl optionally having substituent(s), lower alkoxy optionally having substituent(s), lower alkanoyl optionally having substituent(s), aryl optionally having substituent(s), aralkyl optionally having substituent(s), an aromatic heterocyclic group optionally having substituent(s) or an aliphatic heterocyclic group optionally having substituent(s), or $R^{1a}$ and $R^{1b}$ form, together with the adjacent nitrogen atom, a nitrogen-containing heterocyclic group optionally having substituent(s)), $-OR^{1c}$ (wherein, $R^{1c}$ represents a hydrogen atom, lower alkyl optionally having substituent(s), cycloalkyl optionally having substituent(s), aryl optionally having substituent(s), aralkyl optionally having substituent(s), an aromatic heterocyclic group optionally having substituent(s) or an aliphatic heterocyclic group optionally having substituent(s)) or $-SR^{1d}$ (wherein, $R^{1d}$ has the same definition as the above $R^{1c}$),
$R^2$ represents

[Chemical Formula 2]

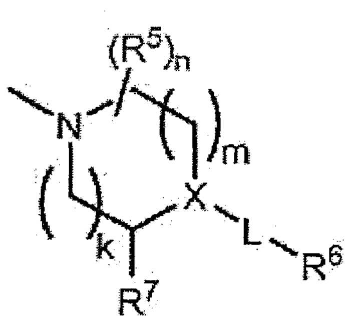

[wherein, k and m each represents an integer of 0 to 2 (provided that, the total of k and m is less than or equal to 3),
n represents an integer of 0 to 4, and when n is 2, 3 or 4, respective $R^5$s may be the same or different,
L represents a single bond, alkylene, C(=O) or $SO_2$,
$R^5$ represents halogen, hydroxy, lower alkyl optionally having substituent(s) or lower alkoxy optionally having substituent(s),
$R^6$ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),

X represents a nitrogen atom or $-CR^8$ (wherein, $R^8$ represents a hydrogen atom, halogen, hydroxy, cyano, lower alkyl optionally having substituent(s) or lower alkoxy optionally having substituent(s), or forms a bond together with $R^7$), and

$R^7$ forms a bond together with $R^8$, or represents a hydrogen atom, halogen, hydroxy, lower alkyl optionally having substituent(s) or lower alkoxy optionally having substituent(s)],

[Chemical Formula 3]

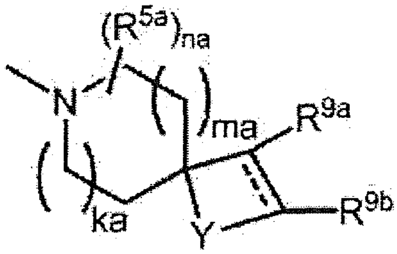

{wherein, ka, ma and na have the same definitions as the above k, m and n, respectively,

$R^{5a}$ has the same definition as the above $R^5$,

--- represents a single bond or a double bond,

$R^{9a}$ and $R^{9b}$ are the same or different and each represents a hydrogen atom or lower alkyl optionally having substituent(s), or $R^{9a}$ and $R^{9b}$ form, together with the respectively adjacent carbon atoms, an aliphatic ring optionally having substituent(s) or an aromatic ring optionally having substituent(s), and

Y represents $-CHR^{10a}-CHR^{10b}-$ (wherein, $R^{10a}$ and $R^{10b}$ are the same or different and each represents a hydrogen atom, hydroxy, lower alkyl optionally having substituent(s) or lower alkoxy optionally having substituent(s), or $R^{10a}$ and $R^{10b}$ form, together with the respectively adjacent carbon atoms, an aliphatic ring optionally having substituent(s)), $-CR^{10c}=CR^{10d}-$ (wherein, $R^{10c}$ and $R^{10d}$ are the same or different and each represents a hydrogen atom or lower alkyl optionally having substituent(s), or $R^{10c}$ and $R^{10d}$ form, together with the respectively adjacent carbon atoms, an aliphatic ring having at least one double bond and optionally having substituent(s) or an aromatic ring optionally having substituent(s)), $-Z^a-CR^{11a}R^{11b}-$ [wherein, $R^{11a}$ and $R^{11b}$ are the same or different and each represents a hydrogen atom or lower alkyl optionally having substituent(s), or $R^{11a}$ and $R^{11b}$ form carbonyl together with the adjacent carbon atom, and $Z^a$ represents C(=O), O, S, SO, $SO_2$ or $NR^{12}$ (wherein,

$R^{12}$ represents a hydrogen atom, lower alkyl optionally having substituent(s), lower alkanoyl optionally having substituent(s), lower alkoxycarbonyl optionally having substituent(s) or aralkyl optionally having substituent(s))], or $-CR^{11c}R^{11d}-Z^b-$(wherein, $R^{11c}$, $R^{11d}$ and $Z^b$ have the same definitions as the above $R^{11a}$, $R^{11b}$ and $Z^a$, respectively)}, or

[Chemical Formula 4]

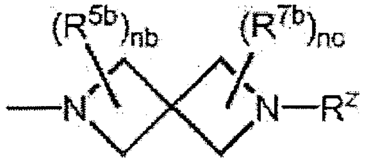

(wherein, $R^z$ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),

$R^{5b}$ and $R^{7b}$ are the same or different and each represents halogen, hydroxy, lower alkyl optionally having substituent(s) or lower alkoxy optionally having substituent(s),

nb represents an integer of 0 to 2, when nb is 2, respective $R^{5b}$s are the same or different, and

nc represents an integer of 0 to 2, when nc is 2, respective $R^{7b}$s are the same or different),

$R^3$ represents $-S(O)_2R^{13a}$ [wherein, $R^{13a}$ represents hydroxy, N, N-di-lower alkylaminomethyleneamino, lower alkoxy optionally having substituent(s), $-NR^{13b}R^{13c}$ (wherein, $R^{13b}$ and $R^{13c}$ are the same or different and each represents a hydrogen atom, lower alkyl optionally having substituent(s), lower alkoxy optionally having substituent(s), cycloalkyl optionally having substituent(s), aralkyl optionally having substituent(s), aryl optionally

having substituent(s), an aromatic heterocyclic group optionally having substituent(s) or an aliphatic heterocyclic group optionally having substituent(s), or $R^{13b}$ and $R^{13c}$ form, together with the adjacent nitrogen atom, a nitrogen-containing heterocyclic group optionally having substituent(s)), $-NR^{13d}C(=O)R^{13e}$ (wherein, $R^{13d}$ represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and $R^{13e}$ represents a hydrogen atom, amino, lower alkyl optionally having substituent(s), cycloalkyl optionally having substituent(s), aralkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s), N,N-di-arylamino optionally having substituent(s), aryl optionally having substituent(s), an aromatic heterocyclic group optionally having substituent(s) or an aliphatic heterocyclic group optionally having substituent(s)), $-NR^{13f}C(=S)R^{13g}$ (wherein, $R^{13f}$ and $R^{13g}$ have the same definitions as the above $R^{13d}$ and $R^{13e}$, respectively) or - $NR^{13h}S(O)_2R^{14}$ (wherein, $R^{13h}$ has the same definition as the above $R^{13d}$, and $R^{14}$ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s))], and
$R^4$ represents a hydrogen atom, halogen, lower alkyl optionally having substituent(s), aralkyl optionally having substituent(s), $-NR^{15a}R^{15b}$ (wherein, $R^{15a}$ and $R^{15b}$ have the same definitions as the above $R^{1a}$ and $R^{1b}$, respectively), $-OR^{15c}$ (wherein, $R^{15c}$ has the same definition as the above $R^{1c}$), or $-SR^{15d}$ (wherein, $R^{15d}$ has the same definition as the above $R^{1c}$)],

or a pharmaceutically acceptable salt thereof, as an active ingredient.

**2.** A chemokine receptor activity modulator containing a pyrazolopyrimidine derivative represented by the formula (IA)

[Chemical Formula 5]

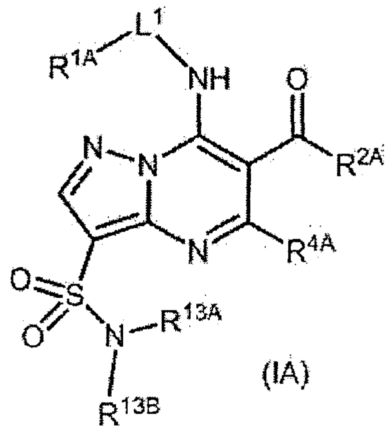

(IA)

[wherein,

$L^1$ represents a single bond or methylene,
$R^{1A}$ represents lower alkyl optionally having substituent(s), aralkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
$R^{2A}$ represents

[Chemical Formula 6]

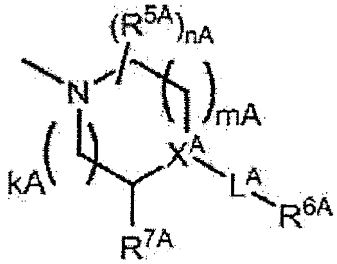

[wherein, nA represents an integer of 0 to 2, and when nA is 2, respective $R^{5A}$s may be the same or different, kA, mA and $L^A$ have the same definitions as the above k, m and 1, respectively,
$R^{5A}$ represents halogen or lower alkyl,
$R^{6A}$ represents cycloalkyl optionally having substituent(s), aryl optionally having substituent(s), an aromatic heterocyclic group optionally having substituent(s) or an aliphatic heterocyclic group optionally having substituent(s),
$X^A$ represents a nitrogen atom or $-CR^{8A}$ (wherein, $R^{8A}$ represents a hydrogen atom, halogen or lower alkyl, or forms a bond together with $R^{7A}$), and
$R^{7A}$ forms a bond together with $R^{8A}$, or represents a hydrogen atom, halogen or lower alkyl],

[Chemical Formula 7]

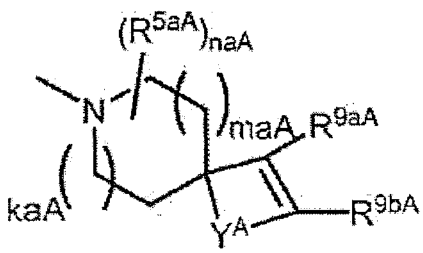

{wherein, naA and $R^{5aA}$ have the same definitions as the above nA and $R^{5A}$, respectively,
maA and kaA have the same definitions as the above ma and ka, respectively,
$R^{9aA}$ and $R^{9bA}$ form, together with the respectively adjacent carbon atoms, an aromatic ring optionally having substituent(s), and
$Y^A$ represents $-CHR^{10aA}-CHR^{10bA}-$ (wherein, $R^{10aA}$ and $R^{10bA}$ are the same or different and each represents a hydrogen atom, hydroxy, lower alkyl optionally having substituent(s) or lower alkoxy optionally having substituent(s)), $-CR^{10cA}=CR^{10dA}-$ (wherein, $R^{10cA}$ and $R^{10dA}$ are the same or different and each represents a hydrogen atom or lower alkyl optionally having substituent(s)), $-Z^{aA}-CR^{11aA}R^{11bA}-$ [wherein, $R^{11aA}$ and $R^{11bA}$ are the same or different and each represents a hydrogen atom or lower alkyl optionally having substituent(s), or $R^{11aA}$ and $R^{11bA}$ form carbonyl together with the adjacent carbon atom, and $Z^{aA}$ represents C(=O), O, S, SO, $SO_2$ or $NR^{12A}$ (wherein, $R^{12A}$ represents a hydrogen atom, lower alkyl optionally having substituent(s), lower alkanoyl optionally having substituent(s), lower alkoxycarbonyl optionally having substituent(s) or aralkyl optionally having substituent(s))], or $-CR^{11cA}R^{11dA}-Z^{bA}-$ (wherein, $R^{11cA}$, $R^{11dA}$ and $Z^{bA}$ have the same definitions as the above $R^{11aA}$, $R^{11bA}$ and $Z^{aA}$, respectively)}, or

[Chemical Formula 8]

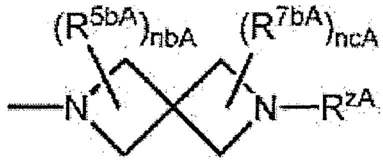

(wherein, $R^{aA}$ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s), $R^{5bA}$ and $R^{7bA}$ are the same or different and each represents halogen, hydroxy, lower alkyl optionally having substituent(s) or lower alkoxy optionally having substituent(s),

nbA represents an integer of 0 to 2, when nbA is 2, respective $R^{5bA}$s are the same or different, and

ncA represents an integer of 0 to 2, when ncA is 2, respective $R^{7bA}$s are the same or different),

$R^{13A}$ represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or $R^{13A}$ form, together with $R^{13B}$ and the adjacent nitrogen atom, a nitrogen-containing heterocyclic group optionally having substituent(s),

$R^{13B}$ represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s), an aliphatic heterocyclic group optionally having substituent(s) or $COR^{13e1}$ (wherein, $R^{13e1}$ has the same definition as the above $R^{13e}$), or $R^{13B}$ form, together with $R^{13A}$ and the adjacent nitrogen atom, a nitrogen-containing heterocyclic group optionally having substituent(s), and

$R^{4A}$ represents a hydrogen atom or lower alkyl optionally having substituent(s)],

or a pharmaceutically acceptable salt thereof, as an active ingredient.

3. The chemokine receptor activity modulator containing the pyrazolopyrimidine derivative, or a pharmaceutically acceptable salt thereof, as an active ingredient, according to claim 2, wherein $L^1$ is a single bond.

4. The chemokine receptor activity modulator containing the pyrazolopyrimidine derivative, or a pharmaceutically acceptable salt thereof, as an active ingredient, according to claim 2 or 3, wherein $R^{1A}$ is aryl optionally having substituent(s).

5. The chemokine receptor activity modulator containing the pyrazolopyrimidine derivative, or a pharmaceutically acceptable salt thereof, as an active ingredient, according to claim 2 or 3, wherein $R^{1A}$ is phenyl optionally having substituent(s).

6. The chemokine receptor activity modulator containing the pyrazolopyrimidine derivative, or a pharmaceutically acceptable salt thereof, as an active ingredient, according to any one of claims 2 to 5, wherein $R^{1A}$ is a hydrogen atom.

7. The chemokine receptor activity modulator containing the pyrazolopyrimidine derivative, or a pharmaceutically acceptable salt thereof, as an active ingredient, according to any one of claims 2 to 6, wherein $R^{13A}$ is a hydrogen atom and $R^{13B}$ is lower alkyl optionally having substituent(s).

8. The chemokine receptor activity modulator containing the pyrazolopyrimidine derivative, or a pharmaceutically acceptable salt thereof, as an active ingredient, according to any one of claims 2 to 6, wherein $R^{13A}$ is a hydrogen atom and $R^{13B}$ is $COR^{13e1}$ (wherein, $R^{13e1}$ has the same definition as described above).

9. The chemokine receptor activity modulator containing the pyrazolopyrimidine derivative, or a pharmaceutically acceptable salt thereof, as an active ingredient, according to any one of claims 2 to 6, wherein $R^{13A}$ is a hydrogen atom and $R^{13B}$ is $COR^{13e2}$ (wherein, $R^{13e2}$ represents lower alkyl optionally having substituent(s), cycloalkyl optionally having substituent(s), N-mono-lower alkylamino optionally having substituent(s) or N-mono-arylamino optionally having substituent(s)).

10. The chemokine receptor activity modulator containing the pyrazolopyrimidine derivative, or a pharmaceutically acceptable salt thereof, as an active ingredient, according to any one of claims 2 to 9, wherein $R^{2A}$ is

[Chemical Formula 9]

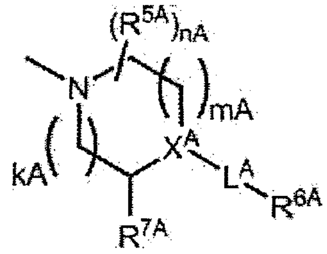

(wherein, nA, mA, kA, $R^{5A}$, $R^{6A}$, $R^{7A}$, $L^A$ and $X^A$ have the same definitions as described above, respectively).

**11.** The chemokine receptor activity modulator containing the pyrazolopyrimidine derivative, or a pharmaceutically acceptable salt thereof, as an active ingredient, according to claim 10, wherein $R^{6A}$ is phenyl optionally having substituent(s).

**12.** The chemokine receptor activity modulator containing the pyrazolopyrimidine derivative, or a pharmaceutically acceptable salt thereof, as an active ingredient, according to any one of claims 2 to 9, wherein $R^{2A}$ is

[Chemical Formula 10]

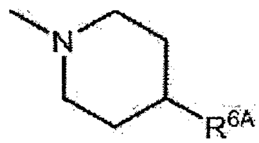

(wherein, $R^{6A}$ represents phenyl optionally having substituent(s)).

**13.** The chemokine receptor activity modulator containing the pyrazolopyrimidine derivative, or a pharmaceutically acceptable salt thereof, as an active ingredient, according to any one of claims 2 to 9, wherein $R^{2A}$ is

[Chemical Formula 11]

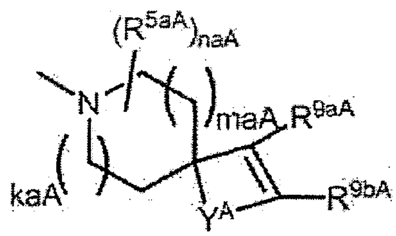

(wherein, naA, maA, kaA, $R^{5aA}$, $R^{9aA}$, $R^{9bA}$ and $Y^A$ have the same definitions as described above, respectively).

**14.** The chemokine receptor activity modulator containing the pyrazolopyrimidine derivative, or a pharmaceutically acceptable salt thereof, as an active ingredient, according to claim 13, wherein $R^{9aA}$ and $R^{9bA}$ form, together with the respectively adjacent carbon atoms, a benzene ring optionally having substituent(s), and $Y^A$ is $-CHR^{10aA}-CHR^{10bA}-$ (wherein, $R^{10aA}$ and $R^{10bA}$ have the same definitions as described above, respectively), $-CR^{10cA}=CR^{10dA}-$ (wherein, $R^{10cA}$ and $R^{10dA}$ have the same definitions as described above, respectively), $-O-CR^{11aA}R^{11bA}-$ (wherein, $R^{11aA}$ and $R^{11bA}$ have the same definitions as described above, respectively) or $-CR^{11cA}R^{11dA}-O-$ (wherein, $R^{11cA}$ and $R^{11dA}$ have the same definitions as described above, respectively).

**15.** The chemokine receptor activity modulator containing the pyrazolopyrimidine derivative, or a pharmaceutically acceptable salt thereof, as an active ingredient, according to any one of claims 2 to 9, wherein $R^{2A}$ is

[Chemical Formula 12]

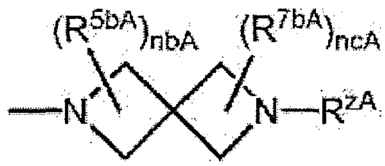

(wherein, $R^{zA}$, $R^{5bA}$, $R^{7bA}$, nbA and ncA have the same definitions as described above, respectively).

**16.** The chemokine receptor activity modulator containing the pyrazolopyrimidine derivative, or a pharmaceutically acceptable salt thereof, as an active ingredient, according to claim 15, wherein $R^{zA}$ is phenyl optionally having substituent(s).

**17.** The chemokine receptor activity modulator according to any one of claims 1 to 16, wherein the chemokine receptor activity modulator is a chemokine receptor antagonist.

**18.** The chemokine receptor activity modulator according to any one of claims 1 to 17, wherein the chemokine receptor is that selected from CCR4, CCR8, CCR9 and CCR10.

**19.** The chemokine receptor activity modulator according to any one of claims 1 to 17, wherein the chemokine receptor is CCR10.

**20.** A preventive and/or therapeutic agent for a disease involving a chemokine receptor, containing the pyrazolopyrimidine derivative described in any one of claims 1 to 16 or a pharmaceutically acceptable salt thereof as an active ingredient.

**21.** The preventive and/or therapeutic agent for a disease involving a chemokine receptor according to claim 20, wherein the chemokine receptor is that selected from CCR4, CCR8, CCR9 and CCR10.

**22.** The preventive and/or therapeutic agent for a disease involving a chemokine receptor according to claim 20, wherein the chemokine receptor is CCR10.

**23.** The preventive and/or therapeutic agent for a disease involving a chemokine receptor according to any one of claims 20 to 22, wherein the disease involving a chemokine receptor is a dermatic disease.

**24.** The preventive and/or therapeutic agent according to claim 23, wherein the dermatic disease is that selected from acne vulgaris, drug eruption, contact dermatitis, lepidopteran dermatitis, pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis.

**25.** The preventive and/or therapeutic agent according to claim 23, wherein the dermatic disease is dermatitis.

**26.** The preventive and/or therapeutic agent according to claim 23, wherein the dermatic disease is that selected from contact dermatitis and atopic dermatitis.

**27.** A method of modulating a chemokine receptor activity, comprising administering an effective amount of the pyrazolopyrimidine derivative described in any one of claims 1 to 16 or a pharmaceutically acceptable salt thereof.

**28.** The method of modulating a chemokine receptor activity according to claim 27, wherein the method of modulating a chemokine receptor is a method of antagonizing a chemokine receptor.

**29.** The method of modulating a chemokine receptor activity according to claim 27 or 28, wherein the chemokine receptor

is that selected from CCR4, CCR8, CCR9 and CCR10.

**30.** The method of modulating a chemokine receptor activity according to claim 27 or 28, wherein the chemokine receptor is CCR10.

**31.** A method for preventing and/or treating a disease involving a chemokine receptor, comprising administering an effective amount of the pyrazolopyrimidine derivative described in any one of claims 1 to 16 or a pharmaceutically acceptable salt thereof.

**32.** The method for preventing and/or treating a disease involving a chemokine receptor according to claim 31, wherein the chemokine receptor is that selected from CCR4, CCR8, CCR9 and CCR10.

**33.** The method for preventing and/or treating a disease involving a chemokine receptor according to claim 31, wherein the chemokine receptor is CCR10.

**34.** The method for preventing and/or treating a disease involving a chemokine receptor according to any one of claims 31 to 33, wherein the disease involving a chemokine receptor is a dermatic disease.

**35.** The method for preventing and/or treating a disease involving a chemokine receptor according to claim 34, wherein the dermatic disease is that selected from acne vulgaris, drug eruption, contact dermatitis, lepidopteran dermatitis, pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis.

**36.** The method for preventing and/or treating a disease involving a chemokine receptor according to claim 34, wherein the dermatic disease is dermatitis.

**37.** The method for preventing and/or treating a disease involving a chemokine receptor according to claim 34, wherein the dermatic disease is that selected from contact dermatitis and atopic dermatitis.

**38.** The pyrazolopyrimidine derivative described in any one of claims 1 to 16 or a pharmaceutically acceptable salt thereof for use in the modulation of chemokine receptor activity.

**39.** The pyrazolopyrimidine derivative according to claim 38 or a pharmaceutically acceptable salt thereof, wherein the modulation of chemokine receptor activity is the antagaonism of chemokine receptor.

**40.** The pyrazolopyrimidine derivative according to claim 38 or 39 or a pharmaceutically acceptable salt thereof, wherein the chemokine receptor is that selected from CCR4, CCR8, CCR9 and CCR10.

**41.** The pyrazolopyrimidine derivative according to claim 38 or 39 or a pharmaceutically acceptable salt thereof, wherein the chemokine receptor is CCR10.

**42.** The pyrazolopyrimidine derivative described in any one claims 1 to 16 or a pharmaceutically acceptable salt thereof for use in the prevention and/or treatment of a disease involving a chemokine receptor.

**43.** The pyrazolopyrimidine derivative according to claim 42 or a pharmaceutically acceptable salt thereof, wherein the chemokine receptor is that selected from CCR4, CCR8, CCR9 and CCR10.

**44.** The pyrazolopyrimidine derivative according to claim 42 or a pharmaceutically acceptable salt thereof, wherein the chemokine receptor is CCR10.

**45.** The pyrazolopyrimidine derivative according to any one of claims 42 to 44 or a pharmaceutically acceptable salt thereof, wherein the disease involving a chemokine receptor is a dermatic disease.

**46.** The pyrazolopyrimidine derivative according to claim 45 or a pharmaceutically acceptable salt thereof, wherein the dermatic disease is that selected from acne vulgaris, drug eruption, contact dermatitis, lepidopteran dermatitis,

pollen dermatitis, urticaria, psoriasis, atopic dermatitis, candidal dermatitis, seborrheic dermatitis, eczema, Stevens-Johnson syndrome, toxic epidermal necrosis, erythema multiforme, erythema nodosum, granuloma annulare, pityriasis rosea, rosacea, lichen planus, lichen pilaris (keratosis pilaris), photosensitivity, solar dermatitis, miliaria, herpes simplex, Kaposi's varicelliform eruption, impetigo contagiosa, staphylococcal scalded skin syndrome, erysipelas, erythema infectiosum, lupus erythematosus, keloid, Hailey-Hailey disease, scabies and linear dermatitis.

**47.** The pyrazolopyrimidine derivative according to claim 45 or a pharmaceutically acceptable salt thereof, wherein the dermatic disease is dermatitis.

**48.** The pyrazolopyrimidine derivative according to claim 45 or a pharmaceutically acceptable salt thereof, wherein the dermatic disease is that selected from contact dermatitis and atopic dermatitis.

## INTERNATIONAL SEARCH REPORT

International application No.
PCT/JP2012/072104

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K31/519, A61K31/5377, A61P17/00, A61P17/02, A61P17/04, A61P17/06, A61P17/10, A61P29/00, A61P37/08, A61P43/00, C07D487/04, C07D519/00, C12N15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2009/041663 A1 (Kyowa Hakko Kirin Co., Ltd.),<br>02 April 2009 (02.04.2009),<br>entire text<br>& TW 200918071 A & EP 2206714 A1<br>& KR 10-2010-087298 A & US 2010/256134 A1<br>& CN 101878217 A | 20-26,38-48<br>1-19 |
| Y | Edited by Tetsuo NAGANO et al., Soyaku Kagaku, Tokyo Kagaku Dojin, 2004, pages 134 to 135, fig. 7.8 | 20-26,38-48 |
| P,X<br>P,A | WO 2011/108689 A1 (Kyowa Hakko Kirin Co., Ltd.),<br>09 September 2011 (09.09.2011),<br>entire text<br>(Family: none) | 20-26,38-48<br>1-19 |

[X] Further documents are listed in the continuation of Box C.   [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 October, 2012 (18.10.12) | 30 October, 2012 (30.10.12) |
| Name and mailing address of the ISA/<br>Japanese Patent Office<br>Facsimile No. | Authorized officer<br><br>Telephone No. |

Form PCT/ISA/210 (second sheet) (July 2009)

INTERNATIONAL SEARCH REPORT

International application No. PCT/JP2012/072104

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-007341 A (Mitsubishi Tanabe Pharma Corp.), 15 January 2009 (15.01.2009), entire text (Family: none) | 1-19 |
| A | JP 2009-007342 A (Mitsubishi Tanabe Pharma Corp.), 15 January 2009 (15.01.2009), entire text (Family: none) | 1-19 |

Form PCT/ISA/210 (continuation of second sheet) (July 2009)

**INTERNATIONAL SEARCH REPORT**

International application No.
PCT/JP2012/072104

Continuation of A. CLASSIFICATION OF SUBJECT MATTER
(International Patent Classification (IPC))

*A61K31/519*(2006.01)i, *A61K31/5377*(2006.01)i, *A61P17/00*(2006.01)i, *A61P17/02*(2006.01)i, *A61P17/04*(2006.01)i, *A61P17/06*(2006.01)i, *A61P17/10*(2006.01)i, *A61P29/00*(2006.01)i, *A61P37/08*(2006.01)i, *A61P43/00*(2006.01)i, *C07D487/04*(2006.01)i, *C07D519/00*(2006.01)i, *C12N15/09*(2006.01)n

(According to International Patent Classification (IPC) or to both national classification and IPC)

Form PCT/ISA/210 (extra sheet) (July 2009)

**INTERNATIONAL SEARCH REPORT**

International application No.
PCT/JP2012/072104

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 27-37
because they relate to subject matter not required to be searched by this Authority, namely:
Claims 27-37 involve "method for treatment of the human body or animal body by surgery or therapy" and thus relate to a subject matter on which this International Searching Authority is not required to carry out an international search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0059908 A
- JP 2000038350 A
- JP 10101672 A
- JP 7157485 A
- WO 0053605 A
- WO 9854093 A
- JP 4270285 A
- WO 03091256 A
- WO 0240485 A
- WO 2004110454 A
- JP 5112571 A
- EP 0328700 A
- WO 0044754 A
- JP 9169762 A
- WO 2009041663 A
- US 3849458 A
- JP 5019536 B
- JP 61277648 A
- JP 61275241 A
- JP 63198638 A
- WO 03087366 A
- JP 2011192130 A


### Non-patent literature cited in the description


- *Clinical Dermatology,* 2008, vol. 26, 539
- *Journal of Allergy and Clinical Immunology,* 2006, vol. 118, 178
- *Nature Medicine,* 2002, vol. 8, 157-165
- *International Immunology,* 2006, vol. 18, 1233-1242
- *European Journal of Immunology,* 2008, vol. 38, 647-657
- *Experimental Dermatology,* 2007, vol. 17, 30-34
- Journal of the American Chemical Society. *J. Am. Chem. Soc.,* 2002, vol. 124 (10), 2092
- *LIPIDS,* 1974, vol. 9 (11), 913
- **T.W. GREENE.** Protective Groups in Organic Synthesis. John Wiley & Sons Inc, 1999
- *Journal of Medicinal Chemistry,* 2006, vol. 49, 2526
- **T. W. GREENE.** Protective groups in Organic Synthesis. John Wiley& Sons Inc, 1999
- **R.C. LAROCK.** Comprehensive Organic Transformations. Vch Verlagsgesellschaft Mbh, 1999
- *Analytical Biochemistry,* 2006, vol. 400, 163
- *Analy Biochem,* 2006, vol. 400, 163
- *Cytotechnology,* 1990, vol. 3, 133
- *Organic Letters,* 2008, vol. 10, 3525

SEQUENCE LISTING

<110> Kyowa Hakko Kirin Co., Ltd.

<120> Chemokine Receptor Activity Regulator

<130> X1290 EP S3

<140> EP 12 82 7928.8
<141> 2012-08-31

<150> JP2011-192130
<151> 2011-09-02

<160> 4

<170> PatentIn version 3.4

<210> 1
<211> 61
<212> DNA
<213> Artificial

<220>
<223> synthetic DNA

<400> 1
cagtcaagct tccaccatgg ggacggaggc cacagagcag gtttcctggg gccatttact 60

c 61

<210> 2
<211> 37
<212> DNA
<213> Artificial

<220>
<223> synthetic DNA

<400> 2
gttatagcgg ccgcagcctg ccccctcctc tagattc 37

<210> 3
<211> 25
<212> DNA
<213> Artificial

<220>
<223> synthetic DNA

<400> 3
cggagactct agagggtata taatg 25

<210> 4
<211> 21
<212> DNA
<213> Artificial

<220>
<223> synthetic DNA

<400> 4
ctaatacgac tcactatagg g 21